# EUROPÄISCHE PATENTSCHRIFT

(11) **EP 1 527 168 B1**
(45) Veröffentlichungstag und Bekanntmachung des Hinweises auf die Patenterteilung: **07.05.2008**
(21) Anmeldenummer: 03764996.9
(22) Anmeldetag: 16.07.2003
(51) Int. Cl.: C12N 9/14, C12N 15/82

(54) **SACCHAROSE-6-PHOSPHAT PHOSPHATASE ALS TARGET FÜR HERBIZIDE**
SACCHAROSE-6-PHOSPHATE PHOSPHATASE AS A TARGET FOR HERBICIDES
SACCHAROSE-6-PHOSPHATE PHOSPHATASE EN TANT QUE CIBLE D'HERBICIDES

(30) Priorität: 23.07.2002 DE 10233522
(43) Veröffentlichungstag der Anmeldung: 04.05.2005
(73) Patentinhaber: BASF SE, 67056 Ludwigshafen (DE)
(72) Erfinder: EHRHARDT, Thomas, 67346 Speyer (DE); SONNEWALD, Uwe, 06484 Quedlinburg (DE); BÖRNKE, Frederik, 06484 Quedlinburg (DE); CHEN, Shuai, 06466 Gatersleben (DE)
(74) Vertreter: Fitzner, Uwe
(86) Internationale Anmeldenummer: PCT/EP2003/007686
(87) Internationale Veröffentlichungsnummer: WO 2004/009808

(56) Entgegenhaltungen:
- EP-A- 1 033 405
- WO-A-01/79514
- US-B1- 6 323 015
- LUNN JOHN E ET AL: "Purification, molecular cloning, and sequence analysis of sucrose-6F-phosphate phosphohydrolase from plants" PROCEEDINGS OF THE NATIONAL ACADEMY OF SCIENCES OF THE UNITED STATES, Bd. 97, Nr. 23, 7. November 2000 (2000-11-07), Seiten 12914-12919, XP002263676 November 7, 2000 ISSN: 0027-8424
- LUNN J E: "Sucrose-phosphatase gene families in plants" GENE: AN INTERNATIONAL JOURNAL ON GENES AND GENOMES, ELSEVIER SCIENCE PUBLISHERS, BARKING, GB, Bd. 303, 16. Januar 2003 (2003-01-16), Seiten 187-196, XP004404827 ISSN: 0378-1119

### SEQUENCE LISTING

<110> BASF Aktiengesellschaft
<120> Saccharose-6-Phosphat Phosphatase als Target für Herbizide
<130> PF 53772
<150> DE 102 33 552.2
   <151> 2002-07-23
<160> 19
<170> PatentIn version 3.1
<210> 1
   <211> 1278
   <212> DNA
   <213> Nicotiana tabacum
<220>
   <221> CDS
   <222> (1)..(1275)
   <223>
<400> 1
   atg gat cag cta acc agt gcc gca cgt ctc atg ata gtc tca gat cta      48
   Met Asp Gln Leu Thr Ser Ala Ala Arg Leu Met Ile Val Ser Asp Leu
   1               5                   10                  15
   gac cat aca atg gta gat cat cat gat gcc gag aac ctt tct ctg ctt      96
   Asp His Thr Met Val Asp His His Asp Ala Glu Asn Leu Ser Leu Leu
               20                  25                  30
   aga ttt aat gct tta tgg gag gcg aat tat cgt gat aac tct ttg tta      144
   Arg Phe Asn Ala Leu Trp Glu Ala Asn Tyr Arg Asp Asn Ser Leu Leu
           35                  40                  45
   gtg ttc tca act ggg aga tca cct aca ctt tac aag gag ttg agg aaa      192
   Val Phe Ser Thr Gly Arg Ser Pro Thr Leu Tyr Lys Glu Leu Arg Lys
       50                  55                  60
   gaa aag ccc atg cta acc cca gat att act att atg tcg gtg gga act      240
   Glu Lys Pro Met Leu Thr Pro Asp Ile Thr Ile Met Ser Val Gly Thr
   65                  70                  75                  80
   gaa ata aca tat ggt aac tct gtg gtg cct gat gat ggt tgg gaa gct      288
   Glu Ile Thr Tyr Gly Asn Ser Val Val Pro Asp Asp Gly Trp Glu Ala
                   85                  90                  95
   ttt cta aat aac aag tgg gac aga aag ata gta aca gag gag act agc      336
   Phe Leu Asn Asn Lys Trp Asp Arg Lys Ile Val Thr Glu Glu Thr Ser
               100                 105                 110
   aag ttt cct gaa ctc act cta cag tca gaa acg gag cag cga cca cac      384
   Lys Phe Pro Glu Leu Thr Leu Gln Ser Glu Thr Glu Gln Arg Pro His
           115                 120                 125
   aag gtc agt ttc tat gtt cag aaa gac aaa gca caa gat ata atg aaa      432
   Lys Val Ser Phe Tyr Val Gln Lys Asp Lys Ala Gln Asp Ile Met Lys
       130                 135                 140
   act ctt tcc aag cgc ttc gaa gaa cgt ggg ctg gat gtc aaa ata att      480
   Thr Leu Ser Lys Arg Phe Glu Glu Arg Gly Leu Asp Val Lys Ile Ile
   145                 150                 155                 160
   tac agt gga ggc atg gat cta gat ata tta cca caa ggt gct ggc aaa      528
   Tyr Ser Gly Gly Met Asp Leu Asp Ile Leu Pro Gln Gly Ala Gly Lys
                   165                 170                 175
   gga caa gca ctt gca tat ttg ctt aag aaa ttg aag agt gag gga aaa      576
   Gly Gln Ala Leu Ala Tyr Leu Leu Lys Lys Leu Lys Ser Glu Gly Lys
               180                 185                 190
   tta cca aac aac acc ctt gcc tgt ggt gac tct ggg aat gat gct gag      624
   Leu Pro Asn Asn Thr Leu Ala Cys Gly Asp Ser Gly Asn Asp Ala Glu
           195                 200                 205
   cta ttc agt atc cca gat gtg tat ggt gta atg gta gct aat gca cag      672
   Leu Phe Ser Ile Pro Asp Val Tyr Gly Val Met Val Ala Asn Ala Gln
       210                 215                 220
   gag gaa tta ttg caa tgg cat gct gca aat gcg aag aat aat cct aaa      720
   Glu Glu Leu Leu Gln Trp His Ala Ala Asn Ala Lys Asn Asn Pro Lys
   225                 230                 235                 240
   gta att cat gca aca gag agg tgt gct gcc ggt atc ata caa gct att      768
   Val Ile His Ala Thr Glu Arg Cys Ala Ala Gly Ile Ile Gln Ala Ile
                   245                 250                 255
   ggt cat tcc aac cta ggt cca agt acc tcc cct aga gat gtt atg gat      816
   Gly His Ser Asn Leu Gly Pro Ser Thr Ser Pro Arg Asp Val Met Asp
               260                 265                 270
   ttg tca gac tgc aag atg gag aac ttt gtt ccc gcc tat gaa gtt gtc      864
   Leu Ser Asp Cys Lys Met Glu Asn Phe Val Pro Ala Tyr Glu Val Val
           275                 280                 285
   aaa ttt tac cta ttt ttt gag aaa tgg agg cgt gga gaa att gag cat      912
   Lys Phe Tyr Leu Phe Phe Glu Lys Trp Arg Arg Gly Glu Ile Glu His
       290                 295                 300
   tct gag cat tac ctg tca aac ctt aaa gca gtg tgt aga cca tct ggt      960
   Ser Glu His Tyr Leu Ser Asn Leu Lys Ala Val Cys Arg Pro Ser Gly
   305                 310                 315                 320
   act ttt gtc cac cca tct ggt gtt gag aaa tcc ctc cag gaa tgt gta     1008
   Thr Phe Val His Pro Ser Gly Val Glu Lys Ser Leu Gln Glu Cys Val
                   325                 330                 335
   act tta ttc ggg aca tgt cat ggt gac aaa cag ggg aaa caa ttt cgt     1056
   Thr Leu Phe Gly Thr Cys His Gly Asp Lys Gln Gly Lys Gln Phe Arg
               340                 345                 350
   att tgg gtc gat caa gtt tta cct gta cag gtt ggt tcg gac tca tgg     1104
   Ile Trp Val Asp Gln Val Leu Pro Val Gln Val Gly Ser Asp Ser Trp
           355                 360                 365
   tta gtg agt ttc aag aaa tgg gag ctc tct ggt gaa gac agg cga tgt     1152
   Leu Val Ser Phe Lys Lys Trp Glu Leu Ser Gly Glu Asp Arg Arg Cys
       370                 375                 380
   tgc ata act aca gtc cta tta agt tca aag aat aag act gtc gca gat      1200
   Cys Ile Thr Thr Val Leu Leu Ser Ser Lys Asn Lys Thr Val Ala Asp
   385                 390                 395                 400
   gga ctc act tgg acc cac gta cat cag aca tgg ctg aat gga gct gca      1248
   Gly Leu Thr Trp Thr His Val His Gln Thr Trp Leu Asn Gly Ala Ala
                   405                 410                 415
   gca agt gac tcc gcg tcc tgg ttc ttt tag                              1278
   Ala Ser Asp Ser Ala Ser Trp Phe Phe
               420                 425
<210> 2
   <211> 425
   <212> PRT
   <213> Nicotiana tabacum
<400> 2
   Met Asp Gln Leu Thr Ser Ala Ala Arg Leu Met Ile Val Ser Asp Leu
   1               5                   10                  15
   Asp His Thr Met Val Asp His His Asp Ala Glu Asn Leu Ser Leu Leu
               20                  25                  30
   Arg Phe Asn Ala Leu Trp Glu Ala Asn Tyr Arg Asp Asn Ser Leu Leu
           35                  40                  45
   Val Phe Ser Thr Gly Arg Ser Pro Thr Leu Tyr Lys Glu Leu Arg Lys
       50                  55                  60
   Glu Lys Pro Met Leu Thr Pro Asp Ile Thr Ile Met Ser Val Gly Thr
   65                  70                  75                  80
   Glu Ile Thr Tyr Gly Asn Ser Val Val Pro Asp Asp Gly Trp Glu Ala
   85 90 95
   Phe Leu Asn Asn Lys Trp Asp Arg Lys Ile Val Thr Glu Glu Thr Ser
   100 105 110
   Lys Phe Pro Glu Leu Thr Leu Gln Ser Glu Thr Glu Gln Arg Pro His
   115 120 125
   Lys Val Ser Phe Tyr Val Gln Lys Asp Lys Ala Gln Asp Ile Met Lys
   130 135 140
   Thr Leu Ser Lys Arg Phe Glu Glu Arg Gly Leu Asp Val Lys Ile Ile
   145 150 155 160
   Tyr Ser Gly Gly Met Asp Leu Asp Ile Leu Pro Gln Gly Ala Gly Lys
   165 170 175
   Gly Gln Ala Leu Ala Tyr Leu Leu Lys Lys Leu Lys Ser Glu Gly Lys
   180 185 190
   Leu Pro Asn Asn Thr Leu Ala Cys Gly Asp Ser Gly Asn Asp Ala Glu
   195 200 205
   Leu Phe Ser Ile Pro Asp Val Tyr Gly Val Met Val Ala Asn Ala Gln
   210 215 220
   Glu Glu Leu Leu Gln Trp His Ala Ala Asn Ala Lys Asn Asn Pro Lys
   225 230 235 240
   Val Ile His Ala Thr Glu Arg Cys Ala Ala Gly Ile Ile Gln Ala Ile
   245 250 255
   Gly His Ser Asn Leu Gly Pro Ser Thr Ser Pro Arg Asp Val Met Asp
   260 265 270
   Leu Ser Asp Cys Lys Met Glu Asn Phe Val Pro Ala Tyr Glu Val Val
           275                 280                 285
   Lys Phe Tyr Leu Phe Phe Glu Lys Trp Arg Arg Gly Glu Ile Glu His
       290                 295                 300
   Ser Glu His Tyr Leu Ser Asn Leu Lys Ala Val Cys Arg Pro Ser Gly
   305                 310                 315                 320
   Thr Phe Val His Pro Ser Gly Val Glu Lys Ser Leu Gln Glu Cys Val
                   325                 330                 335
   Thr Leu Phe Gly Thr Cys His Gly Asp Lys Gln Gly Lys Gln Phe Arg
               340                 345                 350
   Ile Trp Val Asp Gln Val Leu Pro Val Gln Val Gly Ser Asp Ser Trp
           355                 360                 365
   Leu Val Ser Phe Lys Lys Trp Glu Leu Ser Gly Glu Asp Arg Arg Cys
       370                 375                 380
   Cys Ile Thr Thr Val Leu Leu Ser Ser Lys Asn Lys Thr Val Ala Asp
   385                 390                 395                 400
   Gly Leu Thr Trp Thr His Val His Gln Thr Trp Leu Asn Gly Ala Ala
                   405                 410                 415
   Ala Ser Asp Ser Ala Ser Trp Phe Phe
               420                 425
<210> 3
   <211> 1278
   <212> DNA
   <213> Nicotiana tabacum
<220>
   <221> CDS
   <222> (1) .. (1275)
   <223>
<400> 3
   atg gat cag cta acc agt gcc gca cgt ctc atg ata gtc tca gat ctt        48
   Met Asp Gln Leu Thr Ser Ala Ala Arg Leu Met Ile Val Ser Asp Leu
   1               5                   10                  15
   gac cat acc atg gtt gat cat cat gat cct gag aac ctt tct ctg ctt        96
   Asp His Thr Met Val Asp His His Asp Pro Glu Asn Leu Ser Leu Leu
               20                  25                  30
   agg ttt aat gct tta tgg gag gcc aat tat cgt gaa aac tcc ttg tta       144
   Arg Phe Asn Ala Leu Trp Glu Ala Asn Tyr Arg Glu Asn Ser Leu Leu
           35                  40                  45
   gtg ttc tca act ggg aga tca cct acc ctt tac aag gag ttg aga aaa       192
   Val Phe Ser Thr Gly Arg Ser Pro Thr Leu Tyr Lys Glu Leu Arg Lys
       50                  55                  60
   gag aag ccc atg cta acc cca gat att acc att atg tct gtg ggg act       240
   Glu Lys Pro Met Leu Thr Pro Asp Ile Thr Ile Met Ser Val Gly Thr
   65                  70                  75                  80
   gaa ata act tat ggt aac tct atg gag cca gat gat ggt tgg gaa gca       288
   Glu Ile Thr Tyr Gly Asn Ser Met Glu Pro Asp Asp Gly Trp Glu Ala
                   85                  90                  95
   ttt tta aat gat aag tgg gat cgg aaa ata gtg aca gag gag aca agc       336
   Phe Leu Asn Asp Lys Trp Asp Arg Lys Ile Val Thr Glu Glu Thr Ser
               100                 105                 110
   aaa ttt cct gaa ctc acc ctt cag tca gaa aca gag cag cga cca cac       384
   Lys Phe Pro Glu Leu Thr Leu Gln Ser Glu Thr Glu Gln Arg Pro His
           115                 120                 125
   aag gtc agt ttc tat gtt cag aaa gac aag gct caa gat ata acg gga       432
   Lys Val Ser Phe Tyr Val Gln Lys Asp Lys Ala Gln Asp Ile Thr Gly
       130                 135                 140
   act ctt tcc aag cgc ttg gaa gaa cgt ggg ttg gat gtc aaa ata att         480
   Thr Leu Ser Lys Arg Leu Glu Glu Arg Gly Leu Asp Val Lys Ile Ile
   145                 150                 155                 160
   tat agc gga ggg atg gat ttg gac att ttg cca caa ggt gct ggc aaa         528
   Tyr Ser Gly Gly Met Asp Leu Asp Ile Leu Pro Gln Gly Ala Gly Lys
                   165                 170                 175
   gga cga gca ctt gca tat ttg ctt aag aaa tta aag agt gag ggc aag         576
   Gly Arg Ala Leu Ala Tyr Leu Leu Lys Lys Leu Lys Ser Glu Gly Lys
               180                 185                 190
   tta cca aac aac acg ctt gcc tgt ggt gac tct gga aat gat gct gag         624
   Leu Pro Asn Asn Thr Leu Ala Cys Gly Asp Ser Gly Asn Asp Ala Glu
           195                 200                 205
   ctt ttc agt atc cca gat gtt tat ggt gtg atg gta gcg aat gca cag         672
   Leu Phe Ser Ile Pro Asp Val Tyr Gly Val Met Val Ala Asn Ala Gln
       210                 215                 220
   gag gag tta tta caa tgg cgt gct gca aat gca aaa gat agt cca aaa         720
   Glu Glu Leu Leu Gln Trp Arg Ala Ala Asn Ala Lys Asp Ser Pro Lys
   225                 230                 235                 240
   gta att cat gca aca gag aga tgt gcc gcg ggt ata ata caa gca att         768
   Val Ile His Ala Thr Glu Arg Cys Ala Ala Gly Ile Ile Gln Ala Ile
                   245                 250                 255
   ggg cat ttc aac ctg gga cca aat acc tct cct aga gat gtt aca gat         816
   Gly His Phe Asn Leu Gly Pro Asn Thr Ser Pro Arg Asp Val Thr Asp
               260                 265                 270
   atg tca gac tgc aag atg gag aat ttt gtt cct gct tat gaa gtc gtc         864
   Met Ser Asp Cys Lys Met Glu Asn Phe Val Pro Ala Tyr Glu Val Val
           275                 280                 285
   aaa ttt tac ttg ttt ttc gag aaa tgg agg cgt gga gaa att gag aat         912
   Lys Phe Tyr Leu Phe Phe Glu Lys Trp Arg Arg Gly Glu Ile Glu Asn
       290                 295                 300
   tct gac ctt cac ttg tca aac ctg aaa gca gtt tgt aga cca tcc ggt         960
   Ser Asp Leu His Leu Ser Asn Leu Lys Ala Val Cys Arg Pro Ser Gly
   305                 310                 315                 320
   act ttt gtg cac cca tct gga gtt gag aaa tat ctt gag gac tgc ata        1008
   Thr Phe Val His Pro Ser Gly Val Glu Lys Tyr Leu Glu Asp Cys Ile
                   325                 330                 335
   aat aca ttg aga act tgt cac ggt gac aaa cag ggt aaa caa ttt cgt      1056
   Asn Thr Leu Arg Thr Cys His Gly Asp Lys Gln Gly Lys Gln Phe Arg
                   340                 345                 350
   att tgg gtt gat cta gtg tta cct aca cag gtt ggt tca gat tca tgg      1104
   Ile Trp Val Asp Leu Val Leu Pro Thr Gln Val Gly Ser Asp Ser Trp
           355                 360                 365
   tta gtg agt ttc aag aaa tgg gag ctt tgt ggc gaa gag cga caa tgt      1152
   Leu Val Ser Phe Lys Lys Trp Glu Leu Cys Gly Glu Glu Arg Gln Cys
       370                 375                 380
   tgc ata act act gtc ttg tta agt tca aag aat gtg acg gtc gcg gat      1200
   Cys Ile Thr Thr Val Leu Leu Ser Ser Lys Asn Val Thr Val Ala Asp
   385                 390                 395                 400
   ggg ctc act tgg aca cat gtg cat cag act tgg ctg cag gga gca gca      1248
   Gly Leu Thr Trp Thr His Val His Gln Thr Trp Leu Gln Gly Ala Ala
                   405                 410                 415
   gca agt gac tcc gcg tcc tgg ttc ttt taa                              1278
   Ala Ser Asp Ser Ala Ser Trp Phe Phe
                   420                 425
<210> 4
   <211> 425
   <212> PRT
   <213> Nicotiana tabacum
<400> 4
   Met Asp Gln Leu Thr Ser Ala Ala Arg Leu Met Ile Val Ser Asp Leu
   1               5                   10                  15
   Asp His Thr Met Val Asp His His Asp Pro Glu Asn Leu Ser Leu Leu
               20                  25                  30
   Arg Phe Asn Ala Leu Trp Glu Ala Asn Tyr Arg Glu Asn Ser Leu Leu
           35                  40                  45
   Val Phe Ser Thr Gly Arg Ser Pro Thr Leu Tyr Lys Glu Leu Arg Lys
   50 55 60
   Glu Lys Pro Met Leu Thr Pro Asp Ile Thr Ile Met Ser Val Gly Thr
   65 70 75 80
   Glu Ile Thr Tyr Gly Asn Ser Met Glu Pro Asp Asp Gly Trp Glu Ala
   85 90 95
   Phe Leu Asn Asp Lys Trp Asp Arg Lys Ile Val Thr Glu Glu Thr Ser
   100 105 110
   Lys Phe Pro Glu Leu Thr Leu Gln Ser Glu Thr Glu Gln Arg Pro His
   115 120 125
   Lys Val Ser Phe Tyr Val Gln Lys Asp Lys Ala Gln Asp Ile Thr Gly
   130 135 140
   Thr Leu Ser Lys Arg Leu Glu Glu Arg Gly Leu Asp Val Lys Ile Ile
   145 150 155 160
   Tyr Ser Gly Gly Met Asp Leu Asp Ile Leu Pro Gln Gly Ala Gly Lys
   165 170 175
   Gly Arg Ala Leu Ala Tyr Leu Leu Lys Lys Leu Lys Ser Glu Gly Lys
   180 185 190
   Leu Pro Asn Asn Thr Leu Ala Cys Gly Asp Ser Gly Asn Asp Ala Glu
   195 200 205
   Leu Phe Ser Ile Pro Asp Val Tyr Gly Val Met Val Ala Asn Ala Gln
   210 215 220
   Glu Glu Leu Leu Gln Trp Arg Ala Ala Asn Ala Lys Asp Ser Pro Lys
   225 230 235 240
   Val Ile His Ala Thr Glu Arg Cys Ala Ala Gly Ile Ile Gln Ala Ile
   245 250 255
   Gly His Phe Asn Leu Gly Pro Asn Thr Ser Pro Arg Asp Val Thr Asp
   260 265 270
   Met Ser Asp Cys Lys Met Glu Asn Phe Val Pro Ala Tyr Glu Val Val
   275 280 285
   Lys Phe Tyr Leu Phe Phe Glu Lys Trp Arg Arg Gly Glu Ile Glu Asn
   290 295 300
   Ser Asp Leu His Leu Ser Asn Leu Lys Ala Val Cys Arg Pro Ser Gly
   305 310 315 320
   Thr Phe Val His Pro Ser Gly Val Glu Lys Tyr Leu Glu Asp Cys Ile
   325 330 335
   Asn Thr Leu Arg Thr Cys His Gly Asp Lys Gln Gly Lys Gln Phe Arg
   340 345 350
   Ile Trp Val Asp Leu Val Leu Pro Thr Gln Val Gly Ser Asp Ser Trp
   355 360 365
   Leu Val Ser Phe Lys Lys Trp Glu Leu Cys Gly Glu Glu Arg Gln Cys
   370 375 380
   Cys Ile Thr Thr Val Leu Leu Ser Ser Lys Asn Val Thr Val Ala Asp
   385 390 395 400
   Gly Leu Thr Trp Thr His Val His Gln Thr Trp Leu Gln Gly Ala Ala
   405 410 415
   Ala Ser Asp Ser Ala Ser Trp Phe Phe
   420 425
<210> 5
   <211> 1642
   <212> DNA
   <213> Solanum tuberosum
<220>
   <221> CDS
   <222> (70)..(1344)
   <223>
<400> 5
   acacaaaaaa gatttcattt ttttgtttcc ccaattccca tttcaggttg aagccaattt      60
   acatcaatc atg gat cgg cta acc agt gct gca cgt ctc atg ata gtc tca     111
             Met Asp Arg Leu Thr Ser Ala Ala Arg Leu Met Ile Val Ser
             1               5                   10
   gat ctt gac cat aca atg gta gat cat cac gat tcc gag aac ctt tct       159
   Asp Leu Asp His Thr Met Val Asp His His Asp Ser Glu Asn Leu Ser
   15                  20                  25                  30
   ctg ctt agg ttc aat gct tta tgg gaa gcc aat tat cgt gat aac tct       207
   Leu Leu Arg Phe Asn Ala Leu Trp Glu Ala Asn Tyr Arg Asp Asn Ser
                   35                  40                  45
   ttg tta gtg ttc tct act ggg aga tca cct aca ctt tac aag gaa tta       255
   Leu Leu Val Phe Ser Thr Gly Arg Ser Pro Thr Leu Tyr Lys Glu Leu
               50                  55                  60
   agg aaa gaa aag ccc atg cta acc cca gat att aca att atg tct gtg       303
   Arg Lys Glu Lys Pro Met Leu Thr Pro Asp Ile Thr Ile Met Ser Val
           65                  70                  75
   gga act gaa ata aca tat ggt aac gct atg gtg cct gat gat ggt tgg       351
   Gly Thr Glu Ile Thr Tyr Gly Asn Ala Met Val Pro Asp Asp Gly Trp
       80                  85                  90
   gaa aca ttt ctg aat aac aag tgg gat aga aag ata gta aca gag gag        399
   Glu Thr Phe Leu Asn Asn Lys Trp Asp Arg Lys Ile Val Thr Glu Glu
   95                  100                 105                 110
   aca agc aag ttt cct gaa ctc agt ctg cag tca gaa aca gag cag cga        447
   Thr Ser Lys Phe Pro Glu Leu Ser Leu Gln Ser Glu Thr Glu Gln Arg
                   115                 120                 125
   cca cac aag gtc agt ttc tat gtt cag aaa gag aaa gct caa gat ata        495
   Pro His Lys Val Ser Phe Tyr Val Gln Lys Glu Lys Ala Gln Asp Ile
               130                 135                 140
   atg aaa act ctt tcc aag cgc ttg gaa gaa cgt ggg ctg gat gtc aaa        543
   Met Lys Thr Leu Ser Lys Arg Leu Glu Glu Arg Gly Leu Asp Val Lys
           145                 150                 155
   ata att tac agt gga ggg atg gat cta gat ata tta cca cag ggt gct        591
   Ile Ile Tyr Ser Gly Gly Met Asp Leu Asp Ile Leu Pro Gln Gly Ala
       160                 165                 170
   ggc aaa gga caa gca ctt gca tat ctg ctt aag aaa ctg aag agc gag        639
   Gly Lys Gly Gln Ala Leu Ala Tyr Leu Leu Lys Lys Leu Lys Ser Glu
   175                 180                 185                 190
   gga aaa tta cca agc aac acc ctt gcc tgc ggc gac tcc ggg aat gac        687
   Gly Lys Leu Pro Ser Asn Thr Leu Ala Cys Gly Asp Ser Gly Asn Asp
                   195                 200                 205
   gct gaa tta ttc agt atc cca gat gtg tat ggt gta atg gta gct aat        735
   Ala Glu Leu Phe Ser Ile Pro Asp Val Tyr Gly Val Met Val Ala Asn
               210                 215                 220
   gcg cag aag gaa tta ctg cag tgg cat gct gca aat gca aaa aat aat        783
   Ala Gln Lys Glu Leu Leu Gln Trp His Ala Ala Asn Ala Lys Asn Asn
           225                 230                 235
   ccc aaa gta att cat gca tca gag agg tgt gcc gcc ggt atc ata caa        831
   Pro Lys Val Ile His Ala Ser Glu Arg Cys Ala Ala Gly Ile Ile Gln
       240                 245                 250
   gcc att ggt cat ttc aaa cta ggt cca agt acc tcc cca aga gac gtt        879
   Ala Ile Gly His Phe Lys Leu Gly Pro Ser Thr Ser Pro Arg Asp Val
   255                 260                 265                 270
   acg gat ttg tca gat tgc aag atg gac aac ttt gtt cct gcc tat gaa        927
   Thr Asp Leu Ser Asp Cys Lys Met Asp Asn Phe Val Pro Ala Tyr Glu
                   275                 280                 285
   gtt gtc aaa ttt tac ctg ttt ttt gag aaa tgg agg cgt gga gaa att      975
   Val Val Lys Phe Tyr Leu Phe Phe Glu Lys Trp Arg Arg Gly Glu Ile
               290                 295                 300
   gag cat tct gag cat tat ctg cca aac ctg aaa gca gtg tgt ata cca     1023
   Glu His Ser Glu His Tyr Leu Pro Asn Leu Lys Ala Val Cys Ile Pro
           305                 310                 315
   tct ggt act ttt gtt cac cca tct ggt gtt gag aaa tcc ctt cag gaa     1071
   Ser Gly Thr Phe Val His Pro Ser Gly Val Glu Lys Ser Leu Gln Glu
       320                 325                 330
   tgt gta act tca ttc gga aca tgt cat gct gac aag cag ggg aaa caa     1119
   Cys Val Thr Ser Phe Gly Thr Cys His Ala Asp Lys Gln Gly Lys Gln
   335                 340                 345                 350
   tat cgt gtt tgg gtc gat caa gtt tta cct tca cag gtt ggt tca gac     1167
   Tyr Arg Val Trp Val Asp Gln Val Leu Pro Ser Gln Val Gly Ser Asp
                   355                 360                 365
   tca tgg tta gtg agt ttc aag aag tgg gag ctc tct ggt gaa gac atg     1215
   Ser Trp Leu Val Ser Phe Lys Lys Trp Glu Leu Ser Gly Glu Asp Met
               370                 375                 380
   cga tgc tgc ata acc aca gtc cta tta agt tca aag aat aag act gtt     1263
   Arg Cys Cys Ile Thr Thr Val Leu Leu Ser Ser Lys Asn Lys Thr Val
           385                 390                 395
   gca gac ggg ctc act tgg act cac gta cat cag aca tgg ctg cac ggt     1311
   Ala Asp Gly Leu Thr Trp Thr His Val His Gln Thr Trp Leu His Gly
       400                 405                 410
   gat gca gca agt gac tcc gca acc tgg ttc ttt tagattgtca tctcagtgta   1364
   Asp Ala Ala Ser Asp Ser Ala Thr Trp Phe Phe
   415                 420                 425
   ttaactctga aaattccgca ccccttttac cagttcacac ccagaataaa cacaacatac   1424
   aaactatagt tgataatcaa tgtattaact ttctccttct ttgataatca atgtattgcc   1484
   atctaaacca gtgaagatgg ctttatcttt tgtgtagtat aaagaattat attagtatca   1544
   tagttgttct tgtatttgat tcagaattca agatgagatt gttgcaaatt gctgcatatt   1604
   taagtttcca aaaaaaaaaa aaaaaaaaaa aaaaaaaa                            1642
<210> 6
   <211> 425
   <212> PRT
   <213> Solanum tuberosum
<400> 6
   Met Asp Arg Leu Thr Ser Ala Ala Arg Leu Met Ile Val Ser Asp Leu
   1               5                   10                  15
   Asp His Thr Met Val Asp His His Asp Ser Glu Asn Leu Ser Leu Leu
               20                  25                  30
   Arg Phe Asn Ala Leu Trp Glu Ala Asn Tyr Arg Asp Asn Ser Leu Leu
           35                  40                  45
   Val Phe Ser Thr Gly Arg Ser Pro Thr Leu Tyr Lys Glu Leu Arg Lys
       50                  55                  60
   Glu Lys Pro Met Leu Thr Pro Asp Ile Thr Ile Met Ser Val Gly Thr
   65                  70                  75                  80
   Glu Ile Thr Tyr Gly Asn Ala Met Val Pro Asp Asp Gly Trp Glu Thr
                   85                  90                  95
   Phe Leu Asn Asn Lys Trp Asp Arg Lys Ile Val Thr Glu Glu Thr Ser
               100                 105                 110
   Lys Phe Pro Glu Leu Ser Leu Gln Ser Glu Thr Glu Gln Arg Pro His
           115                 120                 125
   Lys Val Ser Phe Tyr Val Gln Lys Glu Lys Ala Gln Asp Ile Met Lys
       130                 135                 140
   Thr Leu Ser Lys Arg Leu Glu Glu Arg Gly Leu Asp Val Lys Ile Ile
   145 150 155 160
   Tyr Ser Gly Gly Met Asp Leu Asp Ile Leu Pro Gln Gly Ala Gly Lys
   165 170 175
   Gly Gln Ala Leu Ala Tyr Leu Leu Lys Lys Leu Lys Ser Glu Gly Lys
   180 185 190
   Leu Pro Ser Asn Thr Leu Ala Cys Gly Asp Ser Gly Asn Asp Ala Glu
   195 200 205
   Leu Phe Ser Ile Pro Asp Val Tyr Gly Val Met Val Ala Asn Ala Gln
   210 215 220
   Lys Glu Leu Leu Gln Trp His Ala Ala Asn Ala Lys Asn Asn Pro Lys
   225 230 235 240
   Val Ile His Ala Ser Glu Arg Cys Ala Ala Gly Ile Ile Gln Ala Ile
   245 250 255
   Gly His Phe Lys Leu Gly Pro Ser Thr Ser Pro Arg Asp Val Thr Asp
   260 265 270
   Leu Ser Asp Cys Lys Met Asp Asn Phe Val Pro Ala Tyr Glu Val Val
   275 280 285
   Lys Phe Tyr Leu Phe Phe Glu Lys Trp Arg Arg Gly Glu Ile Glu His
   290 295 300
   Ser Glu His Tyr Leu Pro Asn Leu Lys Ala Val Cys Ile Pro Ser Gly
   305 310 315 320
   Thr Phe Val His Pro Ser Gly Val Glu Lys Ser Leu Gln Glu Cys Val
   325 330 335
   Thr Ser Phe Gly Thr Cys His Ala Asp Lys Gln Gly Lys Gln Tyr Arg
   340 345 350
   Val Trp Val Asp Gln Val Leu Pro Ser Gln Val Gly Ser Asp Ser Trp
   355 360 365
   Leu Val Ser Phe Lys Lys Trp Glu Leu Ser Gly Glu Asp Met Arg Cys
   370 375 380
   Cys Ile Thr Thr Val Leu Leu Ser Ser Lys Asn Lys Thr Val Ala Asp
   385 390 395 400
   Gly Leu Thr Trp Thr His Val His Gln Thr Trp Leu His Gly Asp Ala
   405 410 415
   Ala Ser Asp Ser Ala Thr Trp Phe Phe
   420 425
<210> 7
   <211> 199
   <212> DNA
   <213> Solanum tuberosum
<400> 7
   gtacggaccg tactactcta ttcgtttcaa tatatttatt tgtttcagct gactgcaaga 60
   ttcaaaaatt tctttattat tttaaatttt gtgtcactca aaaccagata aacaatttga 120
   tatagagcca ctatatatat acatattctc gattatatat gtaaatgagt tacccttttt 180
   ttccacttaa attatatag 199
<210> 8
   <211> 26
   <212> DNA
   <213> Primer
<400> 8
   atggatcagc taaccagtcg ccgcac 26
<210> 9
   <211> 27
   <212> DNA
   <213> Primer
<400> 9
   ctaaaagaac caggacgcgg agtcact 27
<210> 10
   <211> 47
   <212> DNA
   <213> Primer
<400> 10
   cctgcaggct cgagactagt agatctggta cggaccgtac tactcta 47
<210> 11
   <211> 48
   <212> DNA
   <213> Primer
<400> 11
   cctgcagggt cgactctaga ggatccccta tataatttaa gtggaaaa 48
<210> 12
   <211> 27
   <212> DNA
   <213> Primer
<400> 12
   ggatccatgg atcagctaac cagtgcc 27
<210> 13
   <211> 28
   <212> DNA
   <213> Primer
<400> 13
   gtcgactacc attacaccat aacacatc 28
<210> 14
   <211> 27
   <212> DNA
   <213> Primer
<400> 14
   gttagtgttc tcaactggga gatcacc 27
<210> 15
   <211> 28
   <212> DNA
   <213> Primer
<400> 15
   cccatttctt gaaactcact aaccatga 28
<210> 16
   <211> 23
   <212> DNA
   <213> Primer
<400> 16
   atggcagacg gtgaggatat tca 23
<210> 17
   <211> 23
   <212> DNA
   <213> Primer
<400> 17
   gcctttgcaa tccacatctg ttg 23
<210> 18
   <211> 27
   <212> DNA
   <213> Primer
<400> 18
   ggatccatgg atcagctaac cagtgcc 27
<210> 19
   <211> 33
   <212> DNA
   <213> Primer
<400> 19
   gtcgacctaa aagaaccagg acgcggagtc act 33

## Beschreibung

Die vorliegende Erfindung betrifft die Verwendung eines Polypeptides mit der biologischen Aktivität einer Saccharose-6-Phosphat Phosphatase, welches bei Nichtanwesenheit Wachstumsretardierungen sowie chlorotische Blätter bedingt und durch die Nukleinsäuresequenzen SEQ ID NO:1, SEQ ID NO:3, SEQ ID NO:5 oder ein funktionelles Äquivalent SEQ ID NO:1, SEQ ID NO:3 oder SEQ ID NO:5 kodiert wird, als Target für Herbizide. Des weiteren betrifft die vorliegende Erfindung die Verwendung des vorstehend genannten Polypeptides in einem Verfahren zur Identifizierung von Verbindungen mit herbizider oder wachstumsregulatorischer Wirkung, welche Saccharose-6-Phosphat Phosphatase irihibieren. Des weiteren betrifft die Erfindung die Verwendung dieser über das Verfahren identifizierten Verbindungen als Herbizide oder Wachstumsregulatoren.

Das grundlegende Prinzip, Herbizide über Inhibierung eines definierten Targets zu identifizieren ist bekannt (z.Bsp. US 5,187,071, WO 98/33925, WO 00/77185, WO 01/79514). Generell besteht ein großer Bedarf, Enzyme zu detektieren, welche neue Targets für Herbizide darstellen könnten. Gründe hierfür sind auftretende Resistenzproblematiken von an bereits bekannten Targets wirkenden herbiziden Wirkstoffen und das ständige Bemühen neue herbizide Wirkstoffe zu identifizieren, die sich durch einen möglichst breiten Wirkungsbereich, ökologische und toxikologische Verträglichkeit und/oder geringe Aufwandmengen auszeichnen.

Die Detektion von neuen Targets ist in der Praxis mit großen Schwierigkeiten verbunden, da die Hemmung eines Enzyms, das Bestandteil eines Stoffwechselweges ist, häufig das Wachstum der Pflanze nicht weiter beeinflusst. Dies kann daran liegen, dass die Pflanze auf alternative Stoffwechselwege ausweicht, deren Existenz nicht bekannt ist, oder dass das inhibierte Enzym nicht limitierend für den Stoffwechselweg ist. Ferner zeichnen sich pflanzliche Genome durch eine große funktionelle Redundanz aus. Im Genom von Arabidopsis thaliana liegen funktional äquivalente Enzyme im Vergleich zu Insekten oder Säugern häufiger in Genfamilien vor (Nature, 2000, 408(6814):796-815). Diese Annahme wird experimentell bestätigt durch die Tatsache, dass grosse Gen-Knock-out-Programme durch T-DNA- oder Transposoninsertion in Arabidopsis bisher weniger ausgeprägte Phänotypen lieferten als erwartet (Curr. Op. Plant Biol. 4, 2001, pp.111-117).

Lunn et al., 2003, "Gene: An International Journal On Genes And Genomes, 303, S. 187-196)" beschreiben die Charakterisierung von Sucrose-Phosphatase aus mehreren Pflanzen, ohne jedoch einen Zusammenhang zur Targeteignung herzustellen.

Die Aufgabe der vorliegenden Erfindung besteht daher darin, neue Targets zu identifizieren, die für das Wachstum von Pflanzen essentiell sind bzw. deren Inhibierung für die Pflanze zu einem verminderten Wachstum führen, sowie Verfahren bereitzustellen, welche zur Identifizierung von Verbindungen mit herbizider Wirkung geeignet sind.

Die Aufgabe wurde gelöst durch die Verwendung von einem Polypeptid mit der biologischen Aktivität einer Saccharose-6-Phosphat Phosphatase kodiert durch eine Nukleinsäuresequenz umfassend
a) eine Nukleinsäuresequenz mit der in SEQ ID NO:1; SEQ ID NO:3 oder SEQ ID NO:5 dargestellten Nukleinsäuresequenz; oder
b) eine Nukleinsäuresequenz, die sich aufgrund des degenerierten genetischen Codes durch Rückübersetzung aus der in SEQ ID NO:2, SEQ ID NO:4 oder SEQ ID NO:6 dargestellten Aminosäuresequenz ableiten läßt; oder
c) funktionelle Äquivalente der Nukleinsäuresequenz SEQ ID NO:1 mit einer Identität von mindestens 55% zu der SEQ ID NO:1; oder funktionelle Äquivalente der Nukleinsäuresequenz SEQ ID NO:3 mit einer Identität von mindestens 55% zu der SEQ ID NO:3; oder funktionelle Äquivalente der Nukleinsäuresequenz SEQ ID NO:5 mit einer Identität von mindestens 51% zu der SEQ ID NO:5; oder
d) eine Nukleinsäuresequenz, die sich aufgrund des degenerierten genetischen Codes durch Rückübersetzung der Aminosäuresequenz eines funktionellen Äquivalents der SEQ ID NO:2, das eine Identität mit der SEQ ID NO:2 von mindestens 55% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:4, das eine Identität mit der SEQ ID NO:4 von mindestens 54% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:6, das eine Identität mit der SEQ ID NO:6 von mindestens 54% aufweist, ableiten läßt;
als Target für Herbizide.

An dieser Stelle werden nun weitere der in der Beschreibung verwendeten Begriffe definiert.

"Affinitäts-Tag": Bezeichnet ein Peptid oder Polypeptid, dessen kodierende Nukleinsäuresequenz mit der erfindungsgemäßen Nukleinsäuresequenz direkt oder mittels eines Linkers über gängige Klonierungstechniken fusioniert werden kann. Das Affinitäts-Tag dient zur Isolierung, Anreicherung und/oder gezielten Aufreinigung des rekombinanten Zielproteins mittels Affinitäts-Chromatographie aus Gesamtzellextrakten. Der oben erwähnte Linker kann vorteilhaft eine Protease-Schnittstelle (z.B. für Thrombin oder Faktor Xa) enthalten, wodurch das Affinitäts-Tag bei Bedarf vom Zielprotein abgespalten werden kann. Beispiele für gängige Affinitäts-Tags sind das "His-Tag" z.B. von Quiagen, Hilden, "Strep-Tag", das "Myc-Tag" (Invitrogen, Carlsberg), das aus einer Chitin bindenden Domäne und einem Intein bestehende Tag von New England Biolabs, das Maltose-bindende Protein (pMal) von New England Biolabs und das sogenannte CBD-Tag von Novagen. Der Affinitäts-Tag kann dabei am 5'- oder 3'-Ende der kodierenden Nukleinsäuresequenz mit der für das Zielprotein kodierenden Sequenz angebracht sein.

"Expressionskassette": Eine Expressionskassette enthält eine erfindungsgemäße Nukleinsäuresequenz funktionell verknüpft mit mindestens einem genetischen Kontrollelement, wie einem Promotor, sowie vorteilhaft mit einem weiteren Kontrollelement, wie einem Terminator. Die Nukleinsäuresequenz der Expressionskassette kann beispielsweise eine genomische oder eine komplementäre DNA-Sequenz oder eine RNA-Sequenz sowie halb- oder vollsynthetische Analoga davon sein. Diese Sequenzen können in linearer oder zirkulärer Form, extra-chromosomal oder integriert in das Genom vorliegen. Die entsprechenden Nukleinsäuresequenzen können synthetisch hergestellt oder natürlich gewonnen werden oder eine Mischung aus synthetischen und natürlichen DNA-Bestandteilen enthalten, sowie aus verschiedenen heterologen Genabschnitten verschiedener Organismen bestehen.

Auch artifizielle Nukleinsäuresequenzen sind hierbei geeignet, solange sie die Expression eines durch eine erfindungsgemäße Nukleinsäuresequenz kodierten Polypeptides mit der biologischen Aktivität einer Saccharose-6-Phosphat Phosphatase in einer Zelle oder einem Organismus ermöglichen. Beispielsweise können synthetische Nukleotid-Sequenzen erzeugt werden, die bezüglich der Kodon-Nutzung des von den zu transformierenden Organismen optimiert wurden.

Alle vorstehend erwähnten Nukleotid-Sequenzen sind in an sich bekannter Weise durch chemische Synthese aus den Nukleotidbausteinen wie beispielsweise durch Fragment-Kondensation einzelner überlappender, komplementärer Nukleotidbausteine der Doppelhelix herstellbar. Die chemische Synthese von Oligonukleotiden kann beispielsweise in bekannter Weise nach der Phosphoamiditmethode (Voet, Voet, 2. Auflage, Wiley Press New York, Seite 896-897) erfolgen. Bei der Präparation einer Expressionskassette können verschiedene DNA-Fragmente so manipuliert werden, dass eine Nukleotid-Sequenz mit korrekter Leserichtung und korrektem Leseraster erhalten wird. Die Verbindung der Nukleinsäure-Fragmente untereinander erfolgt über allgemeine Klonierungstechniken wie sie beispielsweise in T. Maniatis, E.F. Fritsch und J. Sambrook, "Molecuiar Cioning: A Laboratory Manual", Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., "Current Protocols in Molecular Biology", Greene Publishing Assoc. and Wiley-Interscience (1994) beschrieben sind.

"Funktionelle Verknüpfung": Unter einer funktionellen oder operativen Verknüpfung versteht man die sequenzielle Anordnung regulativer Sequenzen bzw. genetischer Kontrollelemente derart, daß jede der regulativen Sequenzen bzw. jedes der genetischer Kontrollelemente ihre Funktion bei der Expression der kodierenden Sequenz bestimmungsgemäß erfüllen kann.

"Funktionelle Äquivalente" beschreiben hier Nukleinsäuresequenzen, die unter Standardbedingungen mit der Nukleinsäuresequenz SEQ ID NO:1, SEQ ID NO:3 oder SEQ ID NO:5 oder Teilen der SEQ ID NO:1, SEQ ID NO:3 oder SEQ ID NO:5 hybridisieren und befähigt sind, die Expression eines Polypeptides mit der biologischen Aktivität einer Saccharose-6-Phosphat Phosphatase in einer Zelle oder einem Organismus zu bewirken.

Zur Hybrisierung werden vorteilhaft kurze Oligonukleotide mit einer Länge von etwa 10-50 bp, vorzugsweise 15-40 bp beispielsweise der konservierten oder sonstigen Bereiche, die über Vergleiche mit anderen verwandten Genen in dem Fachmann bekannter Weise ermittelt werden können, verwendet. Es können aber auch längere Fragmente der erfindungsgemäßen Nukleinsäuren mit einer Länge von 100-500 bp oder die vollständigen Sequenzen für die Hybridisierung verwendet werden. Je nach der verwendeten Nukleinsäure/Oligonukleotid, der Länge des Fragmentes oder der vollständige Sequenz oder je nachdem welche Nukleinsäureart, d.h. DNA oder RNA, für die Hybridisierung verwendet werden, variieren diese Standardbedingungen. So liegen beispielsweise die Schmelztemperaturen für DNA:DNA-Hybride ca 10°C niedriger als die von DNA:RNA-Hybriden gleicher Länge.

Unter Standardhybridisierungsbbedingungen sind beispielsweise je nach Nukleinsäure Temperaturen zwischen 42 und 58°C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50 % Formamid wie beispielsweise 42°C in 5 x SSC, 50 % Formamid zu verstehen. Vorteilhafterweise liegen die Hybridisierungsbedingungen für DNA:DNA-Hybride bei 0,1 x SSC und Temperaturen zwischen etwa 20°C bis 65°C, bevorzugt zwischen etwa 30°C bis 45°C. Für DNA:RNA-Hybride liegen die Hybridisierungsbedingungen vorteilhaft bei 0,1 x SSC und Temperaturen zwischen etwa 30°C bis 65°C, bevorzugt zwischen etwa 45°C bis 55°C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehait von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, "Current Protocols in Molecular Biology", John Wiley & Sons, New York; Hames and Higgins (eds), 1985, "Nucleic Acids Hybridization: A Practical Approach", IRL Press at Oxford University Press, Oxford; Brown (ed), 1991, Essential Molecular Biology: A Practical Approach, IRL Press at Oxford University Press, Oxford.

Unter einem funktionellen Äquivalent der SEQ ID NO:1, SEQ ID NO:3 oder SEQ ID NO:5 versteht man weiterhin auch Nukleinsäuresequenzen die mit der SEQ ID NO:1, SEQ ID NO:3 oder SEQ ID NO:5 bis zu einem definierten Prozentsatz homolog bzw. identisch sind und ferner insbesondere auch natürliche oder künstliche Mutationen der vorstehend genannten Nukleinsäuresequenzen, die für ein Polypeptid mit der biologischen Aktivität einer Saccharose-6-Phosphat Phosphatasekodieren.

Es werden beispielsweise auch solche Nukleotidsequenzen durch die vorliegende Erfindung mit umfasst, welche man durch Modifikation der vorstehend genannten Nukleinsäuresequenzen erhält. Beispielhaft können solche Modifikationen durch dem Fachmann geläufige Techniken, wie "Site Directed Mutagenesis", "Error Prone PCR", "DNAshuffling" (Nature 370, 1994, pp.389-391) oder "Staggered Extension Process" (Nature Biotechnol. 16, 1998, pp.258-261) erzeugt werden. Ziel einer solchen Modifikation kann z.B. die Einfügung weiterer Restriktionsenzymschnittstellen, die Entfernung von DNA zur Verkürzung der Sequenz, der Austausch von Nukleotiden zur Codon-Optimierung oder das Hinzufügen weiterer Sequenzen sein. Proteine, die über modifizierte Nukleinsäuresequenzen kodiert werden, müssen trotz abweichender Nukleinsäuresequenz noch die gewünschten Funktionen besitzen.

Der Begriff des funktionellen Äquivalents kann sich auch auf das durch die entsprechende Nukleinsäuresequenz kodierte Aminosäuresequenz beziehen. In diesem Fall beschreibt der Begriff funktionelles Äquivalent ein Protein, dessen Aminosäuresequenz mit der SEQ ID NO:2, SEQ ID NO:4 oder SEQ ID NO:6 bis zu einem definierten Prozentsatz identisch bzw. homolog ist.

Funktionelle Äquivalente umfassen somit natürlich vorkommende Varianten der hierin beschriebenen Sequenzen sowie künstliche, z.B. durch chemische Synthese erhaltene, an den Kodon-Gebrauch adaptierte Nukleinsäuresequenzen bzw. die davon abgeleiteten Aminosäuresequenzen.

"Genetische Kontrollsequenz" beschreibt Sequenzen, die einen Einfluss auf die Transkription und gegebenenfalls Translation der erfindungsgemäßen Nukleinsäuren in prokaryotischen oder eukaryontischen Organismen haben. Beispiele hierfür sind Promotoren, Terminatoren oder sogenannte "enhancer" Sequenzen. Zusätzlich zu diesen Kontrollsequenzen oder anstelle dieser Sequenzen kann die natürliche Regulation dieser Sequenzen vor den eigentlichen Strukturgenen noch vorhanden sein und gegebenenfalls genetisch so modifiziert worden sein, dass die natürliche Regulation ausgeschaltet und die Expression des Zielgens modifiziert, also erhöht oder erniedrigt wurde. Die Auswahl der Kontrollsequenz erfolgt abhängig vom Wirtsorganismus oder Ausgangsorganismus. Genetische Kontrollsequenzen umfassen ferner auch die 5'-untranslatierte Region, Introns oder die nichtkodierende 3'-Region von Genen. Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Genetische Kontrollsequenzen umfassen auch weitere Promotoren, Promotorelemente oder Minimalpromotoren, sowie die Chromatinstruktur beeinflussende Sequenzen (z.B. Matrix attachment regions (MAR's)) die die expressionssteuernden Eigenschaften modifizieren können. So kann durch genetische Kontrollsequenzen zum Beispiel die gewebespezifische Expression zusätzlich abhängig von bestimmten Stressfaktoren erfolgen. Entsprechende Elemente sind zum Beispiel für Wasserstress, Abscisinsäure (Lam E und Chua NH, J Biol Chem 1991; 266(26): 17131 -17135), Kälte- und Trockenstress (Plant Cell 1994, (6): 251-264) und Hitzestress (Molecular & General Genetics, 1989, 217(2-3): 246-53) beschrieben.

"Homologie" zwischen zwei Nukleinsäuresequenzen oder Polypeptidsequenzen wird durch die Identität der Nukleinsäuresequenz/Polypeptidsequenz über die jeweils gesamte Sequenzlänge definiert, die durch Vergleich mit Hilfe des Programmalgorithmus ClustalW (Thompson, J.D., Higgins, D.G. and Gibson, T.J. (1994) Nucleic Acids Res. 22:4673-80) unter Einstellung folgender Parameter:

| | |
|---|---|
| GAP Penalty 15.00 | DNA transition weight: 0.5 |
| GAP Length Penalty 6.66 | Protein weight matrix: Gonnet Series |
| Delay divergent Seqs (%) 30 | DNA weight matrix: IUB |

berechnet wird.

Anstelle des Begriff "homolog" oder "Homologie" wird im Folgenden auch gleichbedeutend der Begriff Identität verwendet.

"Mutationen" von Nuklein- oder Aminosäuresequenzen umfassen Substitutionen (=Ersetzungen), Additionen (Hinzufügung), Deletionen (Löschung), Inversion (Veränderungen) oder Insertionen (Einfügungen) eines oder mehrerer Nukleotidreste, wodurch sich auch die entsprechende Aminosäuresequenz des Zielproteins mittels Substitution, Insertion oder Deletion einer oder mehrerer Aminosäuren verändern kann, wobei jedoch insgesamt die funktionellen Eigenschaften des Zielproteins im wesentlichen beibehalten werden.

"Natürliche genetische Umgebung" meint den natürlichen chromosomalen Locus in dem Herkunftsorganismus. Im Fall einer genomischen Bibliothek ist die natürliche genetische Umgebung der Nukleinsäuresequenz bevorzugt zumindest noch teilweise erhalten. Die Umgebung flankiert die Nukleinsäuresequenz zumindest an 5'- oder 3'-Seite und hat eine Sequenzlänge von mindestens 50 bp, bevorzugt mindestens 100 bp, besonders bevorzugt mindestens 500 bp, ganz besonders bevorzugt mindestens 1000 bp, am meisten bevorzugt mindestens 5000 bp.

"Pflanzen" im Sinne der Erfindung sind Pflanzenzellen, -gewebe, -organe oder ganzen Pflanzen wie Samen, Knollen, Blüten, Pollen, Früchte, Sämlinge, Wurzeln, Blätter, Stengel oder sonstige Pflanzenteile zu verstehen. Außerdem ist unter Pflanzen Vermehrungsmaterial wie Samen, Früchte, Sämlinge, Stecklinge, Knollen, Schnitte oder Wurzelstöcke zu verstehen.

"Reaktionszeit" bezeichnet die Zeit, die man für die Durchführung eines Aktivitätstests bis zum Erhalt einer signifikanten Aussage über eine Aktivität benötigt und hängt sowohl von der spezifischen Aktivität des im Test eingesetzten Proteins als auch von der verwendeten Methode und der Empfindlichkeit der verwendeten Geräte ab. Dem Fachmann ist die Ermittlung der Reaktionszeiten bekannt. Bei auf photometrischen Methoden basierenden Verfahren zur Identifizierung von Verbindungen mit herbizider Wirkung liegen die Reaktionszeiten beispielsweise im allgemeinen zwischen > 0 bis 120 Minuten.

"Rekombinante DNA" beschreibt eine Kombination von DNA-Sequenzen herstellbar durch rekombinante DNA-Technologie.

"Rekombinate DNA-Technologie": allgemein bekannte Techniken zur Fusionierung von DNA-Sequenzen (z.B. beschrieben in Sambrook et al., 1989, Cold Spring Harbor, NY, Cold Spring Harbor Laboratory Press).

"Replikationsursprünge" gewährleisten die Vermehrung der erfindungsgemäßen Expressionskassetten oder Vektoren in Mikroorganismen und Hefen z.B. der pBR322 ori oder der P15A ori in E. coli (Sambrook et al.: "Molecular Cloning. A Laboratory Manual", 2nd ed. Cold Spring Harbor Laboratory Press, Cold Spring Harbor, NY, 1989) und der ARS1 ori in Hefe (Nucleic Acids Research, 2000, 28(10): 2060-2068).

"Reportergene" kodieren für leicht quantifizierbare Proteine. Über Wachstums-, Fluoreszenz-, Chemo-, Biolumineszenz- oder Resistenzassay oder über eine photometrische Messung (Eigenfarbe) oder Enzymaktivität kann mittels dieser Gene eine Bewertung der Transformationseffizienz oder des Expressionsortes oder -zeitpunktes vorgenommen werden. Ganz besonders bevorzugt sind dabei Reporter-Proteine (Schenbom E, Groskreutz D. Mol Biotechnol. 1999; 13(1):29-44) wie das "green fluorescence protein" (GFP) (Gerdes HH and Kaether C, FEBS Lett. 1996; 389(1):44-47; Chui WL et al., Curr Biol 1996, 6:325-330; Leffel SM et al., Biotechniques. 23(5):912-8, 1997), die Chloramphenicolacetyltransferase, eine Luziferase (Giacomin, Plant Sci 1996, 116:59-72; Scikantha, J Bact 1996, 178:121; Millar et al., Plant Mol Biol Rep 1992 10:324-414), sowie Luziferasegene, im allgemeinen die β-Galactosidase oder die β-Glucuronidase (Jefferson et al., EMBO J. 1987, 6, 3901-3907) oder das Ura3-Gen.

"Selektionsmarker" verleihen eine Resistenz gegen Antibiotika, oder andere toxische Verbindungen: Beispielhaft zu nennen seien hier das Neomycin-Phosphotransferase-Gen, das eine Resistenz gegen die Aminoglycosid-Antibiotika Neomycin (G 418), Kanamycin, Paromycin (Deshayes A et al., EMBO J. 4 (1985) 2731-2737), das sul Gen kodierend für eine mutierte Dihydropteroat Synthase (Guerineau F et al., Plant Mol Biol. 1990; 15(1):127-136), das Hygromycin B Phosphotransferase-Gen (Gen Bank Accession NO: K 01193) und das shble Resistenzgen, das eine Resistenz gegen die Bleomycin Antibiotika wie zB. Zeocin verleiht. Weitere Beispiele für Selektionsmarker-Gene sind Gene, die eine Resistenz gegen 2-Desoxyglucose-6-phosphat (WO 98/45456) oder Phosphinotricin etc. verleihen oder solche, die eine Antimetaboliten-Resistenz verleihen, zum Beispiel das dhfr-Gen (Reiss, Plant Physiol. (Life Sci. Adv.) 13 (1994) 142-149). Geeignet sind ferner Gene wie trpB oder hisD (Hartman SC and Mulligan RC, Proc Natl Acad Sci U S A. 85 (1988) 8047-8051). Geeignet ist auch das Gen der Mannose-Phosphat Isomerase (WO 94/20627), das ODC (Omithin-Decarboxylase) Gen (McConlogue, 1987 in: Current Communications in Molecular Biology, Cold Spring Harbor Laboratory, Hrsg.) oder die Deaminase aus Aspergillus terreus (Tamura K etal., Biosci Biotechnol Biochem. 59 (1995) 2336-2338).

"Transformation" beschreibt einen Prozess zur Einführung heterologer DNA in eine pro- oder eukaryontische Zelle. Mit einer transformierten Zelle ist nicht nur das Produkt das Transformationsprozesses an sich beschrieben, sondern auch alle transgenen Nachkommen des durch die Transformation hergestellten transgenen Organismus

"Target/Target Protein": ein Polypeptid codiert über die erfindungsgemäße Nukleinsäuresequenz, welches ein Enzym im klassischen Sinne sein kann oder z.B. ein Strukturprotein, ein für Entwicklungsprozesse relevantes Protein, Regulationsproteine wie Transkriptionsfaktoren, Kinasen, Phosphatasen, Rezeptoren, Untereinheiten von Kanälen, Transportproteine, regulatorische Untereinheiten die einem Enzymkomplex eine substrat- oder Aktivitätsregulation verleihen. Allen Targets oder Wirkorten gemein ist dabei, dass deren funktionale Anwesenheit essentiell für das Überleben oder die normale Entwicklung und das Wachstum sind.

"Transgen": Bezogen auf eine Nukleinsäuresequenz, eine Expressionskassette oder einen Vektor enthaltend eine erfindungsgemäße Nukleinsäuresequenz oder einen Organismus transformiert mit der vorstehend genannten Nukleinsäuresequenz, Expressionskassette oder Vektor beschreibt der Ausdruck transgen alle solche durch gentechnische Methoden hergestellten Konstruktionen, in denen sich entweder die Nukleinsäuresequenz des Zielproteins oder eine mit der Nukleinsäuresequenz des Zielproteins funktionell verknüpfte genetische Kontrollsequenz oder eine Kombination der vorstehend genannten Möglichkeiten sich nicht in ihrer natürlichen genetischen Umgebung befinden oder durch gentechnische Methoden modifiziert wurden. Die Modifikation kann hier beispielsweise über Mutation eines oder mehrerer Nukleotidreste der entsprechenden Nukleinsäuresequenz erreicht werden.

Saccharose-6-phosphat Phosphatase, ein im Cytosol lokalisiertes Enzym der Saccharose-Biosynthese, katalysiert die Umwandlung von Saccharose-6-phosphat zu Orthophosphat und Saccharose. Ein Enzym mit der biologischen Aktivität einer Saccharose-6-phosphat Phosphatase beschreibt somit ein Enzym, welches in der Lage ist, die voranstehend beschriebene Reaktion zu katalysieren. Entsprechende Aktivitätstest sind weiter unten beschrieben. Saccharose-6-phosphat stammt aus der von der Saccharose-6-phosphat Synthase katalysierten Reaktion, in der UDP-Glukose und Fruktose-6-phosphat zu Saccharose-6-phosphat umgewandelt werden. Neuere Daten sprechen für einen Assoziation der Saccharose-6-phosphat Synthase und Saccharose-6-phosphat Phosphatase in einem Proteinkomplex (Eccheveria et al. 1997, Plant Physiology 115, 223), was auf eine regulatorische Funktion der Saccharose-6-phosphat Phosphatase hindeuten könnte. Ob die Saccharose-6-phosphat Phosphatase für die Pflanze essentiell ist, ist nicht bekannt.

Die Klonierung von Saccharose-6-phosphat Phosphatase kodierenden Genen aus verschiedenen Pflanzspezies ist in der Literatur beschrieben (Lunn et al., 2000, Procl. Natl. Acad. Sci. USA 97: 12914), wobei es allerdings keine Untersuchungen zur in planta Funktion des Enzyms gibt.

Bekannt sind drei für Saccharose-6-Phosphat Phosphatase kodierende Nukleinsäuresequenzen sowie Proteinsequenzen aus Arabidopsis thaliana, Gen Bank Acc. No AF 283565 und AAG31075 (Identität zur SEQ ID NO:1 = 68%; Identität zur SEQ ID NO:2 = 72%; Identität zur SEQ ID NO:3 = 68%; Identität zur SEQ ID NO:4 = 72%; Identität zur SEQ ID NO:5 = 61 %; Identität zur SEQ ID NO:6 = 71 %), Gen. Bank Acc. No. AF434711 und AAL30747 (Identität zur SEQ ID NO:1 = 55%; Identität zur SEQ ID NO:2 = 55%; Identität zur SEQ ID NO:3 = 56%; Identität zur SEQ ID NO:4 = 54%; Identität zur SEQ ID NO:5 = 51%; Identität zur SEQ ID NO:6 = 54%) sowie Gen. Bank Acc. No. AF356816 und AAK40235(ldentität zur SEQ ID NO:1 = 62%; Identität zur SEQ ID NO:2 = 60%; Identität zur SEQ ID NO:3 = 63%, Identität zur SEQ ID NO:4 = 61%; Identität zur SEQ iD NO:5 = 59%, Identität zur SEQ ID NO: = 60%), drei für Saccharose-6-Phosphat Phosphatase kodierende Nukleinsäuresequenzen aus Triticum aestivum, Gen Bank Acc. No AY029159 und AAK31789 (Identität zur SEQ ID NO:1 = 62%; Identität zur SEQ ID NO:2 = 64%; Identität zur SEQ ID NO:3 = 61%; Identität zur SEQ ID NO:4 = 64%; Identität zur SEQ ID NO:5 = 56%; Identität zur SEQ ID NO:6 = 64%), Gen. Bank Acc. No. AF321557 und AAK09372 (Identität zur SEQ ID NO:1 = 61%; Identität zur SEQ ID NO:2 = 64%; Identität zur SEQ ID NO:3 = 60%; Identität zur SEQ ID NO:4 = 64%; Identität zur SEQ ID NO:5 = 60%; Identität zur SEQ ID NO:6 = 64%) sowie Gen. Bank Acc. No. AF321556 und AAK09371 (Identität zur SEQ ID NO:1 = 67%; Identität zur SEQ ID NO:2 = 64%; Identität zur SEQ ID NO:3 = 60%; Identität zur SEQ ID NO:4 = 64%; Identität zur SEQ ID NO:5 = 61%; Identität zur SEQ ID NO:6 = 64%), eine Saccharose-6-Phosphat Phosphatase kodierende Nukleinsäuresequenzen aus Medicago trunculata, Gen Bank Acc. No AF283566 und AAG31076 (Identität zur SEQ ID NO:1 = 67%; Identität zur SEQ ID NO:2 = 67%; Identität zur SEQ ID NO:3 = 67%; Identität zur SEQ ID NO:4 = 69%; Identität zur SEQ ID NO:5 = 62%; Identität zur SEQ ID NO:6 = 66%) und eine Saccharose-6-Phosphat Phosphatase kodierende Nukleinsäuresequenzen aus Zea mays, Gen Bank Acc. No AAG31074 (Identität zur SEQ ID NO:2 = 62%; Identität zur SEQ ID NO:4 = 63%; Identität zur SEQ ID NO:6 = 63%).

Im Rahmen der vorliegenden Erfindung wurde überraschenderweise gefunden, dass Pflanzen, in denen gezielt die Aktivität der Saccharose-6-Phosphat Phosphatase verringert wurde Phänotypen aufwiesen, die mit durch Herbizidapplikation erzeugten Phänotypen vergleichbar sind. Beobachtet wurden Wachstumsretardierungen und chlorotische Blätter sowie in einigen Fällen das Absterben ganzer Pflanzen oder von Pflanzenteilen.

Gegenstand der vorliegenden Erfindung ist die Verwendung von einem Polypeptid mit der biologischen Aktivität einer Saccharose-6-Phosphat Phosphatase kodiert durch eine Nukleinsäuresequenz umfassend
a) eine Nukleinsäuresequenz mit der in SEQ ID NO:1; SEQ ID NO:3 oder SEQ ID NO:5 dargestellten Nukleinsäuresequenz; oder
b) eine Nukleinsäuresequenz, die sich aufgrund des degenerierten genetischen Codes durch Rückübersetzung aus der in SEQ ID NO:2, SEQ ID NO:4 oder SEQ ID NO:6 dargestellten Aminosäuresequenz ableiten läßt; oder
c) funktionelle Äquivalente der Nukleinsäuresequenz SEQ ID NO:1 mit einer Identität von mindestens 55% zu der SEQ ID NO:1; funktionelle Äquivalente der Nukleinsäuresequenz SEQ ID NO:3 mit einer Identität von mindestens 55% zu der SEQ ID NO:3; oder funktionelle Äquivalente der Nukleinsäuresequenz SEQ ID NO:5 mit einer Identität von mindestens 51% zu der SEQ ID NO:5;
d) eine Nukleinsäuresequenz, die sich aufgrund des degenerierten genetischen Codes durch Rückübersetzung der Aminosäuresequenz eines funktionellen Äquivalents der SEQ ID NO:2, das eine Identität mit der SEQ ID NO:2 von mindestens 55% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:4, das eine Identität mit der SEQ ID NO:4 von mindestens 54% aufweist, ableiten läßt; oder eine Nukleinsäuresequenz. die sich aufgrund des degenerierten genetischen Codes durch Rückübersetzung der Aminosäuresequenz eines funktionellen Äquivalents der SEQ ID NO:6, das eine Identität mit der SEQ ID NO:6 von mindestens 54% aufweist, ableiten läßt;
als Target für Herbizide. Die funktionellen Äquivalente nach c) und d) zeichnen sich durch eine gleiche Funktionalität aus, d.h. sie haben die physiologische Funktion einer Saccharose-6-Phosphat Phosphatase.

Die oben genannten Nukleinsäuresequenzen stammen vorzugsweise aus einer Pflanze z.B. aus einer Pflanze aus der Familie der Solanacean.

Die erfindungsgemäßen funktionellen Äquivalente der SEQ ID NO:1 weisen eine Homologie mit der SEQ ID NO:1 von mindestens 55%, 56%, 57%, 58%, 59%, 60%, 61%, 62%, 63%, 64%, 65%, 66%, 67%, 68% vorzugsweise mindestens 69%, 70%, 71%, 72%, 73%, 74% vorzugsweise mindestens 75%, 76%, 77%, 78%, 79%, 80%, 81%, 82%, 83% bevorzugt mindestens 84%, 85%, 86%, 87%, 88%, 89%, 89%, 90%, 91%, 92%, 93% besonders bevorzugt mindestens 94%, 95%, 96%, 97%, 98%, 99% auf.

Die erfindungsgemäßen funktionellen Äquivalente der SEQ ID NO:2 weisen eine Homologie mit der SEQ ID NO:2 von mindestens 55%, 56%, 57%, 58%, 59%, 60%, 61%, 62%,. 63%, 64% vorzugsweise mindestens 65%, 66%, 67%, 68%, 69%, 70%, 71%, 72%, vorzugsweise mindestens 73%, 74%, 75%, 76%, 77%, 78%, 79%, 80%, 81%, 82%, 83% bevorzugt mindestens 84%, 85%, 86%, 87%, 88%, 89%, 89%, 90%, 91 %, 92%, 93% besonders bevorzugt mindestens 94%, 95%, 96%, 97%, 98%, 99% auf.

Die erfindungsgemäßen funktionellen Äquivalente der SEQ ID NO:3 weisen eine Homologie mit der SEQ ID NO:3 von mindestens 55%, 56%, 57%, 58%, 59%, 60%, 61%, 62%, 63%, 64%, 65%, 66%, 67%, 68% vorzugsweise mindestens 69%, 70%, 71 %, 72%, 73%, 74% vorzugsweise mindestens 75%, 76%, 77%, 78%, 79%, 80%, 81%, 82%, 83% bevorzugt mindestens 84%, 85%, 86%, 87%, 88%, 89%, 89%, 90%, 91%, 92%, 93% besonders bevorzugt mindestens 94%, 95%, 96%, 97%, 98%, 99% auf.

Die erfindungsgemäßen funktionellen Äquivalente der SEQ ID NO:4 weisen eine Homologie mit der SEQ ID NO:4 von mindestens 54%, 55%, 56%, 57%, 58%, 59%. 60%, 61%, 62% vorzugsweise mindestens 63%, 64%, 65%, 66%, 67%, 68%, 69%, 70%, 71%, 72% vorzugsweise mindestens 73%, 74%, 75%, 76%, 77%, 78%, 79%, 80%, 81%, 82% bevorzugt mindestens 83%, 84%, 85%, 86%, 87%, 88%, 89%, 89%, 90%, 91%, 92%, 93% besonders bevorzugt mindestens 94%, 95%, 96%, 97%, 98%, 99% auf.

Die erfindungsgemäßen funktionellen Äquivalente der SEQ ID NO:5 weisen eine Homologie mit der SEQ ID NO:5 von mindestens 51%. 52%. 53%, 54%, 55%, 56%, 57%, 58%, 59%, 60%, 61%, 62% vorzugsweise mindestens 63%, 64%, 65%, 66%, 67%, 68%, 69%, 70%, 71%, 72%, 73%, 74% vorzugsweise mindestens 75%, 76%, 77%, 78%, 79%, 80%, 81%, 82%, 83% bevorzugt mindestens 84%, 85%, 86%, 87%, 88%, 89%, 89%, 90%, 91%, 92%, 93% besonders bevorzugt mindestens 94%, 95%, 96%, 97%, 98%, 99% auf.

Die erfindungsgemäßen funktionellen Äquivalente der SEQ ID NO:6 weisen eine Homologie mit der SEQ ID NO:6 von mindestens 54%, 55%, 56%, 57%, 58%, 59%, 60%, 61%, 62%, 63%, 64% vorzugsweise mindestens 65%, 66%, 67%, 68%, 69%, 70%, 71% vorzugsweise mindestens 72%, 73%, 74%, 75%, 76%, 77%, 78%, 79%, 80%, 81%, 82%, 83% bevorzugt mindestens 84%, 85%, 86%, 87%, 88%, 89%, 89%, 90%, 91%, 92%, 93% besonders bevorzugt mindestens 94%, 95%, 96%, 97%, 98%, 99% auf.

Beispiele für funktionelle Äquivalente gemäß c) bzw. d) sind die bereits oben erwähnten für Saccharose-6-Phosphat Phosphatase kodierende Nukleinsäuresequenzen sowie Proteinsequenzen aus Arabidopsis thaliana (Gen Bank Acc. No AF 283565 und AAG31075, Gen. Bank Acc. No. AF434711 und AAL30747; Gen. Bank Acc. No. AF356816 und AAK40235), die drei für Saccharose-6-Phosphat Phosphatase kodierende Nukleinsäuresequenzen aus Triticum aestivum (Gen Bank Acc. No AY029159 und AAK31789, Gen. Bank Acc. No. AF321557 und AAK09372; Gen. Bank Acc. No. AF321556 und AAK09371) die Saccharose-6-Phosphat Phosphatase kodierende Nukleinsäuresequenz aus Medicago trunculata (Gen Bank Acc. No AF283566 und AAG31076) und die Saccharose-6-Phosphat Phosphatase kodierende Nukleinsäuresequenz aus Zea mays (Gen Bank Acc. No AAG31074).

Nukleinsäuresequenzen kodierend für ein Polypeptid mit der biologischen Aktivität einer Saccharose-6-Phosphat Phosphatase umfassend:
a) eine Nukleinsäuresequenz mit der in SEQ ID NO:1, SEQ ID NO:3 oder SEQ ID NO:5 dargestellten Nukleinsäuresequenz; oder
b) eine Nukleinsäuresequenz, die sich aufgrund des degenerierten genetischen Codes durch Rückübersetzung aus der in SEQ ID NO:2, SEQ ID NO:4 oder SEQ ID NO:6 dargestellten Aminosäuresequenz ableiten läßt; oder
c) funktionelle Äquivalente der Nukleinsäuresequenz SEQ ID NO:1 mit einer Identität von mindestens 69% zu der SEQ ID NO:1; oder funktionelle Äquivalente der Nukleinsäuresequenz SEQ ID NO:3 mit einer Identität von mindestens 69% zu der SEQ ID NO:3; oder funktionelle Äquivalente der Nukleinsäuresequenz SEQ ID NO:5 mit einer Identität von mindestens 63% zu der SEQ ID NO:5; oder
d) eine Nukleinsäuresequenz, die sich aufgrund des degenerierten genetischen Codes durch Rückübersetzung der Aminosäuresequenz eines funktionellen Äquivalents der SEQ ID NO:2, das eine Identität mit der SEQ ID NO:2 von mindestens 73% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:4, das eine Identität mit der SEQ ID NO:4 von mindestens 73% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:6, das eine Identität mit der SEQ ID NO:6 von mindestens 72% aufweist, ableiten läßt
wobei die oben genannten Nukleinsäuresequenzen aus einer Pflanze z.B. aus einer Pflanze aus der Familie der Solanacean stammenund ebenfalls wie die durch die vorstehend genannten Nukleinsäuresequenzen kodierten Polypeptide dienen zur Verwendung als Target für Herbizide. Die funktionellen Äquivalente nach c) und d) zeichnen sich durch eine gleiche Funktionalität aus, d.h. sie haben die physiologische Funktion einer Saccharose-6-Phosphat Phosphatase.

Die erfindungsgemäßen funktionellen Äquivalente der SEQ ID NO:1 weisen eine Identität mit der SEQ ID NO:1 von mindestens 69%, 70%, 71%, 72%, 73%, 74% vorzugsweise mindestens 75%, 76%, 77%, 78%, 79%, 80%, 81%, 82%, 83% bevorzugt mindestens 84%, 85%, 86%, 87%, 88%, 89%, 89%, 90%, 91%, 92%, 93% besonders bevorzugt mindestens 94%, 95%, 96%, 97%, 98%, 99% auf.

Die erfindungsgemäßen funktionellen Äquivalente der SEQ ID NO:2 weisen eine Identität mit der SEQ ID NO:2 von mindestens 73% vorzugsweise mindestens 74%, 75%, 76%, 77%, 78%, 79%, 80%, 81%, 82%, 83% bevorzugt mindestens 84%, 85%, 86%, 87%, 88%, 89%, 89%, 90%, 91%, 92%, 93% besonders bevorzugt mindestens 94%, 95%, 96%, 97%, 98%, 99% auf.

Die erfindungsgemäßen funktionellen Äquivalente der SEQ ID NO:3 weisen eine Identität mit der SEQ ID NO:3 von mindestens 69%, 70%, 71%, 72%, 73%, 74% vorzugsweise mindestens 75%, 76%, 77%, 78%, 79%, 80%, 81%, 82%, 83% bevorzugt mindestens 84%, 85%, 86%, 87%, 88%, 89%, 89%, 90%, 91%, 92%, 93% besonders bevorzugt mindestens 94%, 95%, 96%, 97%, 98%, 99% auf.

Die erfindungsgemäßen funktionellen Äquivalente der SEQ ID NO:4 weisen eine Identität mit der SEQ ID NO:4 von mindestens 73% vorzugsweise mindestens 74%, 75%, 76%, 77%, 78%, 79%, 80%, 81%, 82%, 83% bevorzugt mindestens 84%, 85%, 86%, 87%, 88%, 89%, 89%, 90%, 91%, 92%, 93% besonders bevorzugt mindestens 94%, 95%, 96%, 97%, 98%, 99% auf.

Die erfindungsgemäßen funktionellen Äquivalente der SEQ ID NO:5 weisen eine Identität mit der SEQ ID NO:5 von mindestens 63%, 64%, 65%, 66%, 67%, 68%. 69%, 70%, 71%, 72%, 73%, 74% vorzugsweise mindestens 75%, 76%, 77%, 78%, 79%, 80%, 81%, 82%, 83% bevorzugt mindestens 84%, 85%, 86%, 87%, 88%, 89%, 89%, 90%, 91 %, 92%, 93% besonders bevorzugt mindestens 94%, 95%, 96%, 97%, 98%, 99% auf.

Die erfindungsgemäßen funktionellen Äquivalente der SEQ ID NO:6 weisen eine Identität mit der SEQ ID NO:6 von mindestens 72%, 73% vorzugsweise mindestens 74%, 75%, 76%, 77%, 78%, 79%. 80%, 81%, 82%, 83% bevorzugt mindestens 84%, 85%, 86%, 87%, 88%, 89%, 89%, 90%, 91 %, 92%, 93% besonders bevorzugt mindestens 94%, 95%, 96%, 97%, 98%. 99% auf.

Die Nukleinsäuresequenzen kodierend für ein Polypeptid mit der biologischen Aktivität einer Saccharose-6-Phosphat Phosphatase umfassend
a) eine Nukleinsäuresequenz mit der in SEQ ID NO:1; SEQ ID NO:3 oder SEQ ID NO:5 dargestellten Nukleinsäuresequenz; oder
b) eine Nukleinsäuresequenz, die sich aufgrund des degenerierten genetischen Codes durch Rückübersetzung aus der in SEQ ID NO:2, SEQ ID NO:4 oder SEQ ID NO:6 dargestellten Aminosäuresequenz ableiten läßt; oder
c) funktionelle Äquivalente der Nukleinsäuresequenz SEQ ID NO:1 mit einer Identität von mindestens 55% zu der SEQ ID NO:1; oder funktionelle Äquivalente der Nukleinsäuresequenz SEQ ID NO:3 mit einer Identität von mindestens 55% zu der SEQ ID NO:3; oder funktionelle Äquivalente der Nukleinsäuresequenz SEO ID NO:5 mit einer Identität von mindestens 51% zu der SEQ ID NO:5; oder
d) funktionelle Äquivalente der Nukleinsäuresequenz SEQ ID NO:1 mit einer Identität von mindestens 69% zu der SEQ iD NO:1; oder funktionelle Äquivalente der Nukleinsäuresequenz SEQ ID NO:3 mit einer Identität von mindestens 69% zu der SEQ ID NO:3; oder funktionelle Äquivalente der Nukleinsäuresequenz SEQ ID NO:5 mit einer Identität von mindestens 63% zu der SEQ ID NO:5; oder
e) eine Nukleinsäuresequenz, die sich aufgrund des degenerierten genetischen Codes durch Rückübersetzung der Aminosäuresequenz eines funktionellen Äquivalents der SEQ ID NO:2, das eine Identität mit der SEQ ID NO:2 von mindestens 73% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:4, das eine identität mit der SEQ ID NO:4 von mindestens 73% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:6, das eine Identität mit der SEQ ID NO:6 von mindestens 72% aufweist, ableiten läßt; oder
f) eine Nukleinsäuresequenz, die sich aufgrund des degenerierten genetischen Codes durch Rückübersetzung der Aminosäuresequenz eines funktionellen Äquivalents der SEQ ID NO:2, das eine Identität mit der SEQ ID NO:2 von mindestens 55% aufweist; ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:4, das eine Identität mit der SEQ ID NO:4 von mindestens 54% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:6, das eine Identität mit der SEQ ID NO:6 von mindestens 54% aufweist, ableiten läßt;
werden im folgenden als "erfindungsgemäße Nukleinsäuresequenzen" bezeichnet. Die durch eine erfindungsgemäße Nukleinsäuresequenz kodierten Polypeptide mit der biologischen Aktivität einer Saccharose-6-Phosphat Phosphatase werden im folgenden der Einfachheit halber als "SSP" bezeichnet.

SSPs verursachen in reduzierter Menge Wachstumsretardierungen sowie nekrotische Blätter in Pflanzen. Eine Reduktion des Polypeptides bedeutet, daß die Menge des Polypeptides über gentechnische Methoden reduziert wird. Verglichen wird eine derartig modifizierte Pflanze mit einer Pflanze, die bezüglich dieses Polypeptides keine genetischen Modifikationen aufweist, ansonsten aber mit dem Genotyp der genetisch manipulierten Pflanze identisch ist unter identischen Wachstumsbedingungen.

Die Genprodukte der erfindungsgemäßen Nukleinsäuren stellen neue Targets für Herbizide dar, welche die Bereitstellung neuer Herbizide zur Bekämpfung unerwünschter Pflanzen ermöglichen.

Unter unerwünschten Pflanzen sind im weitesten Sinne alle Pflanzen zu verstehen, die an Orten aufwachsen, an denen sie unerwünscht sind, zum Beispiel:

Dikotyle Unkräuter der Gattungen: *Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeonsis, Panaver, Centaurea, Trifolium, Ranunculus, Taraxacum.*

Monokotyle Unkräuter der Gattungen: *Echinochloa, Setaria, Panicum, Digitaria, Phieum, Poa, Festuca, Eleusine, Brachiaria, Lolium, Bromus, Avena, Cyperus, Sorghum, Agropyron, Cynodon, Monochoria, Fimbristyslis, Sagittaria, Eleocharis, Scirpus, Paspalum, Ischaemum, Sphenoclea, Dactyloctenium, Agrostis, Alopecurus, Apera.*

Die SEQ ID NO:1, SEQ ID NO:3 oder SEQ ID NO:5 oder Teile der vorstehend genannten Nukleinsäuresequenzen können für die Herstellung von Hybridisierungssonden verwendet werden, über welche die entsprechenden Vollängengene bzw. funktionelle Äquivalente der SEQ ID NO:1, SEQ ID NO:3 oder SEQ ID NO:5 isoliert werden können. Die Herstellung dieser Sonden sowie die Durchführung der Experimente ist bekannt. Sie kann zum Beispiel über die gezielte Herstellung radioaktiver oder nicht radioaktiver Sonden mittels PCR und der Verwendung von entsprechend markierten Oligonukleotiden mit anschließenden Hybridisierungsexperimenten erfolgen. Die hierfür erforderlichen Technologien sind beispielsweise in T. Maniatis, E.F. Fritsch und J. Sambrook, "Molecular Cloning: A Laboratory Manual", Cold Spring Harbor Laboratory, Cold Spring Harbor, NY (1989) aufgeführt. Die entsprechenden Sonden können weiterhin mittels Standardtechnologien (Lit. SDM bzw. random Mutagenesis) so modifiziert werden, dass sie für weitere Zwecke eingesetzt werden können, z.B. als Sonde, die spezifisch zu mRNA sowie den entsprechenden codierenden Sequenzen hybridisiert zwecks Analyse der entsprechenden Sequenzen in anderen Organismen.

Des weiteren können die oben genannten Sonden für die Detektion und Isolation von funktionellen Äquivalenten der SEQ ID NO:1, SEQ ID NO:3 oder SEQ ID NO:5 aus anderen Pflanzenspezies aufgrund von Sequenzidentitäten verwendet werden. Hierbei wird ein Teil oder die gesamte Sequenz der entsprechenden SEQ ID NO:1, SEQ ID NO:3 oder SEQ ID NO:5 als Sonde zum Screening in einer genomischen oder cDNA Bank der entsprechenden Pflanzenspezies oder in einer Computer-Recherche nach Sequenzen funktioneller Äquivalente in elektronischen Datenbanken verwendet.

Bevorzugte Pflanzenspezies sind hierbei die bereits eingangs erwähnten unerwünschten pflanzen.

Ein weiterer Gegenstand der Erfindung ist die Verwendung von Expressionskassetten enthaltend
a) genetische Kontrollsequenzen in funktioneller Verknüpfung mit einer Nukleinsäuresequenz umfassend
   i. eine Nukleinsäuresequenz mit der in SEQ ID NO:1, SEQ ID NO:3 oder SEQ ID NO:5 dargestellten Nukleinsäuresequenz; oder
   ii. eine Nukleinsäuresequenz, die sich aufgrund des degenerierten genetischen Codes durch Rückübersetzung aus der in SEQ ID NO:2, SEQ ID NO:4 oder SEQ ID NO:6 dargestellten Aminosäuresequenz ableiten läßt; oder
   iii. funktionelle Äquivalente der Nukleinsäuresequenz SEQ ID NO:1 mit einer Identität von mindestens 69% zu der SEQ ID NO:1; oder funktionelle Äquivalente der Nukleinsäuresequenz SEQ ID NO:3 mit einer Identität von mindestens 69% zu der SEQ ID NO:3; oder funktionelle Äquivalente der Nukleinsäuresequenz SEQ ID NO:5 mit einer Identität von mindestens 63% zu der SEQ ID NO:5; oder
   iv. eine Nukleinsäuresequenz, die sich aufgrund des degenerierten genetischen Codes durch Rückübersetzung der Aminosäuresequenz eines funktionellen Äquivalents der SEQ ID NO:2, das eine Identität mit der SEQ ID NO:2 von mindestens 73% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:4, das eine Identität mit der SEQ ID NO:4 von mindestens 73% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:6, das eine Identität mit der SEQ ID NO:6 von mindestens 72% aufweist, ableiten läßt; oder
b) zusätzliche Funktionselemente; oder
c) eine Kombination aus a) und b);
sowie die Verwendung von Expressionskassetten enthaltend
a) genetische Kontrollsequenzen in funktioneller Verknüpfung mit einer erfindungsgemäßen Nukieinsäuresequenz;
b) zusätzliche Funktionselemente; oder
c) eine Kombination aus a) und b);
zur Expression einer SSP, die in vitro Testsystemen verwendet werden kann. Beide Ausführungsformen der vorstehend beschriebenen Expressionskassetten werden im folgenden als erfindungsgemäße Expressionskassette bezeichnet.

Gemäß einer bevorzugten Ausführungsform umfaßt eine erfindungsgemäße Expressionskassette am 5'-Ende der kodierenden Sequenz einen Promotor und am 3'-Ende Transkriptions-Terminations-Signal und gegebenenfalls weitere genetische Kontrollsequenzen, welche mit der dazwischenliegenden erfindungsgemäßen Nukleinsäuresequenz funktionell verknüpft sind.

Unter den erfindungsgemäßen Expressionskassetten sind auch Analoga zu verstehen, die zum Beispiel durch eine Kombination der einzelnen Nukleinsäuresequenzen auf einem Polynukleotid (Mehrfachkonstrukte), auf mehreren Polynukleotiden in einer Zelle (Kotransformation) oder durch sequenzielle Transformation zustande kommen können.

Vorteilhafte genetische Kontrollsequenzen nach Punkt a) für die erfindungsgemäßen Expressionskassetten oder für Vektoren enthaltend erfindungsgemäße Expressionskassetten sind beispielsweise Promotoren wie cos-, tac-, trp-, tet-, Ipp-, lac-, laclq-, T7-, T5-, T3-, gal-, trc-, ara-, SP6-, λ-PR- oder im λ-PL-Promotor, die zur Expression der SSP in gram-negativen Bakterienstämmen verwendet werden können.

Weitere vorteilhafte genetische Kontrollsequenzen sind beispielsweise in den Promotoren amy und SPO2, die zur Expression der SSP in gram-positiven Bakterienstämmen verwendet werden können, sowie in den Hefe- oder Pilzpromotoren AUG1, GPD-1, PX6, TEF, CUP1, PGK, GAP1, TPI, PHO5, AOX1, GAL10/CYC1, CYC1, OliC, ADH, TDH, Kex2, MFa oder NMT oder Kombinationen der vorstehend genannten Promotoren enthalten (Degryse et al., Yeast 1995 Jun 15; 11(7):629-40; Romanos et al. Yeast 1992 Jun;8(6):423-88; Benito et al. Eur. J. Plant Pathol. 104, 207-220 (1998); Cregg et al. Biotechnology (N Y) 1993 Aug;11(8):905-10; Luo X., Gene 1995 Sep 22;163(1):127-31; Nacken et al., Gene 1996 Oct 10;175(1-2): 253-60; Turgeon et al., Mol Cell Biol 1987 Sep;7(9):3297-305) oder den Transkriptionsterminatoren NMT, Gcy1, TrpC, AOX1, nos, PGK oder CYC1 (Degryse et al., Yeast 1995 Jun 15; 11 (7):629-40; Brunelli et al. Yeast 1993 Dec9(12): 1309-18; Frisch et al., Plant Mol. Biol. 27 (2), 405-409 (1995); Scorer et al., Biotechnology (N.Y.) 12 (2), 181-184 (1994), Genbank acc. number Z46232; Zhao et al. Genbank acc number: AF049064; Punt et al., (1987) Gene 56 (1), 117-124), die zur Expression der SSP in Hefestämmen verwendet werden können.

Als zur Expression in Insektenzellen geeignete genetische Kontrollsequenzen sind exemplarisch der Polyhedrin-Promotor sowie der p10-Promotor (Luckow, V.A. and Summers, M.D. (1988) Bio/Techn. 6, 47-55) zu nennen.

Vorteilhafte genetische Kontrollsequenzen zur Expression der SSP in Zellkultur sind sind neben Polyadenylierungssequenzen wie z.B. aus Simian Virus 40 eukaryontische Promotoren viralen Ursprungs wie z.B. Promotoren des Polyoma, Adenovirus 2, Cytomegalovirus oder Simian Virus 40.

Weitere vorteilhafte genetische Kontrollsequenzen zur Expression der SSP in Pflanzen sind in den Pflanzenpromotoren CaMV/35S [Franck et al., Cell 21(1980) 285-294], PRP1 [Ward et al., Plant. Mol. Biol. 22 (1993)], SSU, OCS, LEB4, USP, STLS1, B33, NOS; FBPaseP (WO 98/18940) oder im Ubiquitin- oder Phaseolin-Promotor enthalten, vorzugsweise verwendet man insbesondere einen pflanzlichen Promotor oder einen Promotor, der einem Pflanzenvirus entstammt. Insbesondere bevorzugt sind Promotoren viralen Ursprungs wie der Promotor des 35S-Transkriptes des Blumenkohlmosaikvirus (Franck et al., Cell 21 (1980), 285-294; Odell et al., Nature 313 (1985), 810-812). Weitere bevorzugte konstitutive Promotoren sind zum Beispiel der Promotor der Nopalinsynthase aus Agrobacterium, der TR-Doppelpromotor, der OCS (Octopin Synthase) Promotor aus Agrobacterium, der Ubiquitin Promotor (Holtorf S et al., Plant Mol Biol 1995, 29:637-649), die Promotoren der vakuolärer ATPase Untereinheiten oder der Promotor eines prolinreichen Proteins aus Weizen (WO 91/13991).

Die Expressionskassetten können auch einen chemisch induzierbaren Promotor als genetische Kontrollsequenz enthalten, durch den die Expression des exogenen Gens in der Pflanze zu einem bestimmten Zeitpunkt gesteuert werden kann. Derartige Promotoren, wie z.B. der PRP1 Promotor (Ward et al., Plant. Mol. Biol. 22 (1993), 361-366), ein durch Salicylsäure induzierbarer (WO 95/19443), ein durch Benzolsulfonamid-induzierbarer (EP-A-0388186), ein durch Tetrazyklin-induzierbarer (Gatz et al., (1992) Plant J. 2, 397404), ein durch Abscisinsäure-induzierbarer (EP-A 335528) bzw. ein durch Ethanol- oder Cyclohexanön-induzierbarer (WO 93/21334) Promotor können ebenfalls verwendet werden.

Geeignet sind ferner Promotoren die eine gewebe- oder organspezifische Expression z.B. in Antheren, Ovarien, Blüten und Blütenorganen, Blättern, Schließzellen, Trichomen, Stengel, Leitgeweben, Wurzeln und Samen vermitteln. Ebenfalls geeignet sind hier neben den oben genannten konstitutiven Promotoren, insbesondere solche Promotoren, die eine blattspezifische Expression gewährleisten. Zu nennen sind der Promotor der cytosolischen FBPase aus Kartoffel (WO 97/05900), der SSU Promotor (small subunit) der Rubisco (Ribuiose-1,5-bisphosphatcarboxylase) oder der ST-LSI Promotor aus Kartoffel (Stockhaus et al., EMBO J. 8 (1989), 2445 - 245). Bevorzugt sind ferner Promotoren, die eine Expression in Samen und pflanzlichen Embryonen steuern. Samenspezifische Promotoren sind zum Beispiel der Promotor des Phaseolins (US 5,504,200, Bustos MM et al., Plant Cell. 1989;1 (9):839-53), des 2S Albumingens (Joseffson LG et al., J Biol Chem 1987, 262:12196-12201), des Legumins (Shirsat A et al., Mol Gen Genet. 1989;215(2):326-331), des USP (unknown seed protein; Bäumlein H et al., Molecular & General Genetics 1991, 225(3):459-67) des Napin Gens (Stalberg K, et al., L. Planta 1996, 199:515-519), des Saccharosebindeproteins (WO 00/26388) oder der LeB4-Promotor (Bäumlein H et al., Mol Gen Genet 1991, 225: 121-128; Fiedler, U. et al., Biotechnology (NY) (1995), 13 (10) 1090).

Weitere als genetische Kontrollsequenzen geeignete Promotoren sind beispielsweise spezifische Promotoren für Knollen, Speicherwurzeln oder Wurzeln, wie beispielsweise der Patatin Promotor Klasse I (B33), der Promotor des Cathepsin D Inhibitors aus Kartoffel, der Promotor der Stärke Synthase (GBSS1) oder der Sporamin Promotor, fruchtspezifische Promotoren, wie beispielsweise der fruchtspezifische Promotor aus Tomate (EP-A 409625), fruchtreifung-spezifische Promotoren, wie beispielsweise der fruchtreifung-spezifische Promotor aus Tomate (WO 94/21794), blütenspezifische Promotoren, wie beispielsweise der Phytoen Synthase Promotor (WO 92/16635) oder der Promotor des P-rr Gens (WO 98/22593) oder spezifische Plastiden- oder Chromoplasten-Promotoren, wie beispielsweise der RNA-Polymerase Promotor (WO 97/06250) oder auch der Promotor der Phosphoribosylpyrophosphat Amidotransferase aus Glycine max (siehe auch Genbank Accession Nr U87999) oder ein anderer Nodien-spezifischer Promotor wie in EP-A 249676 können vorteilhaft verwendet werden.

Unter zusätzlichen Funktionselementen b) sind beispielhaft aber nicht einschränkend Reportergene, Replikationsursprünge, Selektionsmarker und sogenannte Affinitäts-Tags, fusioniert mit der SSP direkt oder mittels eines Linkers optional enthaltend eine Protease-Schnittstelle zu verstehen. Weitere geeignete zusätzliche Funktionselemente sind Sequenzen, die ein Targeting in den Apoplasten, in Plastiden, die Vakuole, das Mitochondrium, das Peroxisom, das Endoplasmatische Retikulum (ER) oder durch ein Fehlen entsprechender operativer Sequenzen einen Verbleib im Kompartiment des Entstehens, dem Zytosol, gewährleisten (Kermode, Crit. Rev. Plant Sci. 15, 4 (1996), 285-423).

Erfindungsgemäß ist ferner die Verwendung von Vektoren, die mindestens eine Kopie der erfindungsgemäßen Nukleinsäuresequenzen und/oder der erfindungsgemäßen Expressionskassetten enthalten.

Unter Vektoren sind außer Plasmiden auch alle anderen dem Fachmann bekannten Vektoren, wie beispielsweise Phagen, Viren wie SV40, CMV, Baculovirus, Adenovirus, Transposons, IS-Elemente, Phasmide, Phagemide, Cosmide, lineare oder zirkuläre DNA zu verstehen. Diese Vektoren können autonom im Wirtsorganismus repliziert oder chromosomal repliziert werden bevorzugt ist eine chromosomale Replikation.

In einer weiteren Ausgestaltungsform des Vektors kann die erfindungsgemäße Nukleinsäurekonstrukt auch vorteilhafterweise in Form einer linearen DNA in die Organismen eingeführt werden und über heterologe oder homologe Rekombination in das Genom des Wirtsorganismus integriert werden. Diese lineare DNA kann aus einem linearisierten Plasmid oder nur aus dem Nukleinsäurekonstrukt als Vektor oder den verwendeten Nukleinsäuresequenzen bestehen.

Weitere prokaryotische und eukaryotische Expressionssysteme sind genannt in Kapitel 16 und 17 in Sambrook et al., "Molecular Cloning: A Laboratory Manual." 2nd, ed., Cold Spring Harbor Laboratory, Cold Spring Harbor Laboratory Press, Cold Spring Harbor, NY, 1989. Weitere vorteilhafte Vektoren werden in Hellens et al. (Trends in plant science, 5, 2000) beschrieben.

Die erfindungsgemäße Expressionskassette sowie davon abgeleitete Vektoren können zur Transformation von Bakterien, Cyanobakterien (z.B. der Gattung Synechocystes, Anabaena, Calothrix, Scytonema, Oscillatoria, Plectonema und Nostoc), Proteobakterium wie etwa Magnetococcus sp. MC1, Hefen, filamentösen Pilzen und Algen und eukaryontischen, nicht humanen Zellen (z.B. Insektenzellen) mit dem Ziel der rekombinanten Herstellung der SSP eingesetzt werden, wobei sich die Herstellung einer geeigneten Expressionskassette nach dem Organismus, in welchen das Gen exprimiert werden soll, richtet.

In einer weiteren vorteilhaften Ausführungsform können die im erfindungsgemäßen Verfahren verwendeten Nukleinsäuresequenzen auch alleine in einen Organismus eingebracht werden.

Sollen neben den Nukleinsäuresequenzen weitere Gene in den Organismus eingeführt werden, so können alle zusammen in einem einzigen Vektor oder jedes einzelne Gen in je einem Vektor in den Organismus eingebracht werden, wobei die verschiedenen Vektoren gleichzeitig oder sukzessive eingebracht werden können.

Hierbei kann das Einbringen der erfindungsgemäßen Nukleinsäure(n), der Expressionskassette oder des Vektors in die entsprechenden Organismen (Transformation) prinzipiell nach allen dem Fachmann bekannten Methoden erfolgen.

Für Mikroorganismen kann der Fachmann entsprechende Methoden den Lehrbüchern von Sambrook, J. et al. (1989) "Molecular cloning: A laboratory manual", Cold Spring Harbor Laboratory Press, von F.M. Ausubel et al. (1994) "Current protocols in molecular blology", John Wiley and Sons, von D.M. Glover et al., DNA Cloning Vol.1, (1995), IRL Press (ISBN 019-963476-9), von Kaiser et al. (1994) Methods in Yeast Genetics, Cold Spring Habor Laboratory Press oder Guthrie et al. "Guide to Yeast Genetics and Molecular Biology", Methods in Enzymology, 1994, Academic Press entnehmen. Bei der Transformation von filamentösen Pilzen bieten sich zum einen die Herstellung von Protoplasten und Transformation mit Hilfe von PEG (Wiebe et al. (1997) Mycol. Res. 101 (7): 971-877; Proctor et al. (1997) Microbiol. 143; 2538-2591), zum anderen die Transformation unter zur Hilfe nahme von Agrobacterium tumefaciens (de Groot et al. (1998) Nat. Biotech. 16, 839-842) an.

Für dikotyle Pflanzen können die beschriebenen Methoden zur Transformation und Regeneration von Pflanzen aus Pflanzengeweben oder Pflanzenzellen zur transienten oder stabilen Transformation genutzt werden. Geeignete Methoden sind das biolistische Verfahrens oder durch Protoplastentransformation (vergl. z.B. Willmitzer, L., 1993 Transgenic plants. In: Biotechnology, A Multi-Volume Comprehensive Treatise (H.J. Rehm, G. Reed, A. Pühler, P. Stadler, eds.), Vol. 2, 627-659, VCH Weinheim-New York-Basel-Cambridge), die Elektroporation, die Inkubation trockener Embryonen in DNA-haltiger Lösung, die Mikroinjektion und der durch Agrobacterium vermittelte Gentransfer. Die genannten Verfahren sind beispielsweise in B. Jenes et al., Techniques for Gene Transfer, in: Transgenic Plants, Vol. 1., Engineering and Utilization, herausgegeben von S.D. Kung und R. Wu, Academic Press (1993) 128-143 sowie in Potrykus Annu. Rev. Plant Physiol. Plant Molec.Biol. 42 (1991) 205-225) beschrieben.

Die Transformation mittels Agrobakterien sowie die für die Transformation zu verwendenden Vektoren sind dem Fachmann bekannt und in der Literatur ausführlich beschrieben (Bevan et al., Nucl. Acids Res. 12 (1984) 8711. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf Agrobacterium tumefaciens übertragen werden (Konjugation). Binäre Vektoren können sowohl in E. coli als auch in Agrobakterien replizieren. Sie enthalten ein Selektionsmarker-Gen und einen Linker oder Polylinker, welche von der rechten und linken T-DNA Grenzregion eingerahmt werden. Sie können direkt in die Agrobakterien transformiert werden (Holsters et al. Mol. Gen. Genet. 163 (1978), 181-187) , EP A 0 120 516; Hoekema, In: The Binary Plant Vector System Offsetdrukkerij Kanters B.V., Alblasserdam (1985), Chapter V; Fraley et al., Crit. Rev. Plant. Sci., 4: 1-46 und An et al. EMBO J. 4 (1985), 277-287).

Auch die Transformation monokotyler Pflanzen mittels Agrobacterium basierender Vektoren wurde beschrieben ( Chan et al, Plant Mol. Biol. 22(1993), 491-506; Hiei et al, Plant J. 6 (1994) 271-282; Deng et al; Science in China 33 (1990), 28-34; Wilmink et al, Plant Cell Reports 11,(1992) 76-80; May et al; Biotechnology 13 (1995) 486-492; Conner und Domisse; Int. J. Plant Sci. 153 (1992) 550-555; Ritchie et al; Transgenic Res. (1993) 252-265). Alternative Systeme zur Transformation von monokotylen Pflanzen sind die Transformation mittels des biolistischen Ansatzes (Wan und Lemaux; Plant Physiol. 104 (1994), 37-48; Vasil et al; Biotechnology 11 (1992), 667-674; Ritala et al, Plant Mol. Biol 24, (1994) 317-325; Spencer et al, Theor. Appl. Genet. 79 (1990), 625-631) die Protoplastentransformation, die Elektroporation von partiell permeabilisierten Zellen ; die Einbringung von DNA mittels Glasfasern. Insbesondere die Transformation von Mais wurde in der Literatur mehrfach beschrieben (vgl. z.B.
WO 95/06128; EP 0513849 A1; EP 0465875 A1; EP 0292435 A1; Fromm et al, Biotechnology 8 (1990), 833-844; Gordon-Kamm et al, Plant Cell 2 (1990), 603-618; Koziel et al, Biotechnology 11 (1993) 194-200; Moroc et al, Theor Applied Genetics 80 (190) 721-726).

Auch die erfolgreiche Transformation anderer Getreidearten wurde bereits beschrieben z.B. für Gerste ( Wan und Lemaux, s.o.; Ritala et al, s.o.; Weizen ( Nehra et al, Plant J. 5(1994) 285-297).

Mit einem erfindungsgemäßen Vektor transformierte Agrobakterien können ebenfalls in bekannter Weise zur Transformation von Pflanzen wie Testpflanzen wie Arabidopsis oder Kulturpflanzen wie Getreide, Mais, Hafer, Roggen, Gerste, Weizen, Soja, Reis, Baumwolle, Zuckerrübe, Canola, Sonnenblume, Flachs, Hanf, Kartoffel, Tabak, Tomate, Karotte, Paprika, Raps, Tapioka, Maniok, Pfeilwurz, Tagetes, Alfalfa, Salat und den verschiedenen Baum-, Nuß- und Weinspezies verwendet werden, z.B. indem verwundete Blätter oder Blattstücke in einer Agrobakterienlösung gebadet und anschließend in geeigneten Medien kultiviert werden.

Die genetisch veränderten Pflanzenzellen können über alle dem Fachmann bekannten Methoden regeneriert werden. Entsprechende Methoden können den oben genannten Schriften von S.D. Kung und R. Wu, Potrykus oder Höfgen und Willmitzer entnommen werden.

Die durch Transformation mit einer der oben beschriebenen Ausführungsformen einer Expressionskassette, enthaltend eine erfindungsgemäße Nukleinsäuresequenz oder einem Vektor, enthaltend die vorstehend genannte Expressionskassette, hergestellten transgenen Organismen sowie die mittels Expression aus dem transgenen Organismus erhältliche rekombinante SSP sind Gegenstand der vorliegenden Erfindung. Ebenfalls Gegenstand der vorliegenden Erfindung ist die Verwendung von transgenen Organismen enthaltend eine erfindungsgemäße Expressionskassette z.B. für die Bereitstellung rekombinanten Proteins und/oder die Verwendung dieser Organismen in in vivo Testsystemen.

Bevorzugte Organismen für die rekombinante Expression sind sind neben Bakterien, Hefen, Moose, Algen, Pilze auch eukaryontische Zellinien.

Bevorzugte Moose sind Physcomitrella patens oder weitere in Kryptogamen, Bd.2, Moose, Farne, 1991, Springer Verlag (ISBN 3540536515), beschriebene Moose.

Innerhalb der Bakterien sind Bakterien der Gattung Escherichia, Erwinia, Flavobacterium, Alcaligenes oder Cyanobakterien zum Beispiel der Gattung Synechocystes, Anabaena, Calothrix, Scytonema, Oscillatoria, Plectonema und Nostoc, besonders bevorzugt Synechocystis oder Anabena bevorzugt.

Bevorzugte Hefen sind Candida, Saccharomyces, Schizosaccheromyces, Hansenula oder Pichia.

Bevorzugte Pilze sind Aspergillus, Trichoderma, Ashbya, Neurospora, Fusarium, Beauveria, Mortierella, Saprolegnia, Pythium, oder weitere in Indian Chem Engr. Section B. Vol 37, No 1,2 (1995) beschriebene Pilze.

Bevorzugte Pflanzen sind insbesondere ausgewählt unter monokotylen Kulturpflanzen, wie zum Beispiel Getreidearten wie Weizen, Gerste, Hirse, Roggen, Triticale, Mais, Reis oder Hafer sowie dem Zuckerrohr. Ferner sind die erfindungsgemäßen transgenen Pflanzen insbesondere ausgewählt unter dikotylen Kulturpflanzen, wie zum Beispiel Brassicacae wie Raps, Kresse, Arabidopsis, Kohlarten oder Canola; Leguminosae wie Soja, Alfalfa, Erbse, Bohnengewächsen oder Erdnuss Solanaceae wie Kartoffel, Tabak, Tomate, Aubergine oder Paprika; Asteraceae wie Sonnenblume, Tagetes, Salat oder Calendula; Cucurbitaceae wie Melone, Kürbis oder Zucchini, der Lein, Baumwolle, Hanf, Flachs, Roter Pfeffer, Möhre, Karotte, Zuckerrübe oder verschiedene Baum-, Nuss- und Weinspecies.

Prinzipiell sind als Wirtsorganismen auch transgene Tiere geeignet wie beispielsweise C. elegans.

Bevorzugt ist auch die Verwendung von Expressionssystemen und Vektoren, die öffentlich zugänglich oder kommerziell erhältlich sind.

Zur Verwendung in E. coli Bakterien sind die typischen vorteilhaften, kommerziell erhältlichen Fusions- und Expressionsvektoren pGEX [Pharmacia Biotech Inc; Smith, D.B. and Johnson, K.S. (1988) Gene 67:3.1-40], pMAL (New England Biolabs, Beverly, MA) and pRIT5 (Pharmacia, Piscataway, NJ) welches Glutathion S-transferase beinhaltet (GST), Maltose Bindeprotein, oder Protein A, die pTrc-Vektoren (Amann et al., (1988) Gene 69:301-315) der "pKK233-2" von CLONTECH, Palo Alto, CA und die "pET"-, und die "pBAD"-Vektor-Serien von Stratagene, La Jolla zu nennen.

Weitere vorteilhafte Vektoren zur Verwendung in Hefe sind pYepSec1 (Baldari, et al., (1987) Embo J. 6:229-234), pMFa (Kurjan and Herskowitz, (1982) Cell 30:933-943), pJRY88 (Schultz et al., (1987) Gene 54:113-123), and pYES-Derivate, pGAPZ-Derivate, pPICZ-Derivate sowie die Vektoren des "Pichia Expression Kit" (Invitrogen Corporation, San Diego, CA). Vektoren für die Nutzung in filamentösen Pilzen sind beschrieben in: van den Hondel, C.A.M.J.J. & Punt, P.J. (1991) "Gene transfer systems and vector development for filamentous fungi, in: Applied Molecular Genetics of Fungi, J.F. Peberdy, et al., eds., p. 1-28, Cambridge University Press: Cambridge.

Alternativ können auch vorteilhaft Insektenzellexpressionsvektoren genutzt werden z.B. für die Expression in Sf9, Sf21 oder Hi5 Zellen, welche über rekombinante Baculoviren infiziert werden. Dies sind z.B. die Vektoren der pAc Serie (Smith et al. (1983) Mol. Cell Biol. 3:2156-2165) und der pVL series (Lucklow and Summers (1989) Virology 170:31-39). Weiterhin genannt seien die Baculovirus Expressionssysteme "MaxBac 2.0 Kit" und "Insect Select System" von Invitrogen, Calsbald oder "BacPAK Baculovirus Expressionssystem" von CLONTECH, Palo Alto, CA. Insektenzellen eignen sich in besonderer Weise zur Überexpression eukaryontischer Proteine, da sie posttranslationale Modifikationen der Proteine durchführen, die in Bakterien und Hefen nicht möglich sind. Die Handhabung von Insektenzellen in Zellkultur sowie ihre Infektion zur Expression von Proteinen sind dem Fachmann bekannt und können in Analogie zu bekannten Methoden erfolgen (Luckow und Summers, Bio/Tech. 6, 1988, pp.47-55; Glover and Hames (eds) in DNA Cloning 2, A practical Approach, Expression Systems, Second Edition, Oxford University Press, 1995, 205-244).

Des weiteren können zur Genexpression vorteilhaft Pflanzenzellen oder Algenzellen genutzt werden. Beispiele für Pflanzenexpressionsvektoren finden sich wie obenstehend erwähnt in Becker, D., et al. (1992) "New plant binary vectors with selectable markers located proximal to the left border", Plant Mol. Biol. 20: 1195-1197 oder in Bevan, M.W. (1984) "Binary Agrobacterium vectors for plant transformation", Nucl. Acid. Res. 12: 8711-8721.

Weiterhin können die erfindungsgemäßen Nukleinsäuresequenzen in Säugerzellen exprimiert werden. Beispiel für entsprechende Expressionsvektoren sind pCDM8 und pM-T2PC genannt in: Seed, B. (1987) Nature 329:840 oder Kaufman et al. (1987) EMBO J. 6:187-195). Dabei sind vorzugsweise zu nutzende Promotoren viralen Ursprungs wie z.B. Promotoren des Polyoma, Adenovirus 2, Cytomegalovirus oder Simian Virus 40. Weitere prokaryotische und eukaryotische Expressionssysteme sind genannt in Kapitel 16 und 17 in Sambrook et al., Molecular Cloning: A Laboratory Manual. 2nd, ed., Cold Spring Harbor Laboratory, Cold Spring Harbor Laboratory Press, Cold Spring Harbor, NY, 1989. Weitere vorteilhafte Vektoren werden in Hellens et al. (Trends in plant science, 5, 2000) beschrieben.

Sämtliche, oben beschriebenen Ausführungsformen der transgenen Organismen werden unter dem Begriff "erfindungsgemäßer transgener Organismus" zusammengefaßt.

Ein weiterer Gegenstand der vorliegenden Erfindung ist die Verwendung von SSP in einem Verfahren zur Identifizierung von Testverbindungen mit herbizider Wirkung.

Bevorzugt umfasst das erfindungsgemäße Verfahren zur Identifizierung von Verbindungen mit herbizider Wirkung die folgenden Schritte:
i. Inkontaktbringen von SSP mit einer oder mehreren Testverbindungen unter Bedingungen, die die Bindung der Testverbindung(en) an die SSP erlauben; und
ii. Nachweis, ob die Testverbindung an die SSP aus i) bindet; oder
iii. Nachweis, ob die Testverbindung die Aktivität der SSP aus i) reduziert oder blockiert; oder
iv. Nachweis, ob die Testverbindung die Transkription, Translation oder Expression der SSP reduziert oder blockiert.

Der Nachweis gemäß Schritt (ii) des oben stehenden Verfahrens kann anhand von Techniken, welche die Wechselwirkung zwischen Protein und Ligand aufzeigen, erfolgen. Hierbei kann entweder die Testverbindung oder das Enzym eine detektierbare Markierung enthalten, wie z.B. eine fluoreszierende, radioisotope, chemilumineszierende oder enzymatische Markierung. Beispiele für enzymatische Markierungen sind Meerrettich Peroxidase, alkalische Phosphatase oder Luzifierase. Die anschließende Detektion richtet sich nach der Markierung und ist dem Fachmann bekannt.

Hierbei sind insbesondere fünf bevorzugte Ausführungsformen zu nennen, die im Zusammenhang mit der vorliegenden Erfindung auch für Hochdurchsatzmethoden (High Throughput Screening, HTS) geeignet sind:
1. Über Fluoreszenz-Korrelations-Spektroskopie (FCS) (Proc. Natl. Acad. Sci. USA (1994) 11753-11575) läßt sich die durchschnittliche Diffusionsrate eines Fluoreszenzmoleküls in Abhängigkeit zur Masse in einem kleinen Probenvolumen bestimmen. Durch Messen der Massenänderung bzw. der daraus resultierenden veränderten Diffusionsrate einer Testverbindung beim Binden an die SSP läßt sich FCS zur Bestimmung von Protein-Ligand-Wechselwirkungen einsetzten. Ein erfindungsgemäßes Verfahren kann direkt zur Messung der Bindung einer durch ein Fluoreszenzmolekül markierten Testverbindung aufgebaut werden. Alternativ kann das erfindungsgemäße Verfahren so konzipiert sein, dass eine durch ein Fluoreszenzmolekül markierte chemische Referenzverbindung durch weitere Testverbindungen verdrängt wird ("Verdrängungsassay"). Die so identifizierten Verbindungen können als Inhibitoren geeignet sein.
2. Die Fluoreszenzpolarisation nutzt die Eigenschaft eines mit polarisiertem Licht angeregten ruhenden Fluorophors ebenfalls wieder polarisiertes Licht zu emittieren. Kann der Fluorophor allerdings während des angeregten Zustands rotieren, so geht die Polarisation des emittierten Fluoreszenzlichts mehr oder weniger verloren. Bei sonst gleichen Bedingungen (z.B. Temperatur, Viskosität, Lösungsmittel) ist die Rotation eine Funktion der Molekülgröße, womit man über das Messsignal eine Aussage über die Größe des am Fluorophor gebundenen Rests treffen kann (Methods in Enzymology 246 (1995), pp. 283-300). Ein erfindungsgemäßes Verfahren kann direkt zur Messung der Bindung einer durch ein Fluoreszenzmolekül markierten Testverbindung an die SSP aufgebaut werden. Alternativ kann das erfindungsgemäße Verfahren auch in Form des unter 1 beschriebenen "Verdrängungsassays" konzipiert sein. Die so identifizierten Verbindungen können als Inhibitoren geeignet sein.
3. Fluoreszenz-Resonanz Energie Tranfer" (FRET) basiert auf der strahlungslosen Energieübertragung zwischen zwei räumlich benachbarten Fluoreszenzmolekülen unter geeigneten Bedingungen. Eine Voraussetzung ist die Überlappung des Emissionsspektrums des Donormoleküls mit dem Anregungsspektrum des Akzeptormoleküls. Durch Fluoreszenzmarkierung der SSP und den auf Bindung Tetsverbindung kann mittels FRET die Bindung gemessen werden (Cytometry 34, 1998, pp. 159-179). Alternativ kann das erfindungsgemäße Verfahren auch in Form des unter 1 beschriebenen "Verdrängungsassays" konzipiert sein. Eine besonders geeignete Ausführungsform der FRET Technologie ist die "Homogenous Time Resolved Fluorescence" (HTRF), wie sie von Packard BioScience vertrieben wird. Die so identifizierten Verbindungen können als Inhibitoren geeignet sein.
4. Surface Enhanced-Laser Desorption/lonisation (SELDI) in Kombination mit einem "Time of Flight" Massenspektrometer (MALDI-TOF) ermöglicht die schnelle Analyse von Molekülen auf einem Träger und kann zur Analyse von Protein-Ligand Wechselwirkungen verwendet werden (Worral et al., (1998) Anal. Biochem. 70:750-756). In einer bevorzugten Ausführungsform immobilisiert man nun die SSP auf einem geeigneten Träger und inkubiert diesen mit der Testverbindung. Nach einem oder mehreren geeigneten Waschschritten kann man die an die SSP zusätzlich gebundenen Moleküle der Testverbindung mittels der oben erwähnten Methodik detektieren und somit an die SSP gebundene Testverbindungen selektieren. Die so identifizierten Verbindungen können als Inhibitoren geeignet sein.
5. Die Messung von Oberflächen Plasmonenresonanz basiert auf der Änderung des Brechnungsindexes an einer Oberfläche beim Binden einer Testverbindung an ein auf besagter Oberfläche immobilisierten Protein. Da die Änderung des Brechungsindex für eine definierte Änderung der Massenkonzentration an der Oberfläche quasi für alle Proteine und Polypeptide identisch ist, kann diese Methode prinzipiell auf jedes Protein angewendet werden (Lindberg et al. Sensor Actuators 4 (1983) 299-304; Malmquist Nature 361 (1993) 186-187). Die Messung kann beispielsweise mit Hilfe der von Biacore (Freiburg) vertriebenen auf Oberflächen-Plasmonenresonanz basierenden Analyseautomaten in einem Durchsatz von derzeit bis zu 384 Proben pro Tag durchgeführt werden. Ein erfindungsgemäßes Verfahren kann direkt zur Messung der Bindung einer Testverbindung an die SSP aufgebaut werden. Alternativ kann das erfindungsgemäße Verfahren auch in Form des unter 1 beschriebenen "Verdrängungsassays" konzipiert sein. Die so identifizierten Verbindungen können als Inhibitoren geeignet sein.

Sämtliche, über die oben genannten Verfahren identifizierten Substanzen können anschließend in einer anderen Ausführungsform des erfindungsgemäßen Verfahrens auf ihre herbizide Wirkung überprüft werden.

Auch besteht die Möglichkeit, über Aufklärung der dreidimensionalen Struktur der SSP mittels Röntgenstrukturanalyse weitere potentielle herbizide Wirkstoffe mittels "Molecular Modelling" zu detektieren. Die Herstellung von für die Röntgenstrukturanalyse benötigten Proteinkristallen sowie die entsprechenden Messungen und anschließenden Auswertungen dieser Messungen, die Detektion einer Bindungstelle im Protein sowie die Vorhersage möglicher Inhibitorstrukturen sind dem Fachmann bekannt. Über "Molecular Modelling" ist prinzipiell auch eine Optimierung der über die oben genannten Verfahren identifizierten Verbindungen möglich.

Eine bevorzugte Ausführungsform des erfindungsgemäßen Verfahrens, das auf den Schritten i) und ii) basiert, besteht darin, daß
i. eine SSP in einem erfindungsgemäßen, transgenen Organismus exprimiert wird oder ein Organismus, der naturgemäß eine SSP enthält, kultiviert wird;
ii. die SSP aus Schritt i) im Zellaufschluss des transgenen bzw. nicht transgenen Organismus, in partiell gereinigter Form oder in zur Homogenität gereinigten Form mit einer Testverbindung in Kontakt gebracht wird; und
iii. eine Verbindung selektiert wird, welche die Aktivität der SSP reduziert oder blockiert, wobei die Aktivität der mit der Testverbindung inkubierten SSP mit der Aktivität einer nicht mit einer Testverbindung inkubierten SSP ermittelt wird.

Die SSP enthaltende Lösung kann aus dem Lysat des ursprünglichen Organismus oder des transgenen, mit einer erfindungsgemäßen Expressionskassette transformierten Organismus bestehen. Falls erforderlich kann die SSP partiell oder vollständig über gängige Methoden aufgereinigen werden. Eine allgemeine Übersicht über gängige Techniken zur Reinigung von Proteinen sind beispielsweise in Ausubel, F.M. et al., Current Protocols in Molecular Biology, Greene Publishing Assoc. and Wiley-Interscience (1994); ISBN 0-87969-309-6 beschrieben. Bei rekombinanter Darstellung kann eine Reinigung des mit einem Affinitäts-Tag fusionierten Proteins über nach dem Fachmann bekannten Affinitätschromoatographie erfolgen.

Die für in vitro Verfahren benötigte SSP kann somit entweder mittels heterologer Expression aus einem erfindungsgemäßen, transgenen Organismus oder aus einem Organismus, der eine SSP Aktivität enthält, isoliert werden, vorzugsweise aus einer unerwünschten Pflanze, wobei unter dem Begriff der unerwünschten Pflanze die eingangs erwähnten Spezies zu verstehen sind.

Zur Identifizierung von herbiziden Verbindungen wird nun die SSP mit einer Testverbindung inkubiert: Nach einer Reaktionszeit wird die Aktivität der mit der Testverbindung inkubierten SSP mit der Aktivität einer nicht mit einer Testverbindung inkubierten SSP ermittelt. Bei Inhibition der SSP beobachtet man eine signifikante Abnahme der Aktivität im Vergleich zur Aktivität des nicht inhibierten erfindungsgemäßen Polypeptides,
wobei eine Abnahme von mindestens 10%, vorteilhaft mindestens 20%, bevorzugt mindestens 30%, besonders bevorzugt um mindestens 50% bis hin zu einer 100% Reduktion (Blockierung) erzielt wird. Bevorzugt mindestens 50% Hemmung bei Konzentrationen der Testverbindung von 10⁻⁴ M, bevorzugt bei 10⁻⁵M, besonders bevorzugt von 10⁻⁶ M bezogen auf Enzymkonzentration im mikromolaren Bereich.

Die Bestimmung der enzymatischen Aktivität der SSP kann beispielsweise über einen Aktivitätstest erfolgen, in welchem die Zunahme des Produktes, die Abnahme des Substrates (oder Eduktes) oder über eine Kombination aus mindestens zwei der vorstehend genannten Parameter in Abhängigkeit einer definierten Zeitspanne bestimmt werden.

Beispiele für geeignete Substrate sind z.B. Saccharose-6-phosphat oder Nitrophenylverbindungen wie z.B. p-Nitrophenylphosphat, vorzugsweise Saccharose-6-phosphat, und für geeignete Cofaktoren zweiwertige Metalle wie Magnesium oder Mangan, vorzugsweise Magnesium. Gegebenenfalls können auch Derivate der vorstehend genannten Verbindungen verwendet werden, die eine detektierbare Markierung enthalten, wie z.B. eine fluoreszierende, radioisotope oder chemilumineszierende Markierung.

Die für den Aktivitätstest einzusetzenden Mengen an Substrat können zwischen 0.5-10 mM und Mengen an Cofaktor zwischen 0.1-5 mM bezogen auf 1-100 µg/ml Enzym liegen.

Die Bestimmung der Aktivität kann beispielsweise in Analogie zu dem von Echeverria und Salerno (1994; Plant Sci 96, 15) beschriebenen Verfahren oder nach dem von Whitaker (1984) Phytochemistry 23, 2429) beschriebenen Verfahren erfolgen.

In einer besonders bevorzugten Ausführungsform wird der Umsatz eines Substrates über Quantifizierung des bei der Reaktion entstandenden Phosphates mittels Ascorbat-Ammoniummolybdat-Reagenzes (Ames (1966), Methods Enzymol. 8, 115) erfolgen angelehnt an ein von Lunn et al. (2000, Procl. Natl. Acad. Sci. USA 97:12914) beschriebendes Verfahren. Es können jedoch auch Abwandlungen der von Ames beschrieben Methode zur Phosphatdetektion verwendet werden z.B. das von Chifflet et al. (1988) Analytical Biochemistry 168: 1) beschriebene Verfahren, welche sich besonders für labile organische Phosphate und in Anwesenheit hoher Proteinkonzentrationen eignet, das von Lanzetta et al. (1979, Analytical Biochemistry 100: 95) beschriebene Verfahren, welches eine Methode zum Nachweis von Phosphat im Nanomol-Bereich umfasst, bei der der entstehende Farbkomplex besonders stabilisiert wird. Ebenfalls verwendet werden zur Detektion können kommerziell erhältliche Kits z.B. von Merck (Phosphat-Test (PMB) AM Katalognummer 1.11139.0001)).

In einer weiteren bevorzugten Ausführungsform kann die Bestimmung der Aktivität der aus Saccharose-6-P freigesetzte Saccharose bestimmt werden. Hierzu kommen z.B. optisch-Enzymatische Verfahren in betracht wie z.B. beschrieben bei Sonnewald (1992, Plant Journal 2: 571 ) oder chromatographische Methoden mittels HPLC (Bömke et al. 2001, J Bacteriol 183: 2425). Der Fachmann kann darüber hinaus auch Verfahren zum chemischen Nachweis der entstandenen Saccharose der Literatur entnehmen.

Eine bevorzugte Ausführungsform des erfindungsgemäßen Verfahrens, das auf den Schritten i) und iii) basiert, besteht aus den folgenden Schritten:
i. Herstellung eines erfindungsgemäßen transgenen Organismus;
ii. Aufbringen einer Testverbindung auf den transgenen Organismus nach i) und auf einen nicht-transgenen Organismus des gleichen Genotyps;
iii. Bestimmen des Wachstums oder der Überlebensfähigkeit des transgenen und des nicht transgenen Organismus nach der Aufbringung der Testverbindung; und
iv. Selektion von Testverbindungen, die ein vermindertes Wachstum oder eingeschränkte Überlebensfähigkeit des nicht-transgenen Organismus bewirken verglichen mit dem Wachstum des transgenen Organismus bewirken.

In diesem Verfahren wird in dem transgenen Organismus gemäß i) das Polypeptid mit der biologischen Aktivität einer SSP überexprimiert. Der transgene Organismus weist somit gegenüber einer nicht-transgenen Organismus eine erhöhte SSP-Aktivität auf, wobei unter erhöhter SSP-Aktivität des transgenen Organismus eine gegenüber dem nicht transgenen Organismus der gleichen Gattung um mindestens 10%, bevorzugt um mindestens 25%, besonders bevorzugt um mindestens 40%, ganz besonders bevorzugt um mindestens 50% höhere Aktivität zu verstehen ist.

Hierbei beträgt der Wachstumsunterschied der in Schritt iv) zur Selektion eines Inhibitors mit herbizider Wirkung mindestens 10%, vorzugsweise 20%, bevorzugt 30%. besonders bevorzugt 40% und ganz besonders bevorzugt 50%.

Der transgene Organismus ist hierbei eine Pflanze, Alge, ein Cyanobakterien z.B. der Gattung Synechocystes, Anabaena, Calothrix, Scytonema, Oscillatoria, Plectonema und Nostoc, oder ein Proteobakterium wie etwa Magnetococcus sp. MC1, bevorzugt Pflanzen, die sich mittels gängiger Techniken leicht transformieren lassen, wie Arabidopsis thaliana, Solanum tuberosum, Nicotiana Tabacum, Cyanobakterien die sich leicht transformieren lassen, wie z.B. Synechocystis, in welchen die für ein erfindungsgemäße Polypeptid kodierende Sequenz über Transformation inkorporiert wurde. Diese transgenen Organismen weisen daher eine erhöhte Toleranz gegen Verbindungen auf, welche das erfindungsgemäße Polypeptid inhibieren. Hierbei können auch "knock-out"-Mutanten verwendet werden können bei denen das in diesem Organismus vorhandene analoge SSP-Gen gezielt ausgeschaltet worden ist.

Die vorstehend genannte Ausführungsform des erfindungsgemäßen Verfahrens kann jedoch auch zur Identifizierung von Substanzen mit wachstumsregulatorischer Wirkung verwendet werden. Hierbei wird als transgener Organismus eine Pflanze eingesetzt. Das Verfahren zur Identifizierung von Substanzen mit wachstumsregulatorischer Wirkung umfasst somit die folgenden Schritte:
i. Herstellung einer transgenen Pflanze enthaltend eine erfindungsgemäße Nukleinsäuresequenz;
ii. Aufbringen einer Testsubstanz auf die transgene Pflanze nach i) und auf eine nicht-transgene Pflanze der gleichen Sorte;
iii. Bestimmen des Wachstums oder der Überlebensfähigkeit der transgenen und der nicht transgenen Pflanze nach dem Aufbringen der Testsubstanz; und
iv. Selektion von Testsubstanzen, die ein verändertes Wachstum der nicht-transgenen Pflanze bewirken verglichen mit dem Wachstum der transgenen Pflanze.

In diesem Verfahren wird in der transgenen Pflanze gemäß i) das Polypeptid mit der biologischen Aktivität einer SSP überexprimiert. Die transgene Pflanze weist somit gegenüber einer nicht-transgenen Pflanze der gleichen Gattung eine erhöhte SSP-Aktivität auf, wobei unter erhöhter SSP-Aktivität der transgenen Pflanze eine gegenüber einer nicht transgenen Pflanze der gleichen Gattung eine um mindestens 10% , bevorzugt um mindestens 25%, besonders bevorzugt um mindestens 40%, ganz besonders bevorzugt um mindestens 50% höhere Aktivität zu verstehen ist.

Hierbei werden in Schritt iv) Testverbindungen selektiert, die ein verändertes Wachstum des nicht-transgenen Organismus bewirken verglichen mit dem Wachstum des transgenen Organismus bewirken. Unter verändertem Wachstum ist hierbei eine Hemmung des vegetativen Wachstums der Pflanzen zu verstehen, was sich insbesondere in einer Reduzierung des Längenwachstums äußeren kann. Die behandelten Pflanzen weisen demgemäß einen gedrungenen Wuchs auf; außerdem ist eine dunklere Blattfärbung zu beobachten. Weiterhin ist unter verändertem Wachstum auch eine zeitliche Veränderung des Reifeverlaufs, eine Heummung oder Vermehrungen seitlicher Verzweigungen der Pflanzen, eine Verkürzung bzw. Verlängerung der Entwicklungsstadien, eine Erhöhung der Standfestigkeit, das Wachstum größerer Mengen an Knospen, Blüten, Blättern, Früchten, Samenkörnern, Wurzeln und Knollen, eine Erhöhung des Zuckergehaltes in Pflanzen wie Zuckerrüben, Zuckerrohr sowie Zitrusfrüchten, des Proteingehaltes in Pflanzen wie Getreide oder Soja oder eine Stimulierung des Latexfluß an Gummibäumen zu verstehen. Die Detektion dieses veränderten Wachstums ist dem Fachmann bekannt.

Nach dem erfindungsgemäßen Verfahren können auch mehrere Testverbindungen in einem erfindungsgemäßen Verfahren eingesetzt werden. Wenn durch eine Gruppe von Testverbindungen eine Beeinflussung des Targets erfolgt, dann ist es entweder möglich, die einzelnen Testverbindungen direkt zu isolieren oder die Gruppe von Testverbindungen in verschiedene Untergruppen zu teilen, z.B. wenn sie aus einer Vielzahl von verschiedenen Komponenten besteht, um so die Zahl der verschiedenen Testverbindungen im erfindungsgemäßen Verfahren zu reduzieren. Das erfindungsgemäße Verfahren wiederholt man dann mit der einzelnen Testverbindung oder der entsprechenden Untergruppe von Testverbindungen. Abhängig von der Komplexität der Probe können die oben beschriebenen Schritte mehrmals wiederholt werden, vorzugsweise bis die gemäß der erfindungsgemäßen Methode identifizierte Untergruppe nur noch eine geringe Anzahl von Testverbindungen oder nur noch eine Testverbindung umfaßt.

Alle oben beschriebenen Verfahren für die Identifizierung von Inhibitoren mit herbizider Wirkung werden im folgenden als "erfindungsgemäße Verfahren" bezeichnet.

Sämtliche, über die erfindungsgemäßen Verfahren identifizierten Verbindungen können anschließend auf ihre herbizide Wirkung in vivo überprüft werden. Eine Möglichkeit zur Prüfung der Verbindungen auf herbizide Wirkung ist die Verwendung der Wasserlinse Lemna minor in Mikrotiterplatten. Als Parameter können Veränderungen des Chlorophyllgehalts und die Photosyntheseleistung gemessen werden. Es ist auch möglich, die Verbindung auf unerwünschte Pflanzen direkt zu applizieren, wobei die herbizide Wirkung z.B. über eingeschränktes Wachstum festgestellt werden kann.

Das erfindungsgemäße Verfahren kann auch vorteilhaft in Hochdurchsatzverfahren, sog. HTS durchgeführt werden, welches das parallele Testen einer Vielzahl verschiedener Verbindungen ermöglicht.

Im HTS bietet sich für die praktische Durchführung die Verwendung von Trägem an, die eines oder mehrere der erfindungsgemäßen Nukleinsäuremoleküle, einen oder mehrere die erfindungsgemäße Nukleinsäuresequenz enthaltenden Vektoren, einen oder mehrere transgene Organismen, welche mindestens eine der erfindungsgemäßen Nukleinsäuresequenzen enthalten oder eines oder mehrere (Poly)peptide codiert über die erfindungsgemäßen Nukleinsäuresequenzen enthalten. Der verwendete Träger kann fest oder flüssig sein, ist bevorzugt fest, besonders bevorzugt eine Mikrotiterplatte. Gemäß der am weit verbreitesten Technik werden 96-well, 384-well und 1536 well Mikrotiterplatten verwendet, die in der Regel Volumina von 200µl umfassen können. Neben den Mikrotiterplatten sind die weiteren Bestandteile eines HTS-Systems passend zu den entsprechenden Mikrotiterplatten wie viele Instrumente, Materialien, automatische Pipettiervorichtungen, Robotoren, automatisierte Plattenleser sowie Plattenwascher kommerziell erhältlich.

Neben den auf Mikrotiterplatten basierenden HTS-Verfahren können auch sogenannte "free format assays" oder Testsysteme, die zwischen den Proben keine physischen Barrieren aufweisen, verwendet werden wie z.B. in Jayaickreme et al., Proc. Natl. Acad. Sci U.S.A. 19 (1994) 161418; Chelsky, "Strategies for Screening Combinatorial Libaries, First Annual Conference of The Society for Biomolecular Screening in Philadelphia, Pa. (Nov. 710, 1995); Salmon et al., Molecular Diversity 2 (1996), 5763 und US 5,976,813.

Verbindungen mit herbizider Wirkung identifiziert nach den erfindungsgemäßen Verfahren werden im folgenden "selektierte Verbindungen" genannt. Sie weisen ein Molekulargewicht von kleiner als 1000 g/mol, vorteilhaft kleiner 500 g/mol, bevorzugt kleiner 400 g/mol, besonders bevorzugt kleiner 300 g/mol. Verbindungen mit herbizider Wirkung weisen einem Ki-Wert kleiner 1 mM, bevorzugt kleiner 1 µM, besonders bevorzugt kleiner 0,1 µM ganz besonders bevorzugt kleiner 0,01 µM aufweisen.

Die selektierten Verbindungen können natürlich auch in Form ihrer landwirtschaft brauchbaren Salze vorliegen. Unter landwirtschaftlich brauchbaren Salzen kommen vor allem die Salze derjenigen Kationen oder die Säureadditionssalze derjenigen Säuren in Betracht, deren Kationen beziehungsweise Anionen die herbizide Wirkung der über die erfindungsgemäßen Verfahren identifizierten Verbindungen mit herbizider Wirkung nicht negativ beeinträchtigen.

Ferner können die selektierten Verbindungen sofern sie asymmetrisch substituierte a-Kohlenstoffatome enthalten, entweder als Racemate, Enantiomerengemische, reine Enantiomere oder, sofern sie chirale Substituenten aufweisen, auch als Diastereomerengemische vorliegen.

Die selektierten Verbindungen können chemisch synthetisierte oder mikrobiologisch produzierte Stoffe sein und z.B. in Zellextrakten von z.B. Pflanzen, Tieren oder Mikroorganismen auftreten. Das Reaktionsgemisch kann ein zellfreier Extrakt sein oder eine Zelle oder Zellkultur umfassen. Geeignete Methoden sind dem Fachmann bekannt und werden z.B. allgemein beschrieben in Alberts, Molecular Biology the cell, 3rd Edition (1994), z.B. Kapitel 17. Die selektierten Verbindungen können auch aus umfangreichen Substanzbibliotheken stammen.

Mögliche Testverbindungen können Expressionsbibliotheken wie z.B. cDNA-Expressionsbibliotheken, Peptide, Proteine, Nukleinsäuren, Antikörper, kleine organische Stoffe, Hormone, PNAs oder ähnliches sein (Milner, Nature Medicin 1 (1995), 879-880; Hupp, Cell. 83 (1995), 237-245; Gibbs, Cell. 79 (1994), 193-198 und darin zitierte Referenzen).

Die selektierten Verbindungen können zur Bekämpfung von unerwünschtem Pflanzenwuchs, unter Umständen auch zur Defoliation, beispielsweise von Kartoffeln, oder Desikkation, beispielsweise von Baumwolle, sowie als Wachstumsregulatoren verwendet werden. Herbizide Zusammensetzungen, welche die selektierten Verbindungen enthalten, bekämpfen Pflanzenwuchs auf Nichtkulturflächen sehr gut. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf. Die selektierten Verbindungen können zur Bekämpfung der oben bereits erwähnten Schadpflanzen verwendet werden.

In Abhängigkeit von der jeweiligen Applikationsmethode können selektierten Verbindungen bzw. diese enthaltende herbizide Zusammensetzungen vorteilhaft noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium). Helianthus annuus, Hevea brasiliensis, Hordeum vulgare, Humulus lupulus, lpomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die selektierten Verbindungen auch in Kulturen, die durch Züchtung einschließlich gentechnischer Methoden gegen die Wirkung von Herbiziden tolerant sind, verwandt werden. Die Herstellung dieser Kulturen wird weiter unten beschrieben.

Ein Verfahren zur Herstellung der oben bereits erwähnten herbiziden Zusammensetzung, ist dadurch gekennzeichnet dass man selektierten Verbindungen mit geeigneten Hilfsmitteln zu Pflanzenschutzmitteln formuliert.

Die selektierten Verbindungen können z.B. in Form von direkt versprühbaren wässrigen Lösungen, Pulvern, Suspensionen, auch hochprozentigen wäßrigen, öligen oder sonstigen Suspensionen bzw. Suspoemulsionen oder Dispersionen, emulgierbaren Konzentraten, Emulsionen, Öldispersionen, Pasten, Stäubemitteln, Streumitteln oder Granulaten formuliert werden und durch Versprühen, Vernebeln, Verstäuben, Verstreuen oder Gießen angewendet werden. Die Anwendungsformen richten sich nach den Verwendungszwecken und der Natur der selektierten Verbindungen und sollte in jedem Fall möglichst die feinste Verteilung der selektierten Verbindungen gewährleisten. Die herbiziden Zusammensetzung enthalten eine herbizid wirksame Menge mindestens einer selektierten Verbindung und für die Formulierung von herbiziden Zusammensetzungen übliche Hilfsstoffe.

Zur Herstellung von Emulsionen, Pasten oder wässrigen oder ölhaltigen Formulierungen sowie dispergierbaren Konzentraten (DC) können die selektierten Verbindungen in einem Öl oder Lösungsmittel gelöst oder dispergiert werden, wobei weitere Formulierungshilfsstoffe zur Homogenisierung zugesetzt werden können. Es können aber auch aus selektierter Verbindung, gegebenenfalls Lösungsmitteln oder Öl sowie optional weiteren Hilfsmitteln bestehende flüssige oder feste Konzentrate hergestellt werden, die zur Verdünnung mit Wasser geeignet sind. Hier zu nennen sind emulgierbare Konzentrate (EC, EW), Suspensionen (SC), lösliche Konzentrate (SL), dispergierbaren Konzentrate (DC), Pasten, Pastillen netzbaren Pulvern oder Granulaten, wobei die festen Formulierungen entweder in Wasser löslich (soluble) oder dispergierbar (wettable) sein können. Desweiteren können entsprechende Pulver bzw. Granulate oder Tabletten noch mit einem festen, den Abrieb oder eine vorzeitige Wirkstofffreisetzung verhinderenden Überzug ("coating") versehen werden.

Prinzipiell sind unter dem Begriff "Hilfsmittel" folgende Verbindungsklassen zu verstehen: Antischäumungsmittel, Verdicker, Netzmittel, Haftmittel, Dispergiermittel, Emulgiermittel, Bakterizide und/oder thixotrophe Agentien. Die Bedeutung der oben genannten Mittel ist dem Fachmann bekannt.

SLs, EWs und ECs können durch einfaches Mischen der entsprechenden Inhaltsstoffe hergestellt werden, Pulver über Mischen oder Vermahlen in speziellen Mühlentypen (z.Bsp. Hammermühlen). DC, SCs und SEs werden üblicherweise über Naßvermahlung ("wet milling") hergestellt, wobei ein SE aus einem SC durch Zugabe einer organischen Phase, die weitere Hilfsmittel oder selektierte Verbindungen enthalten kann, hergestellt werden kann. Die Herstellung ist bekannt. Pulver-, Streu- und Stäubemittel können vorteilhaft durch Mischen oder gemeinsames Vermahlen der wirksamen Substanzen mit einem festen Trägerstoff hergestellt werden. Granulate, z.B. Umhüllungs-, Imprägnierungs- und Homogengranulate können durch Bindung der selektierten Verbindungen an feste Trägerstoffe hergestellt werden. Weitere Details der Herstellung sind dem Fachmann bekannt, und z.Bsp. in folgenden Schriften aufgeführt: US 3,060,084, EP-A 707445 (für flüssige Konzentrate), Browning, "Agglomeration", Chemical Engineering, Dec. 4, 1967, 147-48, Peny's Chemical Engineer's Handbook, 4th Ed., McGraw-Hill, New York, 1963, pages 8-57 und ff. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York, 1961, Hance et al., Weed Control Handbook, 8th Ed., Blackwell Scientific Publications, Oxford, 1989 und Mollet, H., Grubemann, A., Formulation technology, Wiley VCH Verlag GmbH, Weinheim (Federal Republic of Germany), 2001.

Inefte flüssige und/oder feste Trägerstoffe geeignet für die erfindungsgemäßen Formulierungen sind dem Fachman in einer Vielzahl bekannt, wie z.Bsp. flüssige Zusatzstoffe wie Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Feste Trägerstoffe sind beispielsweise Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Oberflächenaktive Stoffe (Tenside) geeignet für die erfindungsgemäßen Formulierungen sind dem Fachman in einer Vielzahl bekannt, wie z.Bsp. Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-; Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose.

Die Applikation der herbiziden Zusammensetzungen bzw. der selektierten Verbindungen kann im Vorauflauf oder im Nachauflaufverfahren erfolgen. Sind die selektierten Verbindungen für gewisse Kulturpflanzen weniger verträglich, so können Ausbringungstechniken angewandt werden, bei welchen die selektierten Verbindungen mit Hilfe der Spritzgeräte so gespritzt werden, daß die Blätter der empfindlichen Kulturpflanzen nach Möglichkeit nicht getroffen werden, während die selektierten Verbindungen auf die Blätter darunter wachsender unerwünschter Pflanzen oder die unbedeckte Bodenfläche gelangen (post-directed, lay-by).

Die Aufwandmengen an selektierten Verbindungen betragen je nach Bekämpfungsziel, Jahreszeit, Zielpflanzen und Wachstumsstadium 0.001 bis 3.0, vorzugsweise 0.01 bis 1.0 kg/ha.

Die Bereitstellung des herbiziden Targets ermöglicht weiterhin ein Verfahren zur Identifizierung eines Proteins mit der biologischen Aktivität einer SSP, welches nicht oder nur eingeschränkt durch ein Herbizid, welches als Wirkort die SSP hat, z.B. die herbizid wirkenden selektierten Verbindungen, gehemmt wird. Im folgenden wird ein sich derart von der SSP unterscheidendes Protein als SSP-Variante bezeichnet, welches durch eine Nukleinsäuresequenz codiert wird, welche
i) für ein Polypeptid mit der biologischen Aktivität einer Saccharose-6-Phosphat Phosphatase kodieren, welche durch nach den oben genannten Verfahren ermittelte Substanzen mit herbizider Wirkung, welche SSP inhibieren, nicht inhibiert wird;
ii) durch ein funktionelles Äquivalent der Nukleinsäuresequenz SEQ ID NO:1 mit einer Identität von mindestens 55% zu der SEQ ID NO:1; oder ein funktionelles Äquivalente der Nukleinsäuresequenz SEQ ID NO:3 mit einer Identität von mindestens 55% zu der SEQ ID NO:3; oder funktionelle Äquivalente der Nukleinsäuresequenz SEQ ID NO:5 mit einer Identität von mindestens 51% zu der SEQ ID NO:5 kodiert werden.

In einer bevorzugten Ausführungsform besteht das oben genannte Verfahren zur Erzeugung von Nukleinsäuresequenzen codierend für SSP-Varianten von Nukleinsäuresequenzen umfassen aus folgenden Schritten:
a) Expression der von den oben genannten Nukleinsäuren kodierten Proteine in einem heterologen System oder in einem zellfreien System;
b) Randomisierte oder gerichtete Mutagenese des Proteins durch Modifikation der Nukleinsäure;
c) Messung der Interaktion des veränderten Genprodukts mit dem Herbizid;
d) Identifizierung von Derivaten des Proteins die eine geringere Interaktion aufweisen;
e) Testung der biologischen Aktivität des Proteins nach Applikation des Herbizides;
f) Auswahl der Nukleinsäuresequenzen, die eine veränderte biologische Aktivität gegenüber dem Herbizid aufweisen.

Die funktionellen Äquivalente der SEQ ID NO:1 gemäß ii) weisen eine Homologie mit der SEQ ID NO:1 von mindestens 55%, 56%, 57%, 58%, 59%, 60%, 61%, 62%, 63%, 64%, 65%, 66%, 67%, 68% vorzugsweise mindestens 69%, 70%, 71%, 72%, 73%, 74% vorzugsweise mindestens 75%, 76%, 77%, 78%, 79%, 80%, 81%, 82%, 83% bevorzugt mindestens 84%, 85%, 86%, 87%, 88%, 89%, 89%, 90%, 91%, 92%, 93% besonders bevorzugt mindestens 94%, 95%, 96%, 97%, 98%, 99% auf.

Die funktionellen Äquivalente der SEQ ID NO:3 gemäß ii) weisen eine Homologie mit der SEQ ID NO:3 von mindestens 55%, 56%, 57%, 58%, 59%, 60%, 61%, 62%, 63%, 64%, 65%, 66%, 67%, 68% vorzugsweise mindestens 69%, 70%, 71%, 72%, 73%, 74% vorzugsweise mindestens 75%, 76%, 77%, 78%, 79%, 80%, 81%, 82%, 83% bevorzugt mindestens 84%, 85%, 86%, 87%, 88%, 89%, 89%, 90%, 91%, 92%, 93% besonders bevorzugt mindestens 94%, 95%, 96%, 97%, 98%, 99% auf.

Die funktionellen Äquivalente der SEQ ID NO:5 gemäß ii) weisen eine Homologie mit der SEQ ID NO:5 von mindestens 51%, 52%, 53%, 54%, 55%, 56%, 57%, 58%, 59%, 60%, 61%, 62% vorzugsweise mindestens 63%, 64%, 65%, 66%, 67%, 68%, 69%, 70%, 71%, 72%, 73%, 74% vorzugsweise mindestens 75%, 76%, 77%, 78%, 79%, 80%, 81%, 82%, 83% bevorzugt mindestens 84%, 85%, 86%, 87%, 88%, 89%. 89%, 90%, 91%, 92%, 93% besonders bevorzugt mindestens 94%, 95%, 96%, 97%, 98%, 99% auf.

Die nach dem oben beschriebenen Verfahren nach ausgewählten Sequenzen werden vorteilhaft in einen Organismus eingebracht. Deshalb ist ein weiterer Erfindungsgegenstand ein nach diesem Verfahren hergestellter Organismus. Bevorzugt ist der Organismus eine Pflanze, besonders bevorzugt eine der oben definierten Kulturpflanzen.

Anschließend erfolgt die Regeneration ganzer Pflanzen und Überprüfung der Resistenz gegenüber der selektierten Verbindung in intakten Pflanzen.

Veränderte Proteine und/oder Nukleinsäuren, die in Pflanzen Resistenz gegen die selektierten Verbindungen vermitteln können, können aus den oben genannten Nukleinsäuresequenzen auch Über die sogenannte "site directed mutagenesis" hergestellt werden, durch diese Mutagenese kann beispielsweise die Stabilität und/oder Aktivität des Target Proteins oder die Eigenschaften wie Bindung und Wirkung der oben genannten erfindungsgemäßen Inhibitoren sehr gezielt verbessern bzw. verändert werden.

Beispielsweise wurde von Zhu et al. (Nature Biotech., Vol. 18, May 2000: 555 - 558) eine "site directed mutagenesis"-Methode in Pflanzen beschrieben, die vorteilhaft verwendet werden kann.

Weiterhin können Veränderungen über die von Spee et al. (Nucleic Acids Research, Vol. 21, No. 3, 1993: 777- 78) beschriebenen PCR-Methode unter Verwendung von dlTP zur zufälligen Mutagenese erzielt werden oder durch die von Rellos et al. (Protein Expr. Purif., 5, 1994 : 270-277) weiter verbessert Methode.

Eine weitere Möglichkeit zur Herstellung dieser veränderten Proteine und/oder von Nukleinsäuren ist eine von Stemmer et al. (Proc. Natl. Acad. Sci. USA, Vol. 91, 1994: 10747-10751) beschriebene "in vitro" Rekombinationstechnik für die molekulare Evolution oder die von Moore et al. (Nature Biotechnology Vol. 14, 1996: 458-467) beschriebene Kombination der PCR- und Rekombinationsmethode.

Ein weiterer Weg zur Mutagenese von Proteinen wird von Greener et al. in Methods in Molecular Biology (Vol. 57, 1996: 375-385) beschrieben. In EP-A-0 909 821 wird eine Methode zur Veränderung von Proteinen unter Verwendung des Mikroorganismus E. coli XL-1 Red beschrieben. Dieser Mikroorganismus erzeugt bei der Replikation Mutantionen in den eingeführten Nukleinsäuren und führt so zu einer Veränderung der genetischen Information. Über Isolierung der veränderten Nukleinsäuren bzw. der veränderten Proteine und Testung auf Resistenz lassen sich leicht vorteilhafte Nukleinsäuren und die durch sie kodierten Proteine identifizieren. Diese können dann nach Einbringen in Pflanzen dort die Resistenz ausprägen und so zur Resistenz gegen die Herbizide führen.

Weitere Methoden der Mutagenese und Selektion sind beispielsweise Methoden wie die in vivo Mutagenese von Samen oder Pollen und Selektion resistenter Allele in Anwesenheit der erfindungsgemäßen Inhibitoren, gefolgt von genetischer und molekularer Identifizierung des veränderten, resistenten Allels. Weiterhin die Mutagenese und Selektion von Resistenzen in der Zellkultur durch Vermehrung der Kultur in Anwesenheit von sukzessiv steigenden Konzentrationen der erfindungsgemäßen Inhibitoren. Dabei kann die Erhöhung der spontanten Mutationsrate durch chemische/physikalische mutagene Behandlung ausgenutzt werden. Wie vorgehend beschrieben lassen sich auch mit Mikroorgansimen, die eine endogene oder rekombinante Aktivität der durch die im erfindungsgemäßen Verfahren verwendeten Nukleinsäuren codierten Proteine haben, und die gegenüber den erfindungsgemäß identifizierten Inhibitoren sensitiv sind, veränderte Gene isolieren. Die Anzucht der Mikroorganismen auf Medien mit steigenden Konzentration von erfindungsgemäßen Inhibitoren erlaubt die Selektion und Evolution von resistenten Varianten der erfindungsgemäßen Targets. Die Frequenz der Mutationen kann wiederum durch mutagene Behandlungen erhöht werden.

Daneben stehen Verfahren zur gezielten Veränderungen von Nukleinsäuren zur Verfügung (Zhu et al. Proc. Natl. Acad. Sci. USA, Vol. 96, 8768 - 8773 und Beethem et al., Proc. Natl. Acad. Sci. USA, Vol 96, 8774 - 8778). Diese Methoden ermöglichen es, in den Proteinen solche Aminosäuren, die für die Bindung von Inhibitoren von Bedeutung sind, durch funktionell analoge Aminosäuren zu ersetzen, die jedoch die Bindung des Inhibitors verhindern.

Ein weiterer Erfindungsgegenstand ist deshalb ein Verfahren zur Erstellung von Nukleinsäuresequenzen, welche für Genprodukte kodieren, die eine veränderte biologische Aktivität aufweisen, wobei die biologische Aktivität dahingegen verändert wurde, daß eine erhöhte Aktivität vorliegt. Unter erhöhter Aktivität ist eine gegenüber dem Ausgangsorganismüs bzw. gegenüber dem Ausgangsgenprodukt um mindestens 10% , bevorzugt um mindestens 30%, besonders bevorzugt um mindestens 50%, ganz besonders bevorzugt um mindestens 100% höhere Aktivität zu verstehen. Weiterhin kann die biologische Aktivität dahingegen verändert worden sein, daß die erfindungsgemäßen Substanzen und/oder Mittel nicht mehr oder nicht mehr richtig an die Nukleinsäuresequenzen und/oder die durch sie kodierten Genprodukte binden. Unter nicht mehr oder nicht mehr richtig ist im Sinne der Erfindung zu verstehen, daß die Substanzen um mindestens 30%, bevorzugt mindestens 50%, besonders bevorzugt um mindestens 70%, ganz besonders bevorzugt um mindestens 80% oder gar nicht mehr an die veränderten Nukleinsäuren und/oder Genprodukte im Vergleich zum Ausgangsgenprodukt oder den Ausgangsnukleinsäuren binden.

Noch ein weiterer Aspekt der Erfindung betrifft deshalb eine transgene Pflanze, welche mit einer Nukleinsäuresequenz, welche für ein Genprodukt kodiert, das eine veränderte biologische Aktivität aufweist, oder mit einer Nukleinsäuresequenz codierend für eine SSP-Variante transformiert wurde. Verfahren zur Transformation sind dem Fachmann bekannt und beispielhaft weiter oben ausgeführt.

Genetisch veränderten transgene Pflanzen, die gegen die nach den erfindungsgemäßen Verfahren gefundenen Substanzen und/oder Mittel enthaltend diese Substanzen resistent sind, können auch durch Transformation gefolgt von Überexpression einer erfindungsgemäßen Nukleinsäuresequenz erzeugt werden. Ein Verfahren zur Erzeugung transgener Pflanzen, die gegen Substanzen, die nach einem erfindungsgemäßen Verfahren gefunden wurden, resistent sind, ist dadurch gekennzeichnet, daß in diesen Pflanzen Nukleinsäuren codierend für eine SSP Variante überexprimiert werden. Ein ähnliches Verfahren wird beispielhaft in Lermantova et al., Plant Physiol., 122, 2000: 75 - 83 beschrieben.

Die oben beschriebenen erfindungsgemäßen Verfahren zur Erzeugung resistenter Pflanzen ermöglichen die Entwicklung neuer Herbizide, die eine möglichst umfassende Pflanzenspezies unabhängige Wirkung aufweisen (sog. Totalherbizide), in Kombination mit der Entwicklung von gegenüber dem Totalherbizid resistenten Nutzpflanzen. Gegenüber Totalherbiziden resistente Nutzpflanzen sind bereits verschiedentlich beschrieben worden. Dabei können mehrere Prinzipien zur Erzielung einer Resistenz unterschieden werden:
a) Resistenzerzeugung in einer Pflanze über Mutationsverfahren oder gentechnische Verfahren, indem das als Zielort für das Herbizid dienende Protein deutlich überproduziert wird und indem auf Grund des großen Überschusses des als Zielort für das Herbizid dienende Protein, die von diesem Protein in der Zelle ausgeübte Funktion auch nach Applikation des Herbizides beibehalten wird.
b) Veränderung der Pflanze dahingehend, daß eine modifizierte Version des als Zielort des Herbizid fungierenden Proteins eingeführt wird und daß das neu eingeführte modifizierte Protein vom Herbizid nicht in seiner Funktion beeinträchtigt wird.
c) Veränderung der Pflanze dahingehend, daß ein neues Protein/ eine neue RNA eingeführt wird welches dadurch gekennzeichnet ist, daß die für die herbizide Wirkung der niedermolekularen Substanz verantwortliche chemische Struktur des Proteins oder der Nukleinsäure wie der RNA oder der DNA so verändert wird, daß durch die veränderte Struktur keine herbizide Wirkung mehr entfaltet werden kann, daß heißt die Interaktion des Herbizids mit dem Zielort nicht mehr erfolgen kann.
d) das die Funktion des Targets durch ein neues in die Pflanze eingebrachtes Gen ersetzt wird, und so ein sogenannter "alternativer Pathway" geschaffen wird.
e) Das die Funktion des Targets durch ein anderes in der Pflanze vorhandenes Gen bzw. dessen Genprodukt übernommen wird.

Dem Fachmann sind alternative Verfahren zur Identifizierung von den homologen Nukleinsäuren beispielsweise in anderen Pflanzen mit ähnlichen Sequenzen wie beispielsweise unter Verwendung von Transposons, bekannt. Gegenstand dieser Erfindung ist daher auch die Verwendung von alternativen Insertionsmutageneseverfahren zur Insertion von fremder Nukleinsäuren in die Nukleinsäuresequenzen SEQ ID NO:1, SEQ ID NO:3 oder SEQ ID NO:5 in von diesen Sequenzen aufgrund des genetischen Codes abgeleitete Sequenzen und/oder deren Derivate in anderen Pflanzen.

Die Herstellung der transgenen Pflanzen erfolgt mit einer der oben beschrieben Ausführungsformen der erfindungsgemäßen Expressionskassette nach ebenfalls oben beschriebenen gängigen Transformationsmethoden.

Die Wirksamkeit der Expression der transgen exprimierten SSP kann beispielsweise in vitro durch Sproßmeristemvermehrung oder durch einen Keimungstest ermittelt werden. Zudem kann eine in Art und Höhe veränderte Expression des SSP Gens und deren Auswirkung auf die Resistenz gegenüber Hemmstoffen der SSP an Testpflanzen in Gewächshausversuchen getestet werden.

Die Erfindung wird durch die nachstehenden Beispiele weiter veranschaulicht, die nicht als beschränkend aufgefaßt werden sollten.

### Allgemeine DNA-Manipulations- und Klonierungsverfahren

Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und Sequenzanalyse rekombinanter DNA wurden wie bei Sambrook et al. (1989) (Cold Spring Harbor Laboratory Press: ISBN 0-87969-309-6) und Ausubel, F.M. et al., Current Protocols in Molecular Biology, Greene Publishing Assoc. and Wiley-Interscience (1994); ISBN 0-87969-309-6 beschrieben durchgeführt.

Pflanzenmolekularbiologische Standardverfahren sowie Pflanzentransformationsverfahren sind beschrieben in Schultz et al., Plant Molecular Biology Manual, Kluwer Academic Publishers (1998), Reither et al., Methods in Arabidopsis Research, World svcientific press (1992) und Arabidopsis: A Laboratory Manual (2001), ISBN 0-87969-573-0.

Die im folgenden verwendeten Bakterienstämme (E. coli DH5a, XL-1 blue, BL21 DE(3), JM 109) wurden von Stratagene, BRL Gibco oder Invitrogen, Carlsberg, CA bezogen. Zur Klonierung wurden die Vektoren pCR-Blunt (Invitrogen) und pUC 18 der Firma Amersham Pharmacia (Freiburg), pBinAR (Höfgen und Willmitzer, Plant Science 66, 1990, 221-230), pCR und pQE-9 (Qiagen, Hilden) verwendet.

### Beispiel 1 - Klonierung von SSP kodierenden Sequenzen aus Solanaceaen

Zur Ableitung von für SSP kodierende DNA Sequenzen wurde mit Hilfe des BLAST Algorhythmus (Altschul et al. 1990, J. Mol. Biol. 215, pp. 403-410) und der Proteinsequenz der S801 aus *Arabidopsis thaliana* (Acc. Nr. AF283565) die 6-Frame Translation der EST Datenbank (Genbank) durchmustert. Dabei wurden mehrere signifikante Treffer, unter anderem aus Tomate (Lycopersicon esculentum) und Kartoffel (Solanum tuberosum), identifiziert. Die Hits der ersten Runde wurden für weitere Datenbanksuchen nach obigem Modus eingesetzt bis der gesamte kodierende Bereich einer potentiellen SSP durch überlappende Tomaten bzw. Kartoffel ESTs abgedeckt wurde. Von der so erhaltenen Sequenzinformation wurden die Primer
FB 223 5'-ATG GAT CAG CTA ACC AGTCGCC GCA C-3' (SEQ ID NO:8)
FB 224 5'-CTA AAA GAA CCA GGA CGC GGA GTC ACT-3' (SEQ ID NO:9)
abgeleitet, welche den gesamten kodierenden Bereich flankieren.

Diese Primer wurden in einer Standard-PCR-Reaktion z.B. nach T. Maniatis, E.F. Fritsch und J. Sambrook, "Molecular Cloning: A Laboratory Manual", Cold Spring Harbor Laboratory, Cold Spring Harbor, NY (1989) eingesetzt um SSP kodierende Sequenzen aus cDNA-Banken aus Tabak und Kartoffel (hergestellt nach Standardmethoden über den lambda-ZAP-Kit von Stratagene) zu isolieren. Dabei konnten zwei unterschiedliche Klone aus der Tabak cDNA Bank (SEQ ID NO:1, SEQ ID NO:3) und ein Klon aus der cDNA-Bank aus Kartoffel (SEQ ID NO:5) isoliert werden. Der Kartoffelklon wurde durch RACE PCR kompiettiert (hergestellt nach Standardmethoden über den Clontech "SmartTM RACE cDNA Amplification Kit").

### Beispiel 2 - Erzeugung des Plasmids pBinNtSSP-RNAi

Die *in vivo*-Bedeutung der Saccharose 6-Phosphatase Aktivität (SSP-Aktivität) wurde durch gezielte Suppression der SSP Genexpression in transgenen Pflanzen analysiert. Zur Erstellung eines hierfür geeigneten Konstruktes basierend auf der SEQ ID NO:1 wurde zunächst das erste Intron der GA20-Oxidase aus *Solanum tuberosum* (S*t*GA20oxIN, SEQ ID NO:7) unter Verwendung der Primer und über PCR nach Standardbedingungen (z.B. nach T. Maniatis, E.F. Fritsch und J. Sambrook, "Molecular Cloning: A Laboratory Manual", Cold Spring Harbor Laboratory, Cold Spring Harbor, NY (1989)) amplifiziert, so dass am 5'Ende die Schnittstellen Pstl/Sbf1-Xhol-Spel-Bglll sowie am 3'Ende die Schnittstellen BamHI-XbaI-Sall-PstI/SbfI angefügt wurden. Das erhaltene PCR-Fragment wurde in einen pCR-Blunt Vektor subkloniert (pCR-Blunt-GA20) und nach einem Stul-Verdau "Blunt-end" des pCR-Blunt-GA20 in einen pUC18 Vektor ligiert, der vorher durch einen EcoRI/HindIII-Verdau geöffnet und durch PFU-Polymerase gemäß Herstellerangaben? aufgefüllt wurde. Der so erhaltene Vektor pUC-RNAi wurde als Template in einer PCR nach Standardtbedingungen (z.B. nach T. Maniatis, E.F. Fritsch und J. Sambrook, "Molecular Cloning: A Laboratory Manual", Cold Spring Harbor Laboratory, Cold Spring Harbor, NY (1989)) unter Verwendung der Primer
FB228 5'-GGA TCC ATG GAT CAG CTA ACC AGT GCC -3' (SEQ ID NO: 12) und
FB229 5-GTC GAC TAC CAT TAC ACC ATA ACA CAT C -3' (SEQ ID NO: 13)
eingesetzt, wobei ein mit endständigen BamHI/Sall Restriktionsschnittstellen versehenes 660 bp Fragment der SEQ ID NO:1 (bp 1-660) amplifiziert wurde. Das amplifizierte Fragment (NtSSP2) wurde zunächst in antisense-Orientierung (a) in den BgIII/Xhol geöffneten pUC-RNAi kloniert (ergibt Vektor pUC-RNAi-aNtSSP2). Anschließend wurde das gleiche Fragment über BamHI/Sall in sense Orientierung in den Vektor pUC-RNAi-aNtSSP2 kloniert (ergibt Vektor pUC-RNAi-aNtSSP2-*St*GA20oxIN-sNtSSP2). Die hierbei entstandene Kassette wurde über Pstl aus dem Vektor pUC-RNAi-aNtSSP2-*St*GA20oxIN-sNtSSP2 in einen Sbfl geschnittenen BinAR ligiert, wodurch das Plasmid pBinNtSSP-RNAi erhalten wurde.

### Beispiel 3 - Transformation und Analyse von Tabakpflanzen

Das Konstrukt pBinNtSSP-RNAi wurde nach Deblaere et al. (Nucl. Acids. Res. 13(1984), 4777-4788) in den Agrobacterium tumefaciens Stamm C58C1:pGV2260 transformiert und unter Streptomycin/Spectinomycin-Selektion inkubiert. Zur Transformation von Tabakpflanzen der Sorte Nicotiana tabacum cv. Samsun NN mit dem Konstrukt pBinNtSSP-RNAi wurde eine in YEB-Medium (5g/l beef extract, 1g/l yeast extract, 5g/l peptone, 5g/l sucrose, pH 7,2) auf OD₆₀₀ = 0.8-1.6 verdünnte Übernachtkultur einer positiv transformierten Agrobakterienkolonie benutzt. Nach 5-10 Minuten Inkubation steriler Blattscheiben der Pflanzen (zu je ca. 1 cm²) in einer Petrischale mit der auf OD₆₀₀ = 0.8-1.6 verdünnte Übernachtkultur der Agrobakterien folgte eine 2-tägige Inkubation in Dunkelheit bei 25°C auf Murashige-Skoog Medium (nach Murashige-Skoog, Physiol. Plant. 15(1962), 473) mit 2% Saccharose (2MS-Medium) mit 0,8 % Bacto-Agar). Im Anschluß wurde die Kultivierung für/über einen Zeitraum von mehreren Wochen mit 16 Stunden Licht/8 Stunden Dunkelheit weitergeführt. Die Blattscheiben/Calli wurden wöchentlich auf frisches MS-Medium mit 500mg/l Claforan (Cefotaxime-Natrium), 50mg/l Kanamycin, 1mg/l Benzylaminopurin (BAP), 0,2mg/l Naphtylessigsäure und 1,6g/l Glukose umgesetzt. Wachsende Sprossen wurden auf MS-Medium mit 2% Saccharose, 250 mg/l Claforan und 0,8 % Bacto-Agar überführt und im Anschluß auf 2MS-Medium mit Kanamycin und Claforan selektiert. Die auf diese Weise erhaltenen transgenen Pflanzen wurden in Erde gesetzt und für 2-20 Wochen im Gewächshaus auf die Ausprägung von Phänotypen beobachtet. Dabei stellte sich heraus, dass die transgenen Pflanzen deutliche Wachstumsretardierungen, chlorotische Blätter und vereinzelt Nekrosen aufwiesen. Durch halbquantitative PCR konnte gezeigt werden; das in Pflanzen mit diesen Phänotypen in unterschiedlichem Mass die Expression der SSP unterdrückt war, wodurch der Zusammenhang zwischen Pflanzenwachstum und SSP-Expression gezeigt wurde.

20µg Gesamt-RNA von ausgewählten Linien (Linien 10, 16, 18, 31) sowie von einer nicht-transgenen Kontrollen (WT) wurde zunächst mit DNase (Böhringer Mannheim) bei 37°C für 45 min verdaut und diese anschliessend für 10 min bei 65°C inhibiert. Nach Phenol/Chloroform/Isoamylalkohol (25:24:1) Behandlung wurde die RNA mit Natriumacetat gefällt, mit 70% Ethanol gewaschen, und in 100µl DEPC-behandeltem H20 gelöst. Die cDNA Erststrangsynthese wurde in einem Ansatz mit 12,5µl DNase behandelter RNA, 5µl 5x Reaktions-Puffer, 2µl dNTPs (2,5 mM), 1µl Oligo dT Primer (50 mM, dT[30]V[G/C/A]) und 2,5µl DEPC-behandeltem H₂O nach Inkubation für 5 min bei 65°C, dann für 5 min bei 37°C schliesslich nach Zugabe von 1 µl Reverser Transkriptase (Moloney Murine Leukemia Virus Reverse Transkriptase, Rnase H Minus, M-MLV [H-], Promega) und 1µl RNAse-Inhibitor bei 37°C (60 min) durchgeführt. Nach Hitzeinaktivierung für 5 min bei 95°C wurde die cDNA als Matrize für die anschliessende PCR eingesetzt. NtSSP cDNA wurde mit dem 5' Primer
CS36 (5'-GTT AGT GTT CTC AAC TGG GAG ATC ACC-3') (SEQ ID NO:14)
   und dem 3' Primer
CS37 (5'-CCC ATT TCT TGA AAC TCA CTA ACC ATG A-3'), (SEQ ID NO:15)
   der interne Standard Actin wurde mit dem Primerpaar
D₂O₂ (5'-ATG GCA GAC GGT GAG GAT ATT CA-3') (SEQ ID NO:16)
   Und
D203 (5'-GCC TTT GCA ATC CAC ATC TGT TG-3') (SEQ ID NO:17)
amplifiziert (wie AC1 und AC2, Romeis et al. 2001, EMBO J 20: 5556). Die PCR-Ansätze (Gesamtvolumen 1 00µl) setzten sich wie folgt zusammen: 70µl H₂O, 5µl CS36 5'Primer (5µM), 5µl 3' Primer CS37 (5µM), 8µl dNTPs (2,5 mM), 10µl 10x.Reaktions-Puffer, 1µl cDNA und 5 U rTaq DNA Polymerase (Takara Shouzo, Japan). Vor Beginn der Amplifikationszyklen wurden die Ansätze für 5 min auf 95°C erhitzt. Die Polymerisierungsschritte wurden in einem automatischen T3-Thermocycler (Biometra) nach folgendem Programm durchgeführt: Denaturierung 95°C (1 min), Anlagerung der Primer bei 55°C (45 Sekunden), Polymerase-Reaktion bei 72°C (2 min). Nach 25, 30, und 45 Zyklen wurden jeweils 10µl des PCR-Ansatzes auf ein Gel aufgetragen. Das Ergebnis zeigt im nicht-gesättigten PCR-Bereich (35 Zyklen) bei Verwendung der NtSSP-spezifischen Primer nur die Amplifzierung von Produkten in den Wildtyp-Kontrollen und nicht in 3 der 4 transgenen Linien, was auf sehr effizientes "Silencing" schliessen lässt. Die PCR mit den Actin spezifischen Primern hingegen zeigt im ungesättigten Bereich bei 35 Zyklen gleichmässige DNA-Banden, was den Einssatz vergleichbarer Mengen an eingesetzter Matrize belegt.

Diese Ergebnisse auf mRNA-Ebene wurden durch Westen-Blot Experimente bestätigt. Im Rahmen dieser Experimente wurden je 50 µg von Gesamtproteinextrakten aus Blättern der transgenen Pflanzen und Wildtyp Pflanzen auf 10% SDS-Polyacrylamidgelen separiert. NtSSP wurde nach Transfer auf Nitrocellulose Membranen und Inkubation mit einem Anti-SSP Antirkörper aus Kaninchen mittels ECL-Methode (Amersham Pharmacia, Biotech, nach Herstellerangaben) detektiert. Dabei war NtSSP in den transgenen Pflanzen im Gegensatz zu Wildtyp Pflanzen nicht detektierbar. Ferner wurde die NtSSP-Aktivität in Gesamtproteinextrakten aus Blättern der transgenen Pflanzen und Wildtyp Pflanzen entsprechend Beispiel 6 bestimmt. In transgenen Pflanzen lag die Restaktivität bei 6-10% der Wildtyp Aktivität.

### Beispiel 4 - Herstellung von Konstrukten für die SSP Expression in E. coli

Zur Expression der Saccharose-6-Phosphatase aus N. tabaccum (SSP2 aus *N*. *tabac*cum) in *E*. *coli* wurde SEO ID NO: 1 über PCR unter Verwendung der Primer
FB228 5'-GGA TCC ATG GAT CAG CTA ACC AGT GCC -3' (SEQ ID NO:18)
SSPr 5'-GTC GAC CTA AAA GAA CCA GGA CGC GGA GTC ACT-3' (SEQ ID NO:19)
und der cDNA-Bank aus Nicotiana tabacum als Template amplifiziert, wobei die Primer eine BamHI bzw. Sall Erkennungsstelle in die Sequenz einführen. Das so erhaltene Fragment wurde nach der Ligation in den Vektor pCR-Blunt über BamHI und Sall ausgeschnitten und in den ebenfalls mit BamHI und Sall gespaltenen pQE-9 Vektor ligiert (Konstrukt pQE-*NtSSP2*).

### Beispiel 5 - Expression der NtSSP2 in E. coli

Die Expression des rekombinanten Proteins erfolgte nach Herstellerangaben (Qiagen, Hilden, Deutschland) in einem Kulturmaßstab von 50 ml. Nach Ernte der Zellen durch Zentrifugation wurde das Präzipitat in 1 ml 30 mM HEPES KOH (N-2-Hydroxyethylpiperazin-N'-2-ethansulfonsäure) (pH 7,5) resuspendiert und die lösliche Proteinfraktion durch Ultraschallbehandlung freigesetzt und der nach Zentrifuagion erhaltene Überstand zur Bestimmung der Enzymaktivität eingesetzt.

### Beispiel 6 - Bestimmung der Aktivität von Saccharose-6-Phosphatase (SSP)

Der Nachweis von SSP Aktivität in Proteinextrakten erfolgt über die Erfassung des vom Enzym aus Saccharose-6-phosphat freigesetzten anorganischen Phosphates nach Lunn et al. (2000, Procl. Natl. Acad. Sci. USA 97: 12914). Dazu werden Enzymextrakte in einem Reaktionsansatz enthaltend 1,25 mM Saccharose-6-phosphat und 8 mM Mg-Cl₂ in 25 mM HEPES-KOH, pH 7.0, in einem Gesamtvolumen von 300 µl bei 30°C inkubiert. Durch Zugabe von 30 µl 2M Trichloressigsäure wird die Reaktion abgestoppt. Das während der Reaktion aus S-6-P freigesetzte Orthophosphat wird unter Verwendung des Ascorbat-Ammoniummolybdat-Reagenzes (nach Ames 1966, Methods Enzymol. 8, 115) quantifiziert. Der Nachweis wurde in miniaturisierter Form, wie z.B. in 96well und 384well Mikrotiterplatten durchgeführt.

## Patentansprüche

1. Verwendung eines Polypeptides mit der biologischen Aktivität einer Saccharose-6-Phosphat Phosphatase kodiert durch eine Nukleinsäuresequenz umfassend:
a) eine Nukleinsäuresequenz mit der in SEQ ID NO:1; SEQ ID NO:3 oder SEQ ID NO:5 dargestellten Nukleinsäuresequenz; oder
b) eine Nukleinsäuresequenz, die sich aufgrund des degenerierten genetischen Codes durch Rückübersetzung aus der in SEQ ID NO:2, SEQ ID NO:4 oder SEQ ID NO:6 dargestellten Aminosäuresequenz ableiten läßt; oder
c) funktionelle Äquivalente der Nukleinsäuresequenz SEQ ID NO:1 mit einer Identität von mindestens 55% zu der SEQ ID NO:1; oder funktionelle Äquivalente der Nukleinsäuresequenz SEQ ID NO:3 mit einer Identität von mindestens 55% zu der SEQ ID NO:3; oder funktionelle Äquivalente der Nukleinsäuresequenz SEQ ID NO:5 mit einer Identität von mindestens 51% zu der SEQ ID NO:5; oder
d) eine Nukleinsäuresequenz, die sich aufgrund des degenerierten genetischen Codes durch Rückübersetzung der Aminosäuresequenz eines funktionellen Äquivalents der SEQ ID NO:2, das eine Identität mit der SEQ ID NO:2 von mindestens 55% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:4, das eine Identität mit der SEQ ID NO:4 von mindestens 54% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:6, das eine Identität mit der SEQ ID NO:6 von mindestens 54% aufweist, ableiten läßt;
als Target für Herbizide.

2. Verwendung eines Polypeptides mit der biologischen Aktivität einer Saccharose-6-Phosphat Phosphatase kodiert durch eine Nukleinsäuresequenz umfassend:
a) eine Nukleinsäuresequenz mit der in SEQ ID NO:1, SEQ ID NO:3 oder SEQ ID NO:5 dargestellten Nukleinsäuresequenz; oder
b) eine Nukleinsäuresequenz, die sich aufgrund des degenerierten genetischen Codes durch Rückübersetzung aus der in SEQ ID NO:2, SEQ ID NO:4 oder SEQ ID NO:6 dargestellten Aminosäuresequenz ableiten läßt; oder
c) funktionelle Äquivalente der Nukleinsäuresequenz SEQ ID NO:1 mit einer Identität von mindestens 55% zu der SEQ ID NO:1; oder funktionelle Äquivalente der Nukleinsäuresequenz SEQ ID NO:3 mit einer Identität von mindestens 55% zu der SEQ ID NO:3; oder funktionelle Äquivalente der Nukleinsäuresequenz SEQ ID NO:5 mit einer Identität von mindestens 51% zu der SEQ ID NO:5; oder
d) eine Nukleinsäuresequenz, die sich aufgrund des degenerierten genetischen Codes durch Rückübersetzung der Aminosäuresequenz eines funktionellen Äquivalents der SEQ ID NO:2, das eine Identität mit der SEQ ID NO:2 von mindestens 55% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:4, das eine Identität mit der SEQ ID NO:4 von mindestens 54% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:6, das eine Identität mit der SEQ ID NO:6 von mindestens 54% aufweist, ableiten läßt; in einem Verfahren zur Identifizierung von Verbindungen mit herbizider Wirkung.

3. Verfahren zur Identifizierung von Substanzen mit herbizider Wirkung umfassend die folgenden Schritte:
i. Inkontaktbringen eines Polypeptides mit der biologischen Aktivität einer Saccharose-6-Phosphat Phosphatase kodiert durch eine Nukleinsäuresequenz umfassend:
a) eine Nukleinsäuresequenz mit der in SEQ ID NO:1, SEQ ID NO:3 oder SEQ ID NO:5 dargestellten Nukleinsäuresequenz; oder
b) eine Nukleinsäuresequenz, die sich aufgrund des degenerierten genetischen Codes durch Rückübersetzung aus der in SEQ ID NO:2, SEQ ID NO:4 oder SEQ ID NO:6 dargestellten Aminosäuresequenz ableiten läßt; oder
c) funktionelle Äquivalente der Nukleinsäuresequenz SEQ ID NO:1 mit einer Identität von mindestens 55% zu der SEQ ID NO:1; oder funktionelle Äquivalente der Nukleinsäuresequenz SEQ ID NO:3 mit einer Identität von mindestens 55% zu der SEQ ID NO:3; oder funktionelle Äquivalente der Nukleinsäuresequenz SEQ ID NO:5 mit einer Identität von mindestens 51 % zu der SEQ ID NO:5; oder
d) eine Nukleinsäuresequenz, die sich aufgrund des degenerierten genetischen Codes durch Rückübersetzung der Aminosäuresequenz eines funktionellen Äquivalents der SEQ ID NO:2, das eine Identität mit der SEQ ID NO:2 von mindestens 55% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:4, das eine Identität mit der SEQ ID NO:4 von mindestens 54% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:6, das eine Identität mit der SEQ ID NO:6 von mindestens 54% aufweist, ableiten läßt;
mit einer oder mehreren Testverbindungen unter Bedingungen, die die Bindung der Testverbindung(en) an das Nukleinsäuremolekül oder an die Saccharose-6-Phosphat Phosphatase erlauben; und
ii. Nachweis, ob die Testverbindung an die Saccharose-6-Phosphat Phosphatase aus i) bindet; oder
iii. Nachweis, ob die Testverbindung die Aktivität der Saccharose-6-Phosphat Phosphatase aus i) reduziert oder blockiert; oder
iv. Nachweis, ob die Testverbindung die Transkription, Translation oder Expression der Saccharose-6-Phosphat Phosphatase aus i) reduziert oder blockiert.

4. Verfahren nach Anspruch 3, **dadurch gekennzeichnet, dass**
i. Saccharose-6-Phosphat Phosphatase kodiert durch eine Nukleinsäuresequenz umfassend
a) eine Nukleinsäuresequenz mit der in SEQ ID NO:1, SEQ 10 NO:3 oder SEQ ID NO:5 dargestellten Nukleinsäuresequenz; oder
b) eine Nukleinsäuresequenz, die sich aufgrund des degenerierten genetischen Codes durch Rückübersetzung aus der in SEQ ID NO:2, SEQ ID NO:4 oder SEQ ID NO:6 dargestellten Aminosäuresequenz ableiten läßt; oder
c) funktionelle Äquivalente der Nukleinsäuresequenz SEQ ID NO:1 mit einer Identität von mindestens 55% zu der SEQ ID NO:1; oder funktionelle Äquivalente der Nukleinsäuresequenz SEQ ID NO:3 mit einer Identität von mindestens 55% zu der SEQ ID NO:3; oder funktionelle Äquivalente der Nukleinsäuresequenz SEQ ID NO:5 mit einer Identität von mindestens 51% zu der SEQ ID NO:5; oder
d) eine Nukleinsäuresequenz, die sich aufgrund des degenerierten genetischen Codes durch Rückübersetzung der Aminosäuresequenz eines funktionellen Äquivalents der SEQ ID NO:2, das eine Identität mit der SEQ ID NO:2 von mindestens 55% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:4, das eine Identität mit der SEQ ID NO:4 von mindestens 54% aufweist, ableiten läßt; oder eine Nukleinsäuresequenz; die sich aufgrund des degenerierten genetischen Codes durch Rückübersetzung der Aminosäuresequenz eines funktionellen Äquivalents der SEQ ID NO:6, das eine Identität mit der SEQ ID NO:6 von mindestens 54% aufweist, ableiten läßt;
entweder in einem transgenen Organismus exprimiert wird oder ein Organismus, der naturgemäß Saccharose-6-Phosphat Phosphatase enthält, kultiviert wird, wobei der transgene Organismus aus Pflanzen, Bakterien, Hefen, Moose, Algen, Pilze oder eukaryontische Zelllinien besteht,
ii. die Saccharose-6-Phosphat Phosphatase aus Schritt i) im Zellaufschluss des transgenen bzw. nicht transgenen Organismus, in partiell gereinigter Form oder in zur Homogenität gereinigten Form mit einer Testverbindung in Kontakt gebracht wird; und
iii. eine Testverbindung selektiert wird, welche die Aktivität der Saccharose-6-Phosphat Phosphatase aus Schritt a) reduziert oder blockiert, wobei die Aktivität der mit der Testverbindung inkubierten Saccharose-6-Phosphat Phosphatase mit der Aktivität einer nicht mit einer Testverbindung inkubierten Saccharose-6-Phosphat Phosphatase verglichen wird.

5. Verfahren nach Anspruch 4, **dadurch gekennzeichnet, dass** zur Bestimmung der Aktivität in Schritt iii) Saccharose-6-Phosphat als Substrat eingesetzt wird und das bei der Reaktion entstehende Orthophosphat durch Ammoniummolybdat quantifiziert wird, wobei der transgene Organismus Pflanzen, Bakterien, Hefen, Moose, Algen, Pilze oder eukaryontische Zelllinien umfasst.

6. Verfahren nach Anspruch 5, **dadurch gekennzeichnet, dass** es die folgende Schritte umfasst:
i. Herstellung eines transgenen Organismus enthaltend eine Nukleinsäuresequenz kodierend für ein Polypeptid mit der biologischen Aktivität einer Saccharose-6-Phosphat Phosphatase umfassend
a) eine Nukleinsäuresequenz mit der in SEQ ID NO:1, SEQ ID NO:3 oder SEQ ID NO:5 dargestellten Nukleinsäuresequenz; oder
b) eine Nukleinsäuresequenz, die sich aufgrund des degenerierten genetischen Codes durch Rückübersetzung aus der in SEQ ID NO:2, SEQ ID NO:4 oder SEQ ID NO:6 dargestellten Aminosäuresequenz ableiten läßt; oder
c) funktionelle Äquivalente der Nukleinsäuresequenz SEQ ID NO:1 mit einer Identität von mindestens 55% zu der SEQ ID NO:1; oder funktionelle Äquivalente der Nukleinsäuresequenz SEQ ID NO:3 mit einer Identität von mindestens 55% zu der SEQ ID NO:3; oder funktionelle Äquivalente der Nukleinsäuresequenz SEQ ID NO:5 mit einer Identität von mindestens 51% zu der SEQ ID NO:5; oder
d) eine Nukleinsäuresequenz, die sich aufgrund des degenerierten genetischen Codes durch Rückübersetzung der Aminosäuresequenz eines funktionellen Äquivalents der SEQ ID NO:2, das eine Identität mit der SEQ ID NO:2 von mindestens 55% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:4, das eine Identität mit der SEQ ID NO:4 von mindestens 54% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:6, das eine Identität mit der SEQ ID NO:6 von mindestens 54% aufweist, ableiten läßt;
wobei in dem transgenen Organismus das Polypeptid mit der biologischen Aktivität einer Saccharose-6-Phosphat Phosphatase überexprimiert wird; und
ii. Aufbringen einer Testsubstanz auf den transgenen Organismus nach i) und auf einen nicht-transgenen Organismus des gleichen Genotyps; und
iii. Bestimmen des Wachstums oder der Überlebensfähigkeit des transgenen und des nicht transgenen Organismus nach der Aufbringung der Testsubstanz; und
iv. Selektion von Testsubstanzen, die ein vermindertes Wachstum oder eine eingeschränkte Überlebensfähigkeit des nicht-transgenen Organismus bewirken verglichen mit dem Wachstum des transgenen Organismus
wobei der transgene Organismus aus Pflanzen, Bakterien, Hefen, Moose, Algen, Pilze oder eukaryontische Zelllinien besteht.

7. Verfahren nach Anspruch 6, **dadurch gekennzeichnet, dass** es in einem pflanzlichen Organismus, einem Cyanobakterium oder einem Proteobakterium durchgeführt wird.

8. Verfahren zur Identifizierung von Substanzen mit wachstumsregulatorischer Wirkung, **dadurch gekennzeichnet, dass** es die folgende Schritte umfasst:
i. Herstellung einer transgenen Pflanze enthaltend eine Nukleinsäuresequenz kodierend für ein Polypeptid mit der biologischen Aktivität einer Saccharose-6-Phosphat Phosphatase umfassend
a) eine Nukleinsäuresequenz mit der in SEQ ID NO:1, SEQ ID NO:3 oder SEQ ID NO:5 dargestellten Nukleinsäuresequenz; oder
b) eine Nukleinsäuresequenz, die sich aufgrund des degenerierten genetischen Codes durch Rückübersetzung aus der in SEQ ID NO:2, SEQ ID NO:4 oder SEQ ID NO:6 dargestellten Aminosäuresequenz ableiten läßt; oder
b) funktionelle Äquivalente der Nukleinsäuresequenz SEQ ID NO:1 mit einer Identität von mindestens 55% zu der SEQ ID NO:1; oder funktionelle Äquivalente der Nukleinsäuresequenz SEQ ID NO:3 mit einer Identität von mindestens 55% zu der SEQ ID NO:3; oder funktionelle Äquivalente der Nukleinsäuresequenz SEQ ID NO:5 mit einer Identität von mindestens 51% zu der SEQ ID NO:5; oder
c) eine Nukleinsäuresequenz, die sich aufgrund des degenerierten genetischen Codes durch Rückübersetzung der Aminosäuresequenz eines funktionellen Äquivalents der SEQ ID NO:2, das eine ldentität mit der SEQ ID NO:2 von mindestens 55% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:4, das eine Identität mit der SEQ ID NO:4 von mindestens 54% aufweist, ableiten läßt; oder eine Nukleinsäuresequenz, die sich aufgrund des degenerierten genetischen Codes durch Rückübersetzung der Aminosäuresequenz eines funktionellen Äquivalents der SEQ ID NO:6, das eine Identität mit der SEQ ID NO:6 von mindestens 54% aufweist, ableiten läßt;
wobei in der transgenen Pflanze das Polypeptid mit der biologischen Aktivität einer Saccharose-6-Phosphat Phosphatase überexprimiert wird;
ii. Aufbringen einer Testsubstanz auf die transgene Pflanze nach i) und auf eine nicht-transgene Pflanze der gleichen Sorte;
iii. Bestimmen des Wachstums oder der Überlebensfähigkeit der transgenen und der nicht transgenen Pflanze nach dem Aufbringen der Testsubstanz; und
iv. Selektion von Testsubstanzen, die ein verändertes Wachstum der nicht-transgenen Pflanze bewirken verglichen mit dem Wachstum der transgenen Pflanze.

9. Verfahren nach einem der Ansprüche 3 bis 8, **dadurch gekennzeichnet, dass** die Identifizierung der Substanzen in einem High-Throughput-Screening durchgeführt wird.

10. Verfahren zur Erzeugung von Nukleinsäuresequenzen, welche für ein Polypeptid mit der biologischen Aktivität einer Saccharose-6-Phosphat Phosphatase kodieren, welches durch Verbindungen mit herbizider Wirkung identifiziert über eines der Verfahren nach den Ansprüchen 3 bis 7 und 9 nicht inhibiert wird; und durch ein funktionelles Äquivalent der Nukleinsäuresequenz SEQ ID N0:1 mit einer Identität von mindestens 55% zu der SEQ ID NO:1; oder ein funktionelles Äquivalente der Nukleinsäuresequenz SEQ ID NO:3 mit einer Identität von mindestens 55% zu der SEQ ID NO:3; oder funktionelle Äquivalente der Nukleinsäuresequenz SEQ ID NO:5 mit einer Identität von mindestens 51% zu der SEQ ID NO:5 umfasst werden;
**dadurch gekennzeichnet, daß** es folgende Prozessschritte umfaßt:
a) Expression des von einer der oben genannten Nukleinsäuresequenzen kodierten Proteins in einem heterologen System oder in einem zellfreien System;
b) Randomisierte oder gerichtete Mutagenese des Proteins durch Modifikation der Nukleinsäure;
c) Messung der Interaktion des veränderten Genprodukts mit dem Herbizid;
d) Identifizierung von Derivaten des Proteins die eine geringere Interaktion aufweisen;
e) Testung der biologischen Aktivität des Proteins nach Applikation des Herbizides; und
f) Auswahl der Nukleinsäuresequenzen, die eine veränderte biologische Aktivität gegenüber dem Herbizid aufweisen.

11. Verfahren nach Anspruch 10, **dadurch gekennzeichnet, daß** die gemäß Anspruch 10 f) ausgewählten Sequenzen in einen nicht menschlichen Organismus eingebracht werden.

## Claims

1. The use of a polypeptide with the biological activity of a sucrose-6-phosphate phosphatase encoded by a nucleic acid sequence comprising:
a) a nucleic acid sequence with the nucleic acid sequence shown in SEQ ID NO:1, SEQ ID NO:3 or SEQ ID NO:5; or
b) a nucleic acid sequence which, on the basis of the degeneracy of the genetic code, can be derived from the amino acid sequence shown in SEQ ID NO:2, SEQ ID NO:4 or SEQ ID NO:6 by back translation; or
c) functional equivalents of the nucleic acid sequence SEQ ID NO:1 with at least 55% identity with SEQ ID NO:1; or functional equivalents of the nucleic acid sequence SEQ ID NO:3 with at least 55% identity with SEQ ID NO:3; or functional equivalents of the nucleic acid sequence SEQ ID NO:5 with at least 51% identity with SEQ ID NO:5; or
d) a nucleic acid sequence which, on the basis of the degeneracy of the genetic code, can be derived from the amino acid sequence of a functional equivalent of SEQ ID NO:2 with at least 55% identity with SEQ ID NO:2 by back translation; or a nucleic acid sequence which, on the basis of the degeneracy of the genetic code, can be derived from the amino acid sequence of a functional equivalent of SEQ ID NO:4 with at least 54% identity with SEQ ID NO:4 by back translation; or a nucleic acid sequence which, on the basis of the degeneracy of the genetic code, can be derived from the amino acid sequence of a functional equivalent of SEQ ID NO:6 with at least 54% identity with SEQ ID NO:6 by back translation;
as target for herbicides.

2. The use of a polypeptide with the biological activity of a sucrose-6-phosphate phosphatase encoded by a nucleic acid sequence comprising:
a) a nucleic acid sequence with the nucleic acid sequence shown in SEQ ID NO:1, SEQ ID NO:3 or SEQ ID NO:5; or
b) a nucleic acid sequence which, on the basis of the degeneracy of the genetic code, can be derived from the amino acid sequence shown in SEQ ID NO:2, SEQ ID NO:4 or SEQ ID NO:6 by back translation; or
c) functional equivalents of the nucleic acid sequence SEQ ID NO:1 with at least 55% identity with SEQ ID NO:1; or functional equivalents of the nucleic acid sequence SEQ ID NO:3 with at least 55% identity with SEQ ID NO:3; or functional equivalents of the nucleic acid sequence SEQ ID NO:5 with at least 51% identity with SEQ ID NO:5; or
d) a nucleic acid sequence which, on the basis of the degeneracy of the genetic code, can be derived from the amino acid sequence of a functional equivalent of SEQ ID NO:2 with at least 55% identity with SEQ ID NO:2 by back translation; or a nucleic acid sequence which, on the basis of the degeneracy of the genetic code, can be derived from the amino acid sequence of a functional equivalent of SEQ ID NO:4 with at least 54% identity with SEQ ID NO:4 by back translation; or a nucleic acid sequence which, on the basis of the degeneracy of the genetic code, can be derived from the amino acid sequence of a functional equivalent of SEQ ID NO:6 with at least 54% identity with SEQ ID NO:6 by back translation;
in a method identifying herbicidally active compounds.

3. A method of identifying herbicidally active substances, comprising the following steps:
i) bringing a polypeptide with the biological activity of a sucrose-6-phosphate phosphatase encoded by a nucleic acid sequence comprising
a) a nucleic acid sequence with the nucleic acid sequence shown in SEQ ID NO:1, SEQ ID NO:3 or SEQ ID NO:5; or
b) a nucleic acid sequence which, on the basis of the degeneracy of the genetic code, can be derived from the amino acid sequence shown in SEQ ID NO:2, SEQ ID NO:4 or SEQ ID NO:6 by back translation; or
c) functional equivalents of the nucleic acid sequence SEQ ID NO:1 with at least 55% identity with SEQ ID NO:1; or functional equivalents of the nucleic acid sequence SEQ ID NO:3 with at least 55% identity with SEQ ID NO:3; or functional equivalents of the nucleic acid sequence SEQ ID NO:5 with at least 51% identity with SEQ ID NO:5; or
d) a nucleic acid sequence which, on the basis of the degeneracy of the genetic code, can be derived from the amino acid sequence of a functional equivalent of SEQ ID NO:2 with at least 55% identity with SEQ ID NO:2 by back translation; or a nucleic acid sequence which, on the basis of the degeneracy of the genetic code, can be derived from the amino acid sequence of a functional equivalent of SEQ ID NO:4 with at least 54% identity with SEQ ID NO:4 by back translation; or a nucleic acid sequence which, on the basis of the degeneracy of the genetic code, can be derived from the amino acid sequence of a functional equivalent of SEQ ID NO:6 with at least 54% identity with SEQ ID NO:6 by back translation; into contact with one or more test compounds under conditions which permit the test compound(s) to bind to the nucleic acid molecule or to sucrose-6-phosphate phosphatase; and
ii. detecting whether the test compound binds to the sucrose-6-phosphate phosphatase of i); or
iii.detecting whether the test compound reduces or blocks the activity of the sucrose-6-phosphate phosphatase of i); or
iv. detecting whether the test compound reduces or blocks the transcription, translation or expression of the sucrose-6-phosphate phosphatase of i).

4. The method as claimed in claim 3, which comprises
i. either expressing, in a transgenic organism, sucrose-6-phosphate phosphatase encoded by a nucleic acid sequence comprising
a) a nucleic acid sequence with the nucleic acid sequence shown in SEQ ID NO:1, SEQ ID NO:3 or SEQ ID NO:5; or
b) a nucleic acid sequence which, on the basis of the degeneracy of the genetic code, can be derived from the amino acid sequence shown in SEQ ID NO:2, SEQ ID NO:4 or SEQ ID NO:6 by back translation; or
c) functional equivalents of the nucleic acid sequence SEQ ID NO:1 with at least 55% identity with SEQ ID NO:1; or functional equivalents of the nucleic acid sequence SEQ ID NO:3 with at least 55% identity with SEQ ID NO:3; or functional equivalents of the nucleic acid sequence SEQ ID NO:5 with at least 51% identity with SEQ ID NO:5; or
d) a nucleic acid sequence which, on the basis of the degeneracy of the genetic code, can be derived from the amino acid sequence of a functional equivalent of SEQ ID NO:2 with at least 55% identity with SEQ ID NO:2 by back translation; or a nucleic acid sequence which, on the basis of the degeneracy of the genetic code, can be derived from the amino acid sequence of a functional equivalent of SEQ ID NO:4 with at least 54% identity with SEQ ID NO:4 by back translation; or a nucleic acid sequence which, on the basis of the degeneracy of the genetic code, can be derived from the amino acid sequence of a functional equivalent of SEQ ID NO:6 with at least 54% identity with SEQ ID NO:6 by back translation;
or culturing an organism which naturally comprises sucrose-6-phosphate phosphatase, the transgenic organism being composed of plants, bacteria, yeasts, mosses, algae, fungi or eukaryotic cell lines,
ii. bringing the sucrose-6-phosphate phosphatase of step i) in the cell digest of the transgenic or nontransgenic organism, in partially or homogeneously purified form, into contact with a test compound; and
iii.selecting a test compound which reduces or blocks the activity of the sucrose-6-phosphate phosphatase of step a), where the activity of the sucrose-6-phosphate phosphatase incubated with the test compound is compared with the activity of a sucrose-6-phosphate phosphatase which has not been incubated with a test compound.

5. The method as claimed in claim 4, wherein, in step iii), the activity is determined by employing sucrose-6-phosphate as substrate and the orthophosphate which is formed in the reaction is determined quantitatively by means of ammonium molybdate, the transgenic organism comprising plants, bacteria, yeasts, mosses, algae, fungi or eukaryotic cell lines.

6. The method as claimed in claim 5, which comprises the following steps:
i. generation of a transgenic organism comprising a nucleic acid sequence encoding a polypeptide with the biological activity of a sucrose-6-phosphate phosphatase comprising
a) a nucleic acid sequence with the nucleic acid sequence shown in SEQ ID NO:1, SEQ ID NO:3 or SEQ ID NO:5; or
b) a nucleic acid sequence which, on the basis of the degeneracy of the genetic code, can be derived from the amino acid sequence shown in SEQ ID NO:2, SEQ ID NO:4 or SEQ ID NO:6 by back translation; or
c) functional equivalents of the nucleic acid sequence SEQ ID NO:1 with at least 55% identity with SEQ ID NO:1; or functional equivalents of the nucleic acid sequence SEQ ID NO:3 with at least 55% identity with SEQ ID NO:3; or functional equivalents of the nucleic acid sequence SEQ ID NO:5 with at least 51% identity with SEQ ID NO:5; or
d) a nucleic acid sequence which, on the basis of the degeneracy of the genetic code, can be derived from the amino acid sequence of a functional equivalent of SEQ ID NO:2 with at least 55% identity with SEQ ID NO:2 by back translation; or a nucleic acid sequence which, on the basis of the degeneracy of the genetic code, can be derived from the amino acid sequence of a functional equivalent of SEQ ID NO:4 with at least 54% identity with SEQ ID NO:4 by back translation; or a nucleic acid sequence which, on the basis of the degeneracy of the genetic code, can be derived from the amino acid sequence of a functional equivalent of SEQ ID NO:6 with at least 54% identity with SEQ ID NO:6 by back translation;
where the polypeptide with the biological activity of a sucrose-6-phosphate phosphatase is overexpressed in the transgenic organism; and
ii. applying a test substance to the transgenic organism of i) and to a nontransgenic organism of the same genotype; and
iii.determining the growth or the viability of the transgenic and the nontransgenic organisms after application of the test substance; and
iv. selection of test substances which bring about reduced growth or reduced viability of the nontransgenic organism in comparison with the growth of the transgenic organism;
the transgenic organism being composed of plants, bacteria, yeasts, mosses, algae, fungi or eukaryotic cell lines.

7. The method as claimed in claim 6, which is carried out in a plant organism, a cyanobacterium or a proteobacterium.

8. A method for identifying substances with a growth-regulatory action, which comprises the following steps:
i. generation of a transgenic plant comprising a nucleic acid sequence encoding a polypeptide with the biological activity of a sucrose-6-phosphate phosphatase comprising
a) a nucleic acid sequence with the nucleic acid sequence shown in SEQ ID NO:1, SEQ ID NO:3 or SEQ ID NO:5; or
b) a nucleic acid sequence which, on the basis of the degeneracy of the genetic code, can be derived from the amino acid sequence shown in SEQ ID NO:2, SEQ ID NO:4 or SEQ ID NO:6 by back translation; or
b) functional equivalents of the nucleic acid sequence SEQ ID NO:1 with at least 55% identity with SEQ ID NO:1; or functional equivalents of the nucleic acid sequence SEQ ID NO:3 with at least 55% identity with SEQ ID NO:3; or functional equivalents of the nucleic acid sequence SEQ ID NO:5 with at least 51% identity with SEQ ID NO:5; or
c) a nucleic acid sequence which, on the basis of the degeneracy of the genetic code, can be derived from the amino acid sequence of a functional equivalent of SEQ ID NO:2 with at least 55% identity with SEQ ID NO:2 by back translation; or a nucleic acid sequence which, on the basis of the degeneracy of the genetic code, can be derived from the amino acid sequence of a functional equivalent of SEQ ID NO:4 with at least 54% identity with SEQ ID NO:4 by back translation; or a nucleic acid sequence which, on the basis of the degeneracy of the genetic code, can be derived from the amino acid sequence of a functional equivalent of SEQ ID NO:6 with at least 54% identity with SEQ ID NO:6 by back translation;
where the polypeptide with the biological activity of a sucrose-6-phosphate phosphatase is overexpressed in the transgenic plant;
ii. applying a test substance to the transgenic plant of i) and to a nontransgenic plant of the same genotype;
iii.determining the growth or the viability of the transgenic and the nontransgenic plants after application of the test substance; and
iv. selection of test substances which bring about modified growth of the nontransgenic plant in comparison with the growth of the transgenic plant.

9. The method as claimed in any of claims 3 to 8, wherein the substances are identified in a high-throughput screening.

10. A method for generating nucleic acid sequences which encode a polypeptide with the biological activity of a sucrose-6-phosphate phosphatase, which polypeptide is not inhibited by herbicidally active compounds identified via one of the methods as claimed in claims 3 to 7 and 9; and which are comprised by a functional equivalent of the nucleic acid sequence SEQ ID NO:1 with at least 55% identity with SEQ ID NO:1; or a functional equivalent of the nucleic acid sequence SEQ ID NO:3 with at least 55% identity with SEQ ID NO:3; or a functional equivalent of the nucleic acid sequence SEQ ID NO:5 with at least 51% identity with SEQ ID NO:5;
which comprises the following process steps:
a) expressing the protein encoded by one of the abovementioned nucleic acid sequences in a heterologous system or a cell-free system;
b) random or site-directed mutagenesis of the protein by modification of the nucleic acid;
c) measuring the interaction of the modified gene product with the herbicide;
d) identifying derivatives of the protein which show less interaction;
e) assaying the biological activity of the protein after application of the herbicide;
f) selecting the nucleic acid sequences which have a modified biological activity against the herbicide.

11. The method as claimed in claim 10, wherein the sequences selected in accordance with claim 10 f) are introduced into a non-human organism.

## Revendications

1. Utilisation d'un polypeptide ayant l'activité biologique d'une saccharose-6-phosphate phosphatase, codé par une séquence d'acide nucléique comprenant :
a) une séquence d'acide nucléique ayant la séquence d'acide nucléique représentée dans SEQ ID n° 1, SEQ ID n° 3 ou SEQ ID n° 5 ; ou
b) une séquence d'acide nucléique qui, en raison de la dégénérescence du code génétique, peut dériver par rétrotraduction de la séquence d'aminoacides représentée dans SEQ ID n° 2, SEQ ID n° 4 ou SEQ ID n° 6 ; ou
c) des équivalents fonctionnels de la séquence d'acide nucléique SEQ ID n° 1 ayant une identité de séquence d'au moins 55 % avec la séquence SEQ ID n° 1 ; ou des équivalents fonctionnels de la séquence d'acide nucléique SEQ ID n° 3 ayant une identité de séquence d'au moins 55 % avec la séquence SEQ ID n° 3 ; ou des équivalents fonctionnels de la séquence d'acide nucléique SEQ ID n° 5 ayant une identité de séquence d'au moins 51 % avec la séquence SEQ ID n° 5 ; ou
d) une séquence d'acide nucléique qui, en raison de la dégénérescence du code génétique, peut dériver par rétrotraduction de la séquence d'aminoacides d'un équivalent fonctionnel de la séquence SEQ ID n° 2, qui présente une identité de séquence d'au moins 55 % avec la séquence SEQ ID n° 2 ; ou une séquence d'acide nucléique qui, en raison de la dégénérescence du code génétique, peut dériver par rétrotraduction de la séquence d'aminoacides d'un équivalent fonctionnel de la séquence SEQ ID n° 4, qui présente une identité de séquence d'au moins 54 % avec la séquence SEQ ID n° 4 ; ou une séquence d'acide nucléique qui, en raison de la dégénérescence du code génétique, peut dériver par rétrotraduction de la séquence d'aminoacides d'un équivalent fonctionnel de la séquence SEQ ID n° 6, qui présente une identité de séquence d'au moins 54 % avec la séquence SEQ ID n° 6 ;
en tant que cible pour des herbicides.

2. Utilisation d'un polypeptide ayant l'activité biologique d'une saccharose-6-phosphate phosphatase, codé par une séquence d'acide nucléique comprenant :
a) une séquence d'acide nucléique ayant la séquence d'acide nucléique représentée dans SEQ ID n° 1, SEQ ID n° 3 ou SEQ ID n° 5 ; ou
b) une séquence d'acide nucléique qui, en raison de la dégénérescence du code génétique, peut dériver par rétrotraduction de la séquence d'aminoacides représentée dans SEQ ID n° 2, SEQ ID n° 4 ou SEQ ID n° 6 ; ou
c) des équivalents fonctionnels de la séquence d'acide nucléique SEQ ID n° 1 ayant une identité de séquence d'au moins 55 % avec la séquence SEQ ID n° 1 ; ou des équivalents fonctionnels de la séquence d'acide nucléique SEQ ID n° 3 ayant une identité de séquence d'au moins 55 % avec la séquence SEQ ID n° 3 ; ou des équivalents fonctionnels de la séquence d'acide nucléique SEQ ID n° 5 ayant une identité de séquence d'au moins 51 % avec la séquence SEQ ID n° 5 ; ou
d) une séquence d'acide nucléique qui, en raison de la dégénérescence du code génétique, peut dériver par rétrotraduction de la séquence d'aminoacides d'un équivalent fonctionnel de la séquence SEQ ID n° 2, qui présente une identité de séquence d'au moins 55 % avec la séquence SEQ ID n° 2; ou une séquence d'acide nucléique qui, en raison de la dégénérescence du code génétique, peut dériver par rétrotraduction de la séquence d'aminoacides d'un équivalent fonctionnel de la séquence SEQ ID n° 4, qui présente une identité de séquence d'au moins 54 % avec la séquence SEQ ID n° 4 ; ou une séquence d'acide nucléique qui, en raison de la dégénérescence du code génétique, peut dériver par rétrotraduction de la séquence d'aminoacides d'un équivalent fonctionnel de la séquence SEQ ID n° 6, qui présente une identité de séquence d'au moins 54 % avec la séquence SEQ ID n° 6 ;
dans un procédé pour l'identification de composés à effet herbicide.

3. Procédé pour l'identification de substances à effet herbicide, comprenant les étapes suivantes :
i. mise en contact d'un polypeptide ayant l'activité biologique d'une saccharose-6-phosphate phosphatase, comprenant :
a) une séquence d'acide nucléique ayant la séquence d'acide nucléique représentée dans SEQ ID n° 1, SEQ ID n° 3 ou SEQ ID n° 5 ; ou
b) une séquence d'acide nucléique qui, en raison de la dégénérescence du code génétique, peut dériver par rétrotraduction de la séquence d'aminoacides représentée dans SEQ ID n° 2, SEQ ID n° 4 ou SEQ ID n° 6 ; ou
c) des équivalents fonctionnels de la séquence d'acide nucléique SEQ ID n° 1 ayant une identité de séquence d'au moins 55 % avec la séquence SEQ ID n° 1 ; ou des équivalents fonctionnels de la séquence d'acide nucléique SEQ ID n° 3 ayant une identité de séquence d'au moins 55 % avec la séquence SEQ ID n° 3 ; ou des équivalents fonctionnels de la séquence d'acide nucléique SEQ ID n° 5 ayant une identité de séquence d'au moins 51 % avec la séquence SEQ ID n° 5 ; ou
d) une séquence d'acide nucléique qui, en raison de la dégénérescence du code génétique, peut dériver par rétrotraduction de la séquence d'aminoacides d'un équivalent fonctionnel de la séquence SEQ ID n° 2, qui présente une identité de séquence d'au moins 55 % avec la séquence SEQ ID n° 2 ; ou une séquence d'acide nucléique qui, en raison de la dégénérescence du code génétique, peut dériver par rétrotraduction de la séquence d'aminoacides d'un équivalent fonctionnel de la séquence SEQ ID n° 4, qui présente une identité de séquence d'au moins 54 % avec la séquence SEQ ID n° 4 ; ou une séquence d'acide nucléique qui, en raison de la dégénérescence du code génétique, peut dériver par rétrotraduction de la séquence d'aminoacides d'un équivalent fonctionnel de la séquence SEQ ID n° 6, qui présente une identité de séquence d'au moins 54 % avec la séquence SEQ ID n° 6 ;
avec un ou plusieurs composés d'essai, dans des conditions permettant la liaison du/des composé(s) d'essai à la molécule d'acide nucléique ou à la saccharose-6-phosphate phosphatase ; et
ii. le fait de détecter si le composé d'essai se lie à la saccharose-6-phosphate phosphatase provenant de i) ; ou
iii. le fait de détecter si le composé d'essai réduit ou bloque l'activité de la saccharose-6-phosphate phosphatase provenant de i) ; ou
iv. le fait de détecter si le composé d'essai réduit ou bloque la transcription, la traduction ou l'expression de la saccharose-6-phosphate phosphatase provenant de i).

4. Procédé selon la revendication 3, **caractérisé en ce que**
i. soit de la saccharose-6-phosphate phosphatase codée par une séquence d'acide nucléique comprenant
a) une séquence d'acide nucléique ayant la séquence d'acide nucléique représentée dans SEQ ID n° 1, SEQ ID n° 3 ou SEQ ID n° 5 ; ou
b) une séquence d'acide nucléique qui, en raison de la dégénérescence du code génétique, peut dériver par rétrotraduction de la séquence d'aminoacides représentée dans SEQ ID n° 2, SEQ ID n° 4 ou SEQ ID n° 6 ; ou
c) des équivalents fonctionnels de la séquence d'acide nucléique SEQ ID n° 1 ayant une identité de séquence d'au moins 55 % avec la séquence SEQ ID n° 1 ; ou des équivalents fonctionnels de la séquence d'acide nucléique SEQ ID n° 3 ayant une identité de séquence d'au moins 55 % avec la séquence SEQ ID n° 3 ; ou des équivalents fonctionnels de la séquence d'acide nucléique SEQ ID n° 5 ayant une identité de séquence d'au moins 51 % avec la séquence SEQ ID n° 5 ; ou
d) une séquence d'acide nucléique qui, en raison de la dégénérescence du code génétique, peut dériver par rétrotraduction de la séquence d'aminoacides d'un équivalent fonctionnel de la séquence SEQ ID n° 2, qui présente une identité de séquence d'au moins 55 % avec la séquence SEQ ID n° 2 ; ou une séquence d'acide nucléique qui, en raison de la dégénérescence du code génétique, peut dériver par rétrotraduction de la séquence d'aminoacides d'un équivalent fonctionnel de la séquence SEQ ID n° 4, qui présente une identité de séquence d'au moins 54 % avec la séquence SEQ ID n° 4 ; ou une séquence d'acide nucléique qui, en raison de la dégénérescence du code génétique, peut dériver par rétrotraduction de la séquence d'aminoacides d'un équivalent fonctionnel de la séquence SEQ ID n° 6, qui présente une identité de séquence d'au moins 54 % avec la séquence SEQ ID n° 6 ;
est exprimée dans un organisme transgénique, soit on cultive un organisme qui conformément à la nature contient de la saccharose-6-phosphate phosphatase, l'organisme transgénique consistant en des plantes, des bactéries, des levures, des mousses, des algues, des champignons ou des lignées de cellules eucaryotes ;
ii. on met en contact avec un composé d'essai la saccharose-6-phosphate phosphatase provenant de l'étape i), dans le lysat cellulaire de l'organisme transgénique ou non transgénique, sous forme partiellement purifiée ou sous forme purifiée jusqu'à l'homogénéité ; et
iii. on sélectionne un composé d'essai qui réduit ou bloque l'activité de la saccharose-6-phosphate phosphatase provenant de l'étape a), en comparant l'activité de la saccharose-6-phosphate phosphatase, mise à incuber avec le composé d'essai, avec l'activité d'une saccharose-6-phosphate phosphatase non mise à incuber avec un composé d'essai.

5. Procédé selon la revendication 4, **caractérisé en ce que**, pour la détermination de l'activité dans l'étape iii), on utilise comme substrat du saccharose-6-phosphate et on détermine quantitativement au moyen de molybdate d'ammonium l'orthophosphate formé lors de la réaction, l'organisme transgénique consistant en des plantes, des bactéries, des levures, des mousses, des algues, des champignons ou des lignées de cellules eucaryotes.

6. Procédé selon la revendication 5, **caractérisé en ce qu'**il comprend les étapes suivantes :
i. production d'un organisme transgénique contenant une séquence d'acide nucléique codant pour un polypeptide ayant l'activité biologique d'une saccharose-6-phosphate phosphatase, comprenant
a) une séquence d'acide nucléique ayant la séquence d'acide nucléique représentée dans SEQ ID n° 1, SEQ ID n° 3 ou SEQ ID n° 5 ; ou
b) une séquence d'acide nucléique qui, en raison de la dégénérescence du code génétique, peut dériver par rétrotraduction de la séquence d'aminoacides représentée dans SEQ ID n° 2, SEQ ID n° 4 ou SEQ ID n° 6 ; ou
c) des équivalents fonctionnels de la séquence d'acide nucléique SEQ ID n° 1 ayant une identité de séquence d'au moins 55 % avec la séquence SEQ ID n° 1 ; ou des équivalents fonctionnels de la séquence d'acide nucléique SEQ ID n° 3 ayant une identité de séquence d'au moins 55 % avec la séquence SEQ ID n° 3 ; ou des équivalents fonctionnels de la séquence d'acide nucléique SEQ ID n° 5 ayant une identité de séquence d'au moins 51 % avec la séquence SEQ ID n° 5 ; ou
d) une séquence d'acide nucléique qui, en raison de la dégénérescence du code génétique, peut dériver par rétrotraduction de la séquence d'aminoacides d'un équivalent fonctionnel de la séquence SEQ ID n° 2, qui présente une identité de séquence d'au moins 55 % avec la séquence SEQ ID n° 2 ; ou une séquence d'acide nucléique qui, en raison de la dégénérescence du code génétique, peut dériver par rétrotraduction de la séquence d'aminoacides d'un équivalent fonctionnel de la séquence SEQ ID n° 4, qui présente une identité de séquence d'au moins 54 % avec la séquence SEQ ID n° 4 ; ou une séquence d'acide nucléique qui, en raison de la dégénérescence du code génétique, peut dériver par rétrotraduction de la séquence d'aminoacides d'un équivalent fonctionnel de la séquence SEQ ID n° 6, qui présente une identité de séquence d'au moins 54 % avec la séquence SEQ ID n° 6 ;
le polypeptide ayant l'activité biologique d'une saccharose-6-phosphate phosphatase étant surexprimé dans l'organisme transgénique ; et
ii. application d'une substance d'essai sur l'organisme transgénique selon i) et sur un organisme non transgénique du même génotype ; et
iii. détermination de la croissance ou de la capacité de survie de l'organisme transgénique et de l'organisme non transgénique après l'application de la substance d'essai; et
iv. sélection de substances d'essai qui provoquent une croissance diminuée ou une capacité réduite de survie de l'organisme non transgénique par comparaison avec la croissance de l'organisme transgénique,
l'organisme transgénique consistant en des plantes, des bactéries, des levures, des mousses, des algues, des champignons ou des lignées de cellules eucaryotes.

7. Procédé selon la revendication 6, **caractérisé en ce qu'**il est effectué chez un organisme végétal, une cyanobactérie ou une protéobactérie.

8. Procédé pour l'identification de substances à effet régulateur de croissance, **caractérisé en ce qu'**il comprend les étapes suivantes :
i. production d'une plante transgénique contenant une séquence d'acide nucléique codant pour un polypeptide ayant l'activité biologique d'une saccharose-6-phosphate phosphatase, comprenant
a) une séquence d'acide nucléique ayant la séquence d'acide nucléique représentée dans SEQ ID n° 1, SEQ ID n° 3 ou SEQ ID n° 5 ; ou
b) une séquence d'acide nucléique qui, en raison de la dégénérescence du code génétique, peut dériver par rétrotraduction de la séquence d'aminoacides représentée dans SEQ ID n° 2, SEQ ID n° 4 ou SEQ ID n° 6 ; ou
b) des équivalents fonctionnels de la séquence d'acide nucléique SEQ ID n° 1 ayant une identité de séquence d'au moins 55 % avec la séquence SEQ ID n° 1 ; ou des équivalents fonctionnels de la séquence d'acide nucléique SEQ ID n° 3 ayant une identité de séquence d'au moins 55 % avec la séquence SEQ ID n° 3 ; ou des équivalents fonctionnels de la séquence d'acide nucléique SEQ ID n° 5 ayant une identité de séquence d'au moins 51 % avec la séquence SEQ ID n° 5 ; ou
c) une séquence d'acide nucléique qui, en raison de la dégénérescence du code génétique, peut dériver par rétrotraduction de la séquence d'aminoacides d'un équivalent fonctionnel de la séquence SEQ ID n° 2, qui présente une identité de séquence d'au moins 55 % avec la séquence SEQ ID n° 2 ; ou une séquence d'acide nucléique qui, en raison de la dégénérescence du code génétique, peut dériver par rétrotraduction de la séquence d'aminoacides d'un équivalent fonctionnel de la séquence SEQ ID n° 4, qui présente une identité de séquence d'au moins 54 % avec la séquence SEQ ID n° 4 ; ou une séquence d'acide nucléique qui, en raison de la dégénérescence du code génétique, peut dériver par rétrotraduction de la séquence d'aminoacides d'un équivalent fonctionnel de la séquence SEQ ID n° 6, qui présente une identité de séquence d'au moins 54 % avec la séquence SEQ ID n° 6 ;
le polypeptide ayant l'activité biologique d'une saccharose-6-phosphate phosphatase étant surexprimé dans la plante transgénique ;
ii. application d'une substance d'essai sur la plante transgénique selon i) et sur une plante non transgénique de la même variété ;
iii. détermination de la croissance ou de la capacité de survie de la plante transgénique et de la plante non transgénique après l'application de la substance d'essai ; et
iv. sélection de substances d'essai qui provoquent une croissance modifiée de la plante non transgénique par comparaison avec la croissance de la plante transgénique,

9. Procédé selon l'une quelconque des revendications 3 à 8, **caractérisé en ce que** l'identification des substances est effectuée dans un criblage à haut rendement *(High-Throughput-Screening).*

10. Procédé pour la production de séquences d'acide nucléique qui codent pour un polypeptide ayant l'activité biologique d'une saccharose-6-phosphate phosphatase, qui n'est pas inhibé par des composés à action herbicide, identifiés par l'un des procédés selon les revendications 3 à 7 et 9 ; et sont englobées par un équivalent fonctionnel de la séquence d'acide nucléique SEQ ID n° 1, ayant une identité de séquence d'au moins 55 % avec la séquence SEQ ID n° 1 ; ou un équivalent fonctionnel de la séquence d'acide nucléique SEQ ID n° 3, ayant une identité de séquence d'au moins 55 % avec la séquence SEQ ID n° 3 ; ou des équivalents fonctionnels de la séquence d'acide nucléique SEQ ID n° 5, ayant une identité de séquence d'au moins 51 % avec la séquence SEQ ID n° 5 ;
**caractérisé en ce qu'**il comprend les étapes suivantes de procédé :
a) expression de la protéine codée par l'une des séquences d'acide nucléique mentionnées plus haut, dans un système hétérologue ou dans un système acellulaire ;
b) mutagenèse dirigée ou aléatoire de la protéine par modification de l'acide nucléique ;
c) mesure de l'interaction du produit génique modifié avec l'herbicide ;
d) identification de dérivés de la protéine qui présentent une interaction plus faible ;
e) test de l'activité biologique de la protéine après application de l'herbicide ;
f) choix des séquences d'acide nucléique qui présentent une activité biologique modifiée vis-à-vis de l'herbicide.

11. Procédé selon la revendication 10, **caractérisé en ce que** les séquences choisies selon la revendication 10 f) sont introduites dans un organisme non humain.
